(19) **Europäisches Patentamt / European Patent Office / Office européen des brevets**

(11) **EP 3 783 362 A2**

(12) # EUROPEAN PATENT APPLICATION

(43) Date of publication:
**24.02.2021 Bulletin 2021/08**

(51) Int Cl.:
***G01N 33/68*** *(2006.01)*

(21) Application number: **20192473.5**

(22) Date of filing: **24.08.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.08.2019 EP 19193206**

(71) Applicant: **Laboratorium M. Nuytinck
9940 Evergem (BE)**

(72) Inventors:
- **Van Bruaene, Nathalie
  9700 Mater (BE)**
- **De Ryck, Tine
  9112 Sinaai-Waas (BE)**

(74) Representative: **De Clercq & Partners
Edgard Gevaertdreef 10a
9830 Sint-Martens-Latem (BE)**

(54) **BIOSIGNATURES FOR CHRONIC MENTAL STRESS**

(57) The current invention concerns an *in vitro* method for (i) predicting, diagnosing, prognosing and/or monitoring chronic mental stress or for (ii) determining whether a subject is in need of therapeutic or prophylactic treatment of a chronic mental stress in a subject, comprising detecting at least three biomarkers independently selected at least three, different groups of biomarkers selected from a group of immune function biomarkers, a group of iron metabolism biomarkers, a group of metabolism biomarkers, a group of steroid hormone biomarkers, a group of sympathetic adrenal medullary axis biomarkers, and optionally a group of neurotransmitter biomarkers, in a sample obtained from said subject, and kits and computer programs for use in such methods.

EP 3 783 362 A2

## Description

### TECHNICAL FIELD

The invention pertains to the technical field of an *in vitro* method and/or a diagnostic kit for predicting, diagnosing, prognosing and/or monitoring a fatigue syndrome, more particularly chronic mental stress, in a subject using biosignatures.

### BACKGROUND

Fatigue conditions or fatigue syndromes such as burn-out, overtraining, chronic stress, chronic fatigue and depression disorders are becoming more common in our society. The occurrence of these syndromes is often linked to an evolution in lifestyle, where the pressure to perform has increased considerably in the last decades. In addition to the pressure that individuals experience at work, they are also faced with a pressure to perform outside the working environment. For example, many individuals put a lot of pressure on themselves to be a good partner or a good parent, to be a good friend, etc. On the other hand it is also becoming a trend to excel at personal hobbies further adding to the pressure individuals put on themselves.

The impact of fatigue syndromes in economic terms is huge. The global costs of each of these syndromes are estimated to exceed hundreds of billions of US dollars annually. In addition, these syndromes have a significant impact on the individual himself, especially affecting social aspects of his personal life. Fatigue syndromes are, in general, associated with irritability, disturbed sleep patterns, decreased appetite, changes in hormone levels, increased vulnerability to infections and general malaise.

In work environment, chronic stress and insufficient recovery can lead to physical and emotional exhaustion, resulting in increased absenteeism. This comes with a huge economic cost apart from the extra workload for the remaining employees. As prevention is better than cure, companies are integrating prevention programs and risk analysis in favor for the wellbeing of their employees. Most tools are based on questionnaires integrated in apps providing advice, nevertheless, the compliance to fill questionnaire completely on a regular base is low. In addition, most people susceptible for mental illness or malaise do not recognize the signals and symptoms by themselves, resulting in a wrong image based on questionnaires. Therefore there is a huge need for evidence based objective tools to prevent their employees to develop chronic fatigue symptoms such as burn-out or depression and to measure the effect of wellness programs in companies.

In sport, intensified training results in performance changes such as short-term overreaching (functional overreaching), extreme overreaching (non-functional overreaching), or overtraining syndrome. Overtraining syndrome (OTS) is considered a stage of fatigue combined with maladaptation of various physiological mechanisms to the intensified training, resulting in chronic performance reduction. Athletes suffering from OTS present symptoms such as excessive fatigue, exhaustion, and decreased performance levels. Depending on the level of overtraining it can take years for the athlete to recover.

The onset stage of OTS is referred to as non-functional overreaching (NFO). Adequate rest during the onset stage of OTS (NFO), however, can prevent the development of OTS.

Detection of NFO and OTS has hitherto depended on monitoring the performance of the athlete and inferring fatigue, overreaching or overtraining on the basis of a deterioration in the athlete's performance. Using this technique, however, it is only possible to detect these conditions when the athlete has already passed his peak condition.

Furthermore, fatigue syndromes such as mental disorder and OTS also affect animals. Diagnosis thereof is almost impossible due to lack of verbal communication with the animal patient.

There is a need for a technique which can objectively determine the fatigue status (NFO, OTS, burn-out) of an individual. Due to the huge impact of these fatigue syndromes, there is a further need to diagnose the onset thereof in order to prevent development of more serious forms of the syndromes. Nowadays a lot of health programs try to prevent fatigue status, however as there is no real objective tool to measure the effect of these programs, the impact is unknown.

The invention thereto aims to provide a reliable and objective method for detecting the onset and / or existence of fatigue syndromes such as burn-out syndrome, non-functional overreaching, overtraining syndrome and chronic fatigue syndrome in both humans and animals. This will help in the early detection and also in the follow-up measurements of an intervention.

### SUMMARY OF THE INVENTION

Present inventors found that combining the detection of three or more biomarkers selected from well-defined groups of biomarkers in a multidimensional approach results in (i) a better prediction, diagnosis, prognosis and/or monitoring of a fatigue syndrome, preferably chronic mental stress (e.g. chronic mental stress as the mental status prior to development of mood disorders, such as depression or burn-out), (ii) a better determination whether a subject is in need of therapeutic or prophylactic treatment of a fatigue syndrome, preferably chronic mental stress, and/or (iii) a better prediction of the response of a subject diagnosed with a fatigue syndrome, preferably chronic mental stress, to therapy, compared to single biomarkers.

Accordingly, a first aspect provides an *in vitro* method for predicting, diagnosing, prognosing and/or monitoring chronic mental stress comprising detecting at least three biomarkers independently selected from at least three different groups of biomarkers selected from:

- a group of immune function biomarkers consisting of neopterin, secretory immunoglobulin A (sIgA), interleukin 6 (IL-6), interleukin 10 (IL-10), interleukin-1 receptor A (IL-1RA), epidermal growth factor (EGF), leukocytes, neutrophils, lymphocytes, monocytes, basophils, C-reactive protein (CRP) and myeloperoxidase (MPO);

- a group of iron metabolism biomarkers consisting of ferritin, transferrin, iron, transferrin saturation, hemoglobin in reticulocytes, erythrocytes, mean cell haemoglobin (MCH), mean cell volume (MCV), mean cell haemoglobin concentration (MCHC) and reticulocytes;

- a group of metabolism biomarkers consisting of creatinine, urea, vitamin D, folic acid, riboflavin, vitamin B12, 4-pyridoxic acid, flavin mononucleotide, thiamine, nicotinamide, pyridoxal 5'-phosphate, N1-methylnicotinamide, trigonelline, thiamine monophosphate, and visfatin;

- a group of steroid hormone biomarkers consisting of dehydroepiandrosterone sulfate (DHEA-S), sex hormone binding globulin (SHBG), bio-available testosterone, total testosterone, free testosterone, and dehydroepiandrosterone (DHEA); and

- a group of sympathetic adrenal medullary axis biomarkers consisting of amylase (preferably alpha-amylase), epinephrine, and norepinephrine; and

- optionally a group of neurotransmitter biomarkers consisting of anthranilic acid, cystathionine, picolinic acid, kynurenic acid (KYNA), L-kynurenine, quinolinic acid (QUIN), free tryptophan, xanthurenic acid, brain-derived neurotrophic factor (BDNF), S100 calcium-binding protein B (S100B), and γ-aminobutyric acid (GABA);

in a sample obtained from said subject;
wherein chronic mental stress is the mental status prior to development of mood disorders, such as depression or burn-out.

In particular embodiments, the at least three biomarkers are independently selected from at least three different groups of biomarkers selected from:

- a group of immune function biomarkers consisting of neopterin, sIgA, IL-6, IL-10, IL-1RA, and CRP;

- a group of iron metabolism biomarkers consisting of transferrin, iron, transferrin saturation, and ferritin;

- a group of metabolism biomarkers consisting of creatinine, urea, vitamin D, folic acid, riboflavin, vitamin B12, 4-pyridoxic acid, pyridoxal 5'-phosphate, N1-methylnicotinamide, and visfatin;

- a group of steroid hormone biomarkers consisting of DHEA-S and free testosterone; and

- a group of sympathetic adrenal medullary axis biomarkers consisting of amylase, preferably alpha-amylase.

In particular embodiments, the method as taught herein comprising detecting at least four, preferably at least five, more preferably at least six, biomarkers in a sample obtained from said subject.

In particular embodiments, at least four biomarkers are independently selected from at least four different groups of biomarkers selected from the groups of biomarkers selected from:

- a group of immune function biomarkers consisting of neopterin, sIgA, IL-6, IL-10, IL-1RA, EGF, leukocytes, neutrophils, lymphocytes, monocytes, basophils, CRP and MPO;

- a group of iron metabolism biomarkers consisting of ferritin, transferrin, iron, transferrin saturation, hemoglobin in reticulocytes, erythrocytes, MCH, MCV, MCHC and reticulocytes;

3

- a group of metabolism biomarkers consisting of creatinine, urea, vitamin D, folic acid, riboflavin, vitamin B12, 4-pyridoxic acid, flavin mononucleotide, thiamine, nicotinamide, pyridoxal 5'-phosphate, N1-methylnicotinamide, trigonelline, thiamine monophosphate, and visfatin;

- a group of steroid hormone biomarkers consisting of DHEA-S, SHBG, bio-available testosterone, total testosterone, free testosterone, and DHEA; and

- a group of sympathetic adrenal medullary axis biomarkers consisting of amylase (preferably alpha-amylase), epinephrine, and norepinephrine; and

- optionally a group of neurotransmitter biomarkers consisting of anthranilic acid, cystathionine, picolinic acid, KYNA, L-kynurenine, QUIN, free tryptophan, xanthurenic acid, BDNF, S100B, and GABA;

preferably selected from:

- a group of immune function biomarkers consisting of neopterin, sIgA, IL-6, IL-10, IL-1RA, and CRP;

- a group of iron metabolism biomarkers consisting of transferrin, iron, transferrin saturation, and ferritin;

- a group of metabolism biomarkers consisting of creatinine, urea, vitamin D, folic acid, riboflavin, vitamin B12, 4-pyridoxic acid, pyridoxal 5'-phosphate, N1-methylnicotinamide, and visfatin;

- a group of steroid hormone biomarkers consisting of DHEA-S and free testosterone; and

- a group of sympathetic adrenal medullary axis biomarkers consisting of amylase, preferably alpha-amylase.

In particular embodiments, detecting said at least three biomarkers allows obtaining a biosignature and said method further comprises a step of predicting diagnosing, prognosing and/or monitoring chronic mental stress based on said biosignature comprising multivariate statistical modelling of said biosignature.

In particular embodiments, the method comprises the steps of

(i) determining the status of said at least three biomarkers in the sample from the subject;

(ii) combining the status of said at least three biomarkers as determined in step (i) to a biomarker imbalance risk index (BIRIX);

(iii) finding a deviation or no deviation of the BIRIX compared to a reference value; and

(iv) attributing said finding of deviation or no deviation to a particular diagnosis, prediction, or prognosis of the fatigue syndrome in the subject.

In particular embodiments, the BIRIX is obtained by multiplying the status of each of said at least three biomarkers by a corresponding weight, and wherein an expression signature defines the weight for each biomarker, preferably wherein the expression signature is defined using partial least squares-discriminant analysis (PLS-DA).

A further aspect provides a kit for predicting, diagnosing, prognosing and/or monitoring chronic mental stress, the kit comprising:

- means for measuring the status of at least three biomarkers independently selected from at least three different groups of biomarkers selected from:

- a group of immune function biomarkers consisting of neopterin, sIgA, IL-6, IL-10, IL-1RA, EGF, leukocytes, neutrophils, lymphocytes, monocytes, basophils, CRP and MPO;

- a group of iron metabolism biomarkers consisting of ferritin, transferrin, iron, transferrin saturation, hemoglobin in reticulocytes, erythrocytes, MCH, MCV, MCHC and reticulocytes;

- a group of metabolism biomarkers consisting of creatinine, urea, vitamin D, folic acid, riboflavin, vitamin B12, 4-pyridoxic acid, flavin mononucleotide, thiamine, nicotinamide, pyridoxal 5'-phosphate, N1-methylnicotinamide, trigonelline, thiamine monophosphate, and visfatin;

- a group of steroid hormone biomarkers consisting of DHEA-S, SHBG, bio-available testosterone, total testosterone, free testosterone, and DHEA; and

- a group of sympathetic adrenal medullary axis biomarkers consisting of amylase (preferably alpha-amylase), epinephrine, and norepinephrine; and

- optionally a group of neurotransmitter biomarkers consisting of anthranilic acid, cystathionine, picolinic acid, KYNA, L-kynurenine, QUIN, free tryptophan, xanthurenic acid, BDNF, S100B, and GABA;

preferably selected from:

- a group of immune function biomarkers consisting of neopterin, sIgA, IL-6, IL-10, IL-1RA, and CRP;

- a group of iron metabolism biomarkers consisting of transferrin, iron, transferrin saturation, and ferritin;

- a group of metabolism biomarkers consisting of creatinine, urea, vitamin D, folic acid, riboflavin, vitamin B12, 4-pyridoxic acid, pyridoxal 5'-phosphate, N1-methylnicotinamide, and visfatin;

- a group of steroid hormone biomarkers consisting of DHEA-S and free testosterone; and

- a group of sympathetic adrenal medullary axis biomarkers consisting of amylase, preferably alpha-amylase;

in a sample obtained from said subject; and

- a reference value or means for establishing said reference value, wherein said reference value represents a known diagnosis, prediction and/or prognosis of chronic mental stress;

wherein chronic mental stress is the mental status prior to development of mood disorders, such as depression or burn-out.

In particular embodiments, said subject is an employee.

In particular embodiments, the kit further comprises a computer readable storage medium having recorded thereon one or more programs for carrying out the method as taught herein.

A further aspect provides a computer program product for use in conjunction with a computer having a processor and a memory connected to the processor, said computer program product comprising a computer readable storage medium having a computer program mechanism encoded thereon, wherein said computer program mechanism is loaded into the memory of said computer and causes said computer to carry out the method as taught herein.

A further aspect provides an *in vitro* method for determining whether a subject is in need of therapeutic or prophylactic treatment of chronic mental stress comprising detecting at least three biomarkers independently selected from at least three different groups of biomarkers selected from:

- a group of immune function biomarkers consisting of neopterin, sIgA, IL-6, IL-10, IL-1RA, EGF, leukocytes, neutrophils, lymphocytes, monocytes, basophils, CRP and MPO;

- a group of iron metabolism biomarkers consisting of ferritin, transferrin, iron, transferrin saturation, hemoglobin in reticulocytes, erythrocytes, MCH, MCV, MCHC and reticulocytes;

- a group of metabolism biomarkers consisting of creatinine, urea, vitamin D, folic acid, riboflavin, vitamin B12, 4-pyridoxic acid, flavin mononucleotide, thiamine, nicotinamide, pyridoxal 5'-phosphate, N1-methylnicotinamide, trigonelline, thiamine monophosphate, and visfatin;

- a group of steroid hormone biomarkers consisting of DHEA-S, SHBG, bio-available testosterone, total testosterone, free testosterone, and DHEA; and

- a group of sympathetic adrenal medullary axis biomarkers consisting of amylase (preferably alpha-amylase), epinephrine, and norepinephrine; and

- optionally a group of neurotransmitter biomarkers consisting of anthranilic acid, cystathionine, picolinic acid, KYNA, L-kynurenine, QUIN, free tryptophan, xanthurenic acid, BDNF, S100B, and GABA;

preferably selected from:

- a group of immune function biomarkers consisting of neopterin, sIgA, IL-6, IL-10, IL-1RA, and CRP;

- a group of iron metabolism biomarkers consisting of transferrin, iron, transferrin saturation, and ferritin;

- a group of metabolism biomarkers consisting of creatinine, urea, vitamin D, folic acid, riboflavin, vitamin B12, 4-pyridoxic acid, pyridoxal 5'-phosphate, N1-methylnicotinamide, and visfatin;

- a group of steroid hormone biomarkers consisting of DHEA-S and free testosterone; and

- a group of sympathetic adrenal medullary axis biomarkers consisting of amylase, preferably alpha-amylase;

in a sample obtained from said subject;
wherein chronic mental stress is the mental status prior to development of mood disorders, such as depression or burn-out.

A further aspect provides an *in vitro* method for predicting the response of a subject diagnosed with chronic mental stress to therapy, comprising detecting at least three biomarkers independently selected from at least three different groups of biomarkers selected from:

- a group of immune function biomarkers consisting of neopterin, sIgA, IL-6, IL-10, IL-1RA, EGF, leukocytes, neutrophils, lymphocytes, monocytes, basophils, CRP and MPO;

- a group of iron metabolism biomarkers consisting of ferritin, transferrin, iron, transferrin saturation, hemoglobin in reticulocytes, erythrocytes, MCH, MCV, MCHC and reticulocytes;

- a group of metabolism biomarkers consisting of creatinine, urea, vitamin D, folic acid, riboflavin, vitamin B12, 4-pyridoxic acid, flavin mononucleotide, thiamine, nicotinamide, pyridoxal 5'-phosphate, N1-methylnicotinamide, trigonelline, thiamine monophosphate, and visfatin;

- a group of steroid hormone biomarkers consisting of DHEA-S, SHBG, bio-available testosterone, total testosterone, free testosterone, and DHEA; and

- a group of sympathetic adrenal medullary axis biomarkers consisting of amylase (preferably alpha-amylase), epinephrine, and norepinephrine; and

- optionally a group of neurotransmitter biomarkers consisting of anthranilic acid, cystathionine, picolinic acid, KYNA, L-kynurenine, QUIN, free tryptophan, xanthurenic acid, BDNF, S100B, GABA;

preferably selected from:

- a group of immune function biomarkers consisting of neopterin, sIgA, IL-6, IL-10, IL-1RA, and CRP;

- a group of iron metabolism biomarkers consisting of transferrin, iron, transferrin saturation, and ferritin;

- a group of metabolism biomarkers consisting of creatinine, urea, vitamin D, folic acid, riboflavin, vitamin B12, 4-pyridoxic acid, pyridoxal 5'-phosphate, N1-methylnicotinamide, and visfatin;

- a group of steroid hormone biomarkers consisting of DHEA-S and free testosterone; and

- a group of sympathetic adrenal medullary axis biomarkers consisting of amylase, preferably alpha-amylase;

in a sample obtained from said subject;
wherein chronic mental stress is the mental status prior to development of mood disorders, such as depression or burn-out.

## DESCRIPTION OF FIGURES

Figure 1: Tables 1-11: lists of relevant biomarkers

Figure 2: Boxplot representation of the LAS score.

Figure 3: Boxplot representation of the MAS score.

Figure 4: MAS score for individuals

Figure 5: Relation between the fatigue condition and the corresponding BIRIX.

Figure 6: Comparison of ROC curves of LAS and two commonly used univariate biomarkers.

Figure 7: Boxplot representation of the LAS score in athletes.

Figure 8: LAS profile of an athlete during his training for an IM

Figure 9: Boxplot representation of the MAS score.

Figure 10: MAS profile of an employee

Figure 11: Boxplot representation of the TAS score in team sport athletes.

Figure 12: Team overview of the TAS profiles of a pro-league soccer team

Figure 13: Evaluation of the LAS in practice by medical sport doctors

Figure 14: Implementation of the BIRIX in work environment

Figure 15: Boxplot representation of the MAS score in different stages of burn-out

## DETAILED DESCRIPTION OF THE INVENTION

As used herein, the singular forms "a", "an", and "the" include both singular and plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms also encompass "consisting of' and "consisting essentially of', which enjoy well-established meanings in patent terminology.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any $\geq 3$, $\geq 4$, $\geq 5$, $\geq 6$ or $\geq 7$ etc. of said members, and up to all said members. In another example, "one or more" or "at least one" may refer to 1, 2, 3, 4, 5, 6, 7 or more.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation is meant to apply throughout this specification, i.e., also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

As used herein, the following terms have the following meanings:
Overtraining syndrome (OTS) refers to a stage of fatigue combined with maladaptation of various physiological mechanisms to the intensified training that results in chronic performance reduction. OTS is also referred to by athletes as "staleness".

Non-functional overreaching (NFO) can develop as a consequence of intensified training and constitutes the earliest stage or onset of OTS. At this stage, adequate rest can prevent the development of OTS.

Burn-out syndrome or burn-out refers to the exhaustion of physical or emotional strength or motivation usually as a result of long-term stress or frustration. Burn-out syndrome is often characterized by a reduced self-esteem of the individual at its workplace. Most characteristics of burn-out syndrome are shared with overtraining syndrome. Additionally, burn-out syndrome often leads to the development of depression disorder.

Chronic mental stress refers to a state where an individual is suffering from high mental load. This can be an early stage of (the onset of) burn-out or depression. At this early stage, adequate recovery and/or reducing the stress can prevent further development of the chronic mental stress to burn-out or depression. Chronic mental stress is considered as one of the most potent non-genomic factors in the development of mood disorders (Gold and Chrousos, 2002), such as depression (e.g. major depressive disorder (MDD)) and burn-out. Research demonstrates that chronic stress initiates reduction of synaptic plasticity and dendritic spines, length and complexity of dendrites, and impairment of neurogenesis, which may evolve into worse condition for depression (Christopher Pittenger and Ronald S Duman, 2008; Gal Richter-Levin and Lin Xu, 2018) and burnout (Anna Sjörs Dahlman et al, 2019). Despite substantial interrelationships between symptoms of stress, burnout and depression, evidence exists showing distinct underlying psychological constructs.

Accordingly, in particular embodiments, chronic mental stress is the status, preferably mental status, prior to development of mood disorders, such as depression or burn-out.

Chronic fatigue syndrome (CFS) or myalgic encephalomyelitis (ME) refers to a disorder characterized by long-term fatigue and extreme tiredness that do not go away with rest and result in the limitation of a person's ability to carry out ordinary daily activities.

The term "athlete" or "sportsperson" refers to a person that is skilled or trained in exercises, sports, or games requiring physical strength, agility, endurance and/or stamina. The term "professional athlete" or "elite athlete" refers to an athlete that receives payment for his or her performance. The term "recreational athlete", "amateur athlete" or "hobby" athlete refers to an athlete that trains during his or her spare time, mainly to relax. The term "sport" refers to a physical activity.

The term "employee" refers to a person who is handling commissioned by an "employer" or "firm". The job of the employee can be broad, going from physically active jobs, like but not limited to military training, cleaning managers or masons to people with a sedentary job like, but not limited to managers or manager assistants.

"Biomarker", "marker" or "biological marker" refers to a naturally occurring indicator such as a molecule, gene, metabolite, protein, cell type, vitamin, trace element, feature or characteristic which can be measured accurately, objectively and reproducibly, by which a particular pathological or physiological condition, such as but not limited to a process, disease, syndrome or disorder, can be identified. An example of a feature biomarker is the erythrocyte sedimentation rate. The status of a biomarker refers to the outcome of measurement of said biomarker and comprises the presence, absence and/or amount of said biomarker, wherein the "amount" of said biomarker includes changes in the amount of said biomarker.

The term "univariate" refers to a single variable, which can be related to just one biomarker, or the combination of two or more biomarkers, as for example a ratio of 2 biomarkers.

"Biosignature" signifies the result of measures of a set of biomarkers relevant for the early detection of disease and prediction of treatment effectiveness

The term "BIRIX" or "biomarker imbalance risk index" is based on the biosignature and describes the risk for a fatigue condition or syndrome. It reflects the attribute, characteristic or exposure of an individual that increases the likelihood of developing a disease or injury.

The term "LAS" or "load adaptation score" reflects the BIRIX for athletes when they encounter external and internal training load.

The term "MAS" or "mental adaptation score" reflects the BIRIX of the mental adaption to external and internal stress factors.

The term "TAS" or "training adaptation score" reflects the BIRIX of team athletes towards their training sessions.

The terms "sample" or "biological sample" as used herein include any biological specimen obtained and isolated from a subject (e.g., a human, such as an athlete or an animal, such as a horse). Samples may include, without limitation, organ tissue (i.e., tumour tissue, more particular breast tumour tissue), whole blood, plasma, serum, whole blood cells, red blood cells, white blood cells (e.g., peripheral blood mononuclear cells), saliva, urine, stool (i.e., faeces), tears, sweat, sebum, nipple aspirate, ductal lavage, tumour exudates, synovial fluid, cerebrospinal fluid, lymph, fine needle aspirate, amniotic fluid, any other bodily fluid, cell lysates, cellular secretion products, inflammation fluid, semen and vaginal secretions. Preferably, a sample may be readily obtainable by minimally invasive methods, such as blood collection or tissue biopsy, allowing the removal / isolation / provision of the sample from the subject. The term "tissue" as used herein encompasses all types of cells of the human body including cells of organs but also including blood and other body fluids recited above. The sample typically comprises one or more biomarkers.

The term "subject", "individual" or "patient" as used herein typically and preferably denotes humans, but may also encompass reference to non-human animals, preferably warm-blooded animals, more preferably vertebrates, even more preferably mammals, such as, e.g., non-human primates, rodents, canines, felines, equines, ovines, porcines, and the like. Particularly intended are subjects at high risk to develop a fatigue syndrome (such as athletes and employees (e.g. managers)) or subjects known or suspected to have a fatigue syndrome. Suitable subjects may include ones presenting to a physician for a screening for a fatigue syndrome and/or with symptoms and signs indicative of a fatigue syndrome.

The term "condition" herein refers to "medical condition" as a broad term that includes all diseases and disorders, but can include injuries and normal health situations, such as pregnancy, that might affect a person's health, benefit from medical assistance, or have implications for medical treatments. The term "fatigue condition" or "fatigue syndrome" refers to one or more medical conditions chosen from the group of non-functional overreaching (NFO), overtraining syndrome (OTS), burn-out syndrome, chronic fatigue syndrome (CFS), chronic stress and/or depression disorder.

Most testosterone found in an individual is bound to protein, with a large amount bound to sex hormone-binding globulin (SHBG) and a smaller amount bound weakly to albumin. The biomarker "free testosterone" refers to unbound testosterone. The biomarker "bio-available testosterone" refers to testosterone bound to albumin and free testosterone. The biomarker "total testosterone" refers to both bound and unbound testosterone.

Similarly, most of the cortisol found in an individual is bound to a protein, with a major amount bound to cortisol-binding globulin (CBG) and a smaller amount bound to albumin. The biomarker "free cortisol" refers to unbound cortisol. The biomarker "total cortisol" refers to both bound and unbound cortisol.

Up to now, researchers and medical doctors are mainly interpreting the biomarkers one by one. Present inventors found that combining the detection of three or more biomarkers selected from well-defined groups of biomarkers in a multidimensional approach allows (i) a better prediction, diagnosis, prognosis and/or monitoring of a fatigue syndrome, such as chronic mental stress, (ii) a better determination whether a subject is in need of therapeutic or prophylactic treatment of a fatigue syndrome, such as chronic mental stress, and/or (iii) a better prediction of the response of a subject diagnosed with a fatigue syndrome, such as chronic mental stress, to therapy, compared to single biomarkers. More particularly, combining the status of at least three biomarkers leads to an unexpected increase in accuracy and sensitivity of (i) the prediction, diagnosis, prognosis and/or monitoring of a fatigue syndrome, such as chronic mental stress, (ii) the determination whether a subject is in need of therapeutic or prophylactic treatment of a fatigue syndrome, such as chronic mental stress, and/or (iii) the response of a subject diagnosed with a fatigue syndrome, such as chronic mental stress, to therapy, in a subject. This allows inferring an unambiguous conclusion related to (i) the prediction, diagnosis, prognosis and/or monitoring of a fatigue syndrome, such as chronic mental stress, (ii) the determination whether a subject is in need of therapeutic or prophylactic treatment of a fatigue syndrome, such as chronic mental stress, and/or (iii) the response of a subject diagnosed with a fatigue syndrome, such as chronic mental stress, to therapy in an individual based on the determination of the status of individual biomarkers which may not be informative on their own.

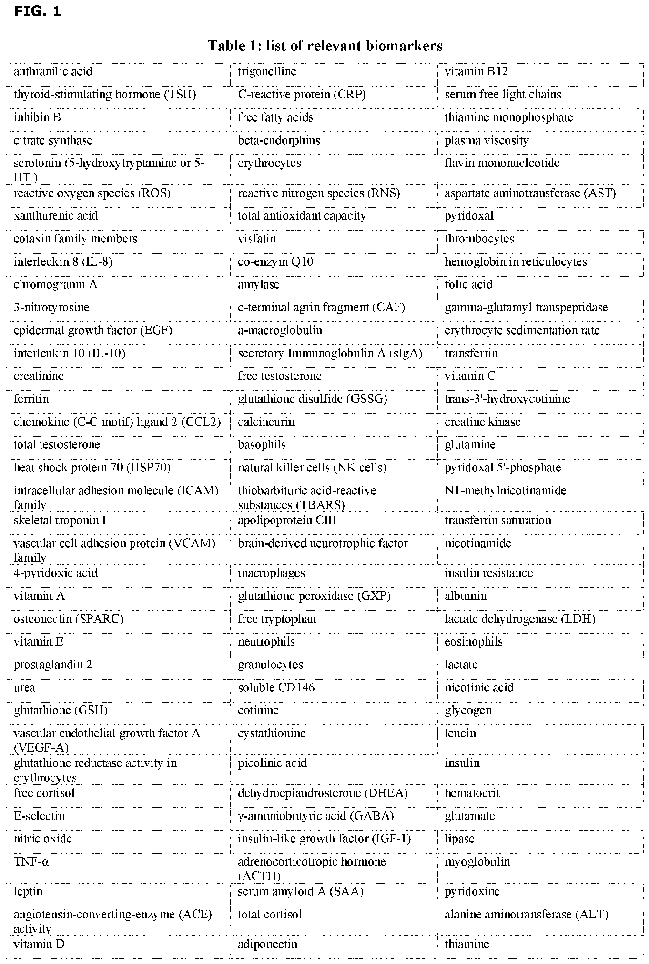

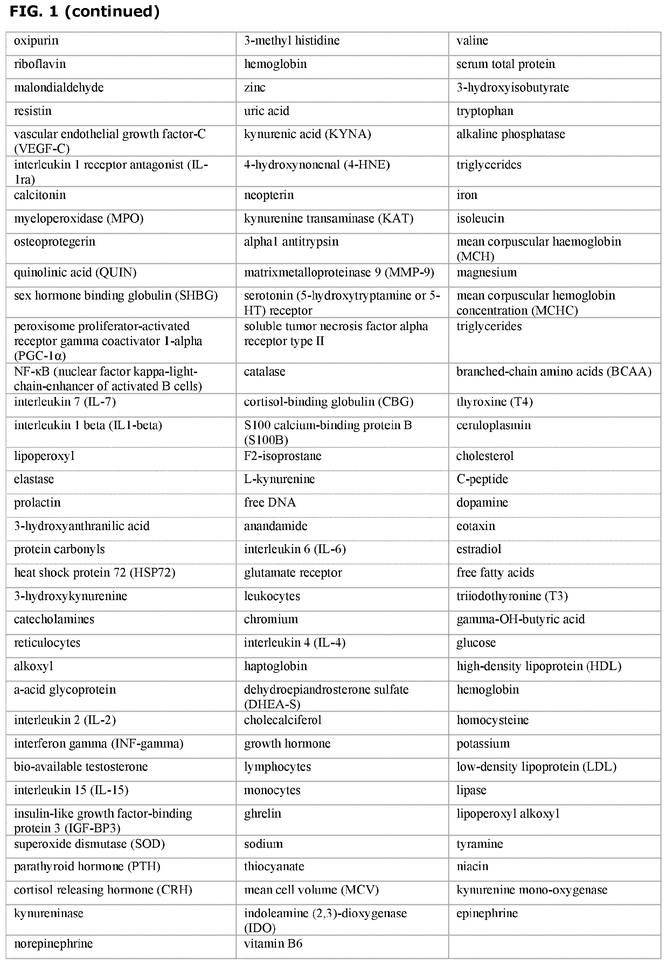

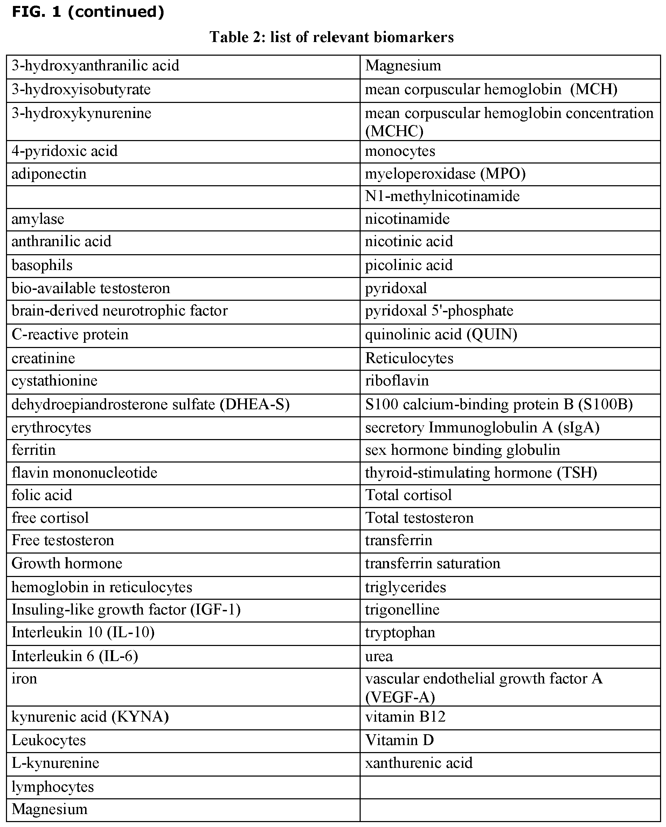

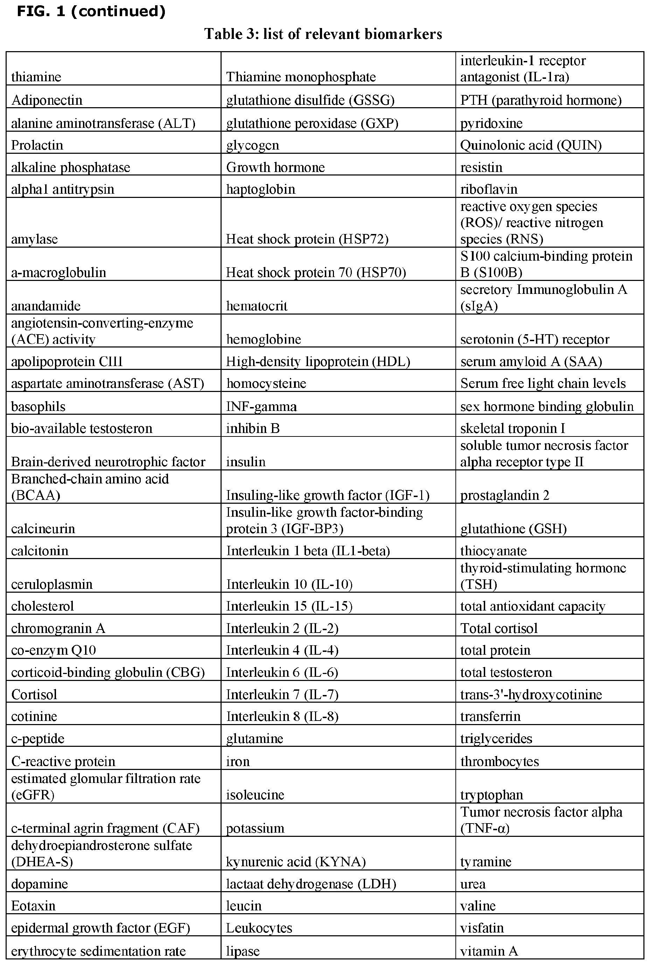

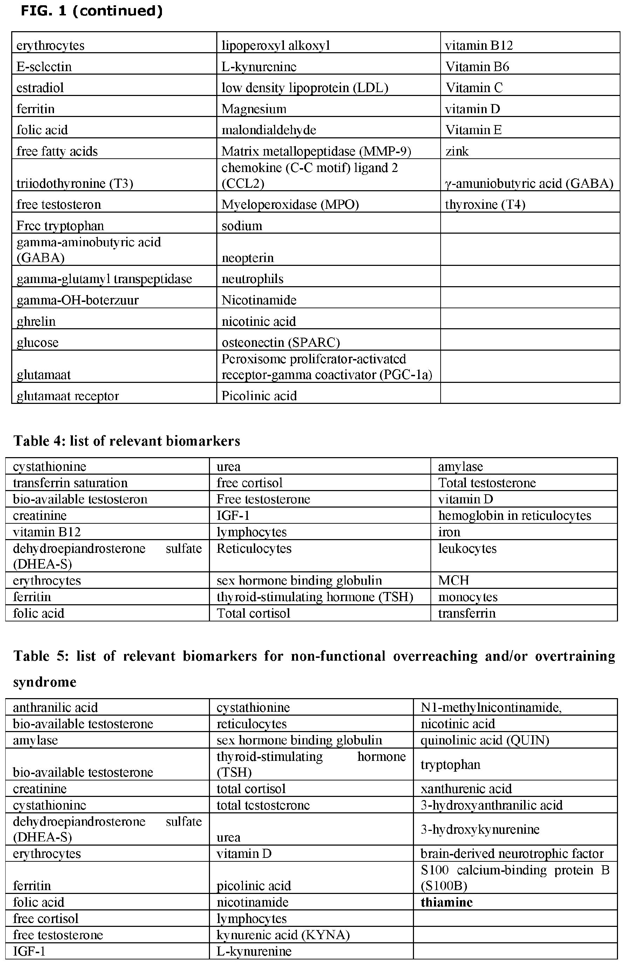

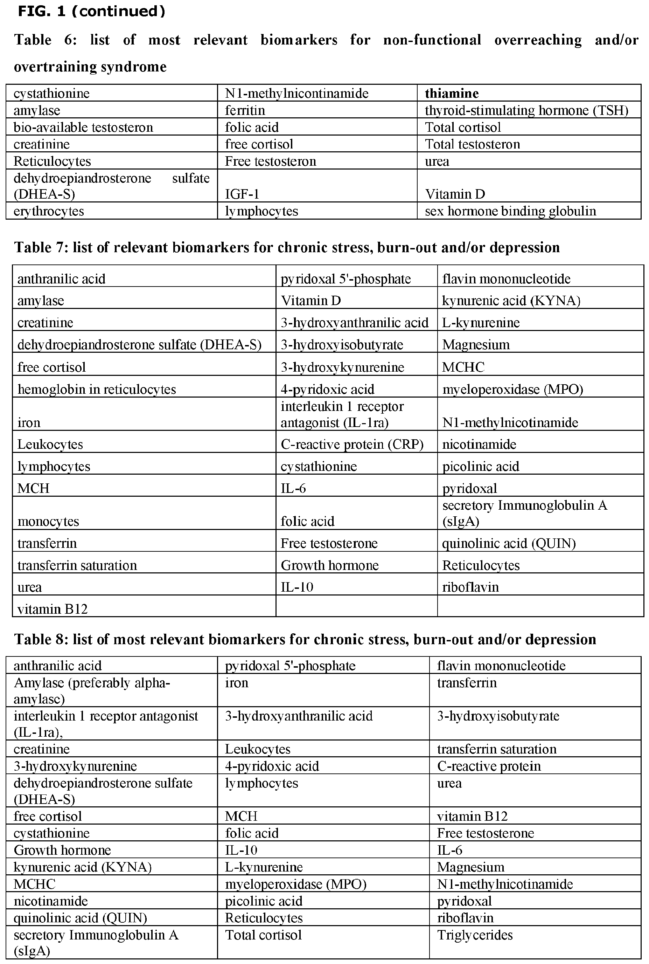

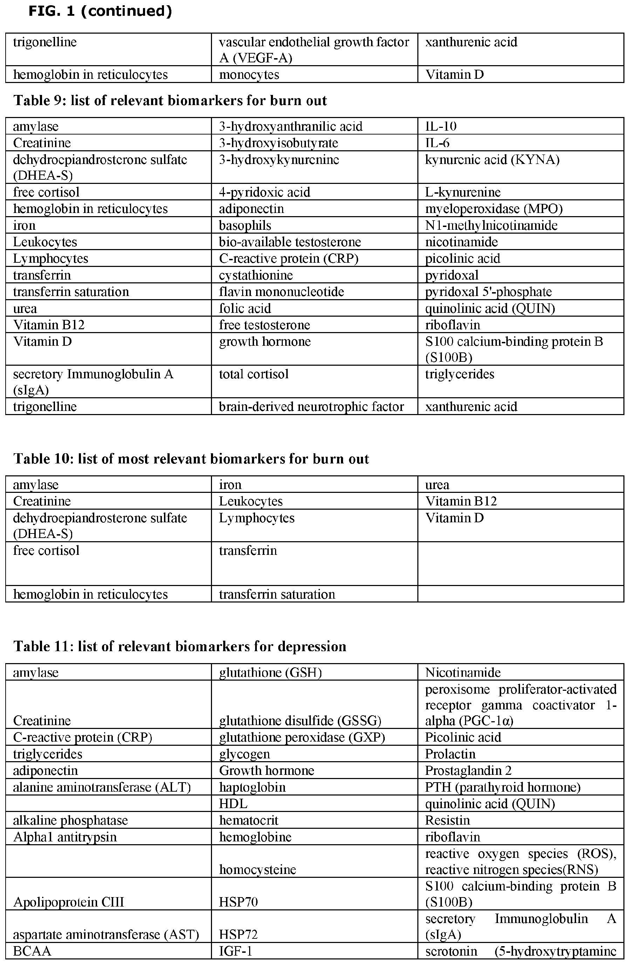

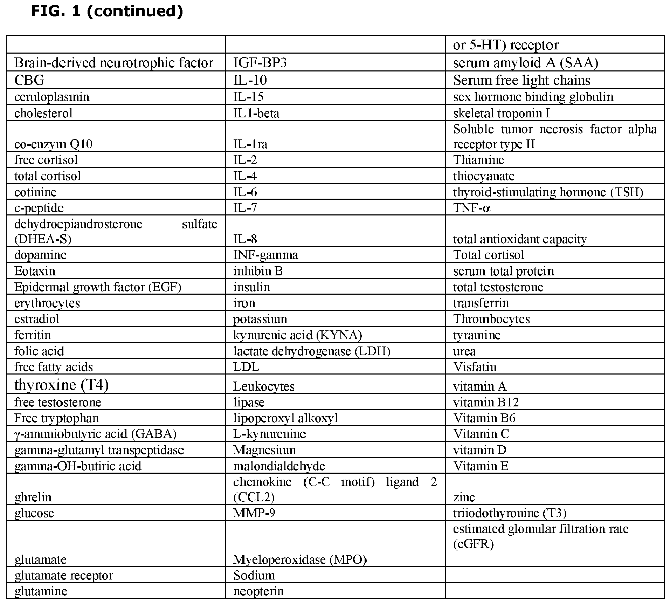

A first aspect of the invention provides an *in vitro* method for predicting, diagnosing, prognosing and/or monitoring a fatigue syndrome in a subject or for determining whether a subject is in need of therapeutic or prophylactic treatment of a fatigue syndrome in a subject, wherein the fatigue syndrome is selected from the group consisting of burn-out, depression, chronic stress (preferably chronic stress or chronic mental stress, more preferably chronic stress or chronic mental stress as a precursor or the indication of the early stage (onset) of burn-out and/or depression), chronic fatigue syndrome, non-functional overreaching and overtraining syndrome, the method comprising detecting at least three biomarkers independently selected from anthranilic acid, trigonelline, vitamin B12, thyroid-stimulating hormone (TSH), C-reactive protein (CRP), serum free light chains, inhibin B, free fatty acids, thiamine monophosphate, citrate synthase, beta-endorphins, plasma viscosity, serotonin (5-hydroxytryptamine or 5-HT), erythrocytes, flavin mononucleotide, reactive oxygen species (ROS), reactive nitrogen species (RNS), Vitamin C, aspartate aminotransferase (AST), xanthurenic acid, total antioxidant capacity, pyridoxal, eotaxin family members, visfatin, thrombocytes, interleukin 8 (IL-8), co-enzym Q10, hemoglobin in reticulocytes, chromogranin A, amylase (preferably alpha-amylase), folic acid, 3-nitrotyrosine, c-terminal agrin fragment (CAF), gamma-glutamyl transpeptidase, epidermal growth factor (EGF), $\alpha$-macroglobulin, erythrocyte sedimentation rate, interleukin 10 (IL-10), secretory Immunoglobulin A (sIgA), transferrin, creatinine, free testosterone, ferritin, glutathione disulfide (GSSG), trans-3'-hydroxycotinine, chemokine (C-C motif) ligand 2 (CCL2), calcineurin, creatine kinase, total testosterone, basophils, glutamine, heat shock protein 70 (HSP70), natural killer cells (NK cells), macrophages, pyridoxal 5'-phosphate, intracellular adhesion molecule (ICAM) family, thiobarbituric acid-reactive substances (TBARS), N1-methylnicotinamide, skeletal troponin I, apolipoprotein CIII, transferrin saturation, vascular cell adhesion protein (VCAM) family, brain-derived neurotrophic factor (BDNF), nicotinamide, 4-pyridoxic acid, insulin resistance, vitamin A, glutathione peroxidase (GXP), albumin, osteonectin (SPARC), free tryptophan, lactate dehydrogenase (LDH), vitamin E, neutrophils, eosinophils, prostaglandin 2, granulocytes, lactate, urea, soluble CD146, nicotinic acid, glutathione (GSH), cotinine, glycogen, vascular endothelial growth factor A (VEGF-A), cystathionine, leucin, glutathione reductase activity in erythrocytes, picolinic acid, insulin, free cortisol, dehydroepiandrosterone (DHEA), hematocrit, E-selectin, $\gamma$-aminobutyric acid (GABA), glutamate, nitric oxide, insulin-like growth factor (IGF-1), lipase, tumor necrosis factor-alfa (TNF-$\alpha$), adrenocorticotropic hormone (ACTH), myoglobulin, leptin, serum amyloid A (SAA), pyridoxine, angiotensin-converting-enzyme (ACE) activity, total cortisol, alanine aminotransferase (ALT), vitamin D, adiponectin, thiamine, oxipurin, 3-methyl histidine, valine, riboflavin, hemoglobin, serum total protein, malondialdehyde, zinc, 3-hydroxyisobutyrate, resistin, uric acid, tryptophan, vascular endothelial growth factor-C (VEGF-C), kynurenic acid (KYNA), alkaline phosphatase, interleukin 1 receptor antagonist (IL-1ra), 4-hydroxynonenal (4-HNE), triglycerides, calcitonin, neopterin, iron, myeloperoxidase (MPO), isoleucin, osteoprotegerin, alpha1 antitrypsin, Mean corpuscular haemoglobin (MCH), quinolinic acid (QUIN), matrixmetalloptease 9 (MMP-9), magnesium, sex hormone binding globulin (SHBG), serotonin (5-hydroxytryptamine or 5-HT) receptor, mean corpuscular hemoglobin concentration (MCHC), peroxisome proliferator-activated receptor gamma coactivator 1-alpha (PGC-1$\alpha$), soluble tumor necrosis factor alpha receptor type II, triglycerides, NF-$\kappa$B (nuclear factor kappa-light-chain-enhancer of activated B cells), catalase, branched-chain amino acids (BCAA), interleukin 7 (IL-7), cortisol-binding globulin (CBG), Interleukin 1 beta (IL1-beta), S100 calcium-binding protein B (S100B), ceruloplasmin, lipoperoxyl, F2-isoprostane, cholesterol, elastase, L-kynurenine, C-peptide, prolactin, free DNA, dopamine, 3-hydroxyanthralic acid, anandamide, eotaxin, protein carbonyls, interleukin 6 (IL-6), estradiol, heat shock protein 72 (HSP72), glutamate receptor, free fatty acids, 3-hydroxykynurenine, leukocytes, triiodothyronine (T3), thyroxine (T4), catecholamines chromium, gamma-OH-butiric acid, reticulocytes, interleukin 4 (IL-4), glucose, alkoxyl, haptoglobin, high-density lipoprotein (HDL), a-acid glycoprotein, dehydroepiandrosterone sulfate (DHEA-S), hemoglobin, interleukin 2 (IL-2), cholecalciferol, homocysteine, interferon gamma (INF-gamma), growth hormone, potassium, bio-available testosterone, lymphocytes, low-density lipoprotein (LDL), interleukin 15 (IL-15), monocytes, lipase, insulin-like growth factor-binding protein 3 (IGF-BP3), ghrelin, lipoperoxyl alkoxyl, superoxide dismutase (SOD), sodium, tyramine, parathyroid hormone (PTH), thiocyanate niacin, mean cell volume (MCV), cortisol releasing hormone (CRH), indoleamine (2,3)-dioxygenase (IDO), kynurenine mono-oxygenase, kynureninase, kynurenine transaminase (KAT), vitamin B6, epinephrine and norepinephrine(which corresponds to the biomarkers listed in Table 1 (Figure 1)) in a sample obtained from said subject.

Reference to any biomarkers as listed in Table 1 corresponds to the marker commonly known under the respective designations in the art. The terms encompasses such markers of any organism where found, and particularly animals, preferably warm-blooded animals, more preferably vertebrates, yet more preferably mammals, including humans and non-human mammals, still more preferably of humans.

The terms particularly encompass such markers with a native sequence, i.e., ones of which the primary sequence is the same as that of the markers found in or derived from nature. A skilled person understands that native sequences may differ between different species due to genetic divergence between such species. Moreover, native sequences may differ between or within different individuals of the same species due to normal genetic diversity (variation) within a given species. Also, native sequences may differ between or even within different individuals of the same species due to post-transcriptional or post-translational modifications. Any such variants or isoforms of markers are intended herein. Accordingly, all sequences of markers found in or derived from nature are considered "native". The terms encompass the markers when forming a part of a living organism, organ, tissue or cell, when forming a part of a biological sample, as well as when at least partly isolated from such sources.

The biomarkers as referred to herein also include variants or mutants thereof, such as variants and mutants of proteins, polypeptides or peptides which carry amino acid sequence variations vis-à-vis a corresponding native proteins, such as, e.g., amino acid deletions, additions and/or substitutions. The term contemplates both full-length biomarkers, biomarker parts or fragments, e.g., naturally-occurring parts that ensue from processing of such full-length biomarkers.

In particular embodiments, the biomarkers as listed in Table 1 may also be replaced by a molecule or measureable fragment of the molecule, found upstream or downstream of the biomarker in a biological pathway.

In particular embodiments, the method comprises detecting at least three biomarkers independently selected from

- the group of biomarkers consisting of 3-hydroxyanthranilic acid, 3-hydroxyisobutyrate, pyridoxal, pyridoxal 5'-phosphate, 3-hydroxykynurenine, 4-pyridoxic acid, adiponectin, albumin, amylase (preferably alpha-amylase), anthranilic acid, basophils, bio-available testosterone, brain-derived neurotrophic factor (BDNF), C-reactive protein (CRP), creatinine, cystathionine, dehydroepiandrosterone sulfate (DHEA-S), erythrocytes, ferritin, flavin mononucleotide, folic acid, free cortisol, free testosterone, growth hormone, hemoglobin in reticulocytes , insulin-like growth factor (IGF-1), IL-10, IL-6, iron, KYNA, leukocytes, L-kynurenine, lymphocytes, magnesium, MCH, MCHC, monocytes, MPO, N1-methylnicotinamide, nicotinamide, nicotinic acid, picolinic acid, quinolinic acid (QUIN), Reticulocytes, riboflavin, S100 calcium-binding protein B (S100B), secretory Immunoglobulin A (sIgA), sex hormone binding globulin (SHBG), thyroid-stimulating hormone (TSH), total cortisol, total testosterone, transferrin, transferrin saturation, triglycerides, trigonelline, tryptophan, urea, vascular endothelial growth factor A (VEGF-A), vitamin B12, vitamin D and xanthurenic acid (which corresponds to the biomarkers listed in Table 2 (Figure 1); and/or

- the group of biomarkers consisting of thiamine, thiamine monophosphate, interleukin-1 receptor antagonist (IL-1ra), alanine aminotransferase (ALT), alkaline phosphatase, alpha1 antitrypsin, α-macroglobulin, anandamide, angiotensin-converting-enzyme (ACE) activity, apolipoprotein CIII, aspartate aminotransferase (AST), branched-chain amino acid (BCAA), calcineurin, calcitonin, ceruloplasmin, cholesterol, chromogranin A, co-enzym Q10, corticoid-binding globulin (CBG), cortisol, cotinine, c-peptide, estimated glomular filtration rate (eGFR), c-terminal agrin fragment (CAF), dopamine, eotaxin, epidermal growth factor (EGF), erythrocyte sedimentation rate, E-selectin, estradiol, free fatty acids, triiodothyronine (T3), thyroxine (T4), free tryptophan, gamma-aminobutyric acid (GABA), gamma-glutamyl transpeptidase, gamma-OH-boterzuur, ghrelin, glucose, glutamaat, glutamaat receptor, glutamine, glutathione (GSH), glutathione disulfide (GSSG), glutathione peroxidase (GXP), glycogen, haptoglobin, heat shock protein (HSP72), heat shock protein 70 (HSP70), hematocrit, hemoglobin, high-density lipoprotein (HDL), homocysteine, INF-gamma, inhibin B, insulin, insulin-like growth factor-binding protein 3 (IGF-BP3), interleukin 1 beta (IL1-beta), interleukin 15 (IL-15), interleukin 2 (IL-2), interleukin 4 (IL-4), interleukin 7 (IL-7), interleukin 8 (IL-8), isoleucin, potassium , lactaat dehydrogenase (LDH), leucin, lipase, lipoperoxyl alkoxyl, low-density lipoprotein (LDL), malondialdehyde, matrix metallopeptidase (MMP-9), monocyte chemoattractant protein-1 (MCP-1/CCL-2), sodium, neopterin, neutrophils, osteonectin (SPARC), peroxisome proliferator-activated receptor-gamma coactivator (PGC-1a), prolactin, prostaglandin 2, parathyroid hormone (PTH), pyridoxine, resistin, reactive oxygen species (ROS), reactive nitrogen species (RNS), serotonin (5-HT) receptor, serum amyloid A (SAA), serum free light chain levels, skeletal troponin I, soluble tumor necrosis factor alpha receptor type II, thiocyanate, total antioxidant capacity, total protein, trans-3'-hydroxycotinine, thrombocytes, tryptophan, tumor necrosis factor alpha (TNF-α), tyramine, valine, visfatin, vitamin A, vitamin B6, vitamin C, vitamin E, and zinc (which corresponds to the biomarkers listed in Table 3 (Figure 1) in a sample obtained from said subject.

In more particular embodiments, the fatigue syndrome is selected from the group consisting of burn-out, chronic stress, non-functional overreaching, and overtraining syndrome, and the at least three biomarkers independently selected from the group of biomarkers consisting of cystathionine, urea, amylase (preferably alpha-amylase), bio-available testosteron, creatinineDHEA-S, erythrocytes, ferritin, folic acid, free cortisol, free testosterone, IGF-1, lymphocytes, reticulocytes, sex hormone binding globulin (SHBG), TSH, total cortisol, total testosterone, hemoglobin in reticulocytes, iron, leukocytes, mean corpuscular hemoglobin (MCH), monocytes, transferrin, transferrin saturation (%), vitamin B 12 and vitamin D (which corresponds to the biomarkers listed in Table 4 (Figure 1).

In particular embodiments, the fatigue syndrome is non-functional overreaching and/or overtraining syndrome and the at least three biomarkers are independently selected from the group of biomarkers consisting of anthranilic acid, cystathionine, N1-methylnicontinamide, thiamine, bio-available testosterone, amylase (preferably alpha-amylase), creatinine, cystathionine, dDHEA-S, erythrocytes, ferritin, folic acid, free cortisol, free testosterone, IGF-1, lymphocytes, Reticulocytes, sex hormone binding globulin (SHBG), TSH, Total cortisol, Total testosterone, urea, Vitamin D, picolinic acid, kynurenic acid (KYNA), L-kynurenine, nicotinamide, nicotinic acid, QUIN, tryptophan, xanthurenic acid, 3-hydroxyanthranilic acid, 3-hydroxykynurenine, brain-derived neurotrophic factor (BDNF) and S100 calcium-binding protein B (S100B) (which corresponds to the biomarkers listed in Table 5 (Figure 1); preferably selected from the group of biomarkers consisting of cystathionine, N1-methylnicontinamide, thiamine, bio-available testosterone, amylase (preferably alpha-amylase), creatinine, DHEA-S, erythrocytes, ferritin, folic acid, free cortisol, free testosterone, IGF-1, lymphocytes, Reticulocytes, sex hormone binding globulin (SHBG), TSH, Total cortisol, Total testosterone, urea, and Vitamin D (which corresponds to the biomarkers listed in Table 6 (Figure 1).

In particular embodiments, the fatigue syndrome is chronic stress, burn-out and/or depression, preferably chronic stress or chronic mental stress, more preferably chronic stress or chronic mental stress as a precursor or the indication of the early stage (onset) of burn-out and/or depression, and the at least three biomarkers are independently selected from the group of biomarkers consisting of anthranilic acid, pyridoxal 5'-phosphate, flavin mononucleotide, IL-1ra, amylase (preferably alpha-amylase), creatinine, DHEA-S, Free cortisol, hemoglobin in reticulocytes, iron, Leukocytes, lymphocytes, MCH, monocytes, transferrin, transferrin saturation, urea, vitamin B12, Vitamin D, 3-hydroxyanthranilic acid, 3-hydroxyisobutyrate, 3-hydroxykynurenine, 4-pyridoxic acid, C-reactive protein (CRP), cystathionine, folic acid, Free testosterone, Growth hormone, IL-10, IL-6, KYNA, L-kynurenine, Magnesium, MCHC, myeloperoxidase (MPO), N1-methylnicotinamide, nicotinamide, picolinic acid, pyridoxal, quinolinic acid (QUIN), Reticulocytes, riboflavin, sIgA, Total cortisol, Triglycerides, trigonelline, vascular endothelial growth factor A (VEGF-A), xanthurenic acid, Adiponectin, alanine aminotransferase (ALT), aspartate aminotransferase (AST), basophils, bio-available testosterone, erythrocyte sedimentation rate, haptoglobin, neutrophils, and S100 calcium-binding protein B (S100B) (which corresponds to the biomarkers listed in Table 7 (Figure 1); preferably selected from the group of biomarkers consisting of anthranilic acid, pyridoxal 5'-phosphate, flavin mononucleotide, interleukin 1 receptor antagonist (IL-1ra), amylase (preferably alpha-amylase), creatinine, dehydroepiandrosterone sulfate (DHEA-S), Free cortisol, hemoglobin in reticulocytes, iron, Leukocytes, lymphocytes, MCH, monocytes, transferrin, transferrin saturation, urea, vitamin B12, Vitamin D, 3-hydroxyanthranilic acid, 3-hydroxyisobutyrate, 3-hydroxykynurenine, 4-pyridoxic acid, C-reactive protein (CRP), cystathionine, folic acid, Free testosterone, Growth hormone, IL-10, IL-6, kynurenic acid (KYNA), L-kynurenine, Magnesium, MCHC, myeloperoxidase (MPO), N1-methylnicotinamide, nicotinamide, picolinic acid, pyridoxal, quinolinic acid (QUIN), Reticulocytes, riboflavin, secretory Immunoglobulin A (sIgA), Total cortisol, Triglycerides, trigonelline, vascular endothelial growth factor A (VEGF-A), and xanthurenic acid (which corresponds to the biomarkers listed in Table 8 (Figure 1); more preferably selected from the group of biomarkers consisting of urea, creatinine, amylase (preferably alpha-amylase), dehydroepiandrosterone sulfate (DHEA-S), Free cortisol, total cortisol, hemoglobin in reticulocytes, iron, Leukocytes, lymphocytes, MCH, monocytes, transferrin, transferrin saturation, vitamin B12, and Vitamin D.

In particular embodiments, the fatigue syndrome is burn-out, and the at least three biomarkers are independently selected from the group of biomarkers consisting 3-hydroxyanthranilic acid, 4-pyridoxic acid, amylase (preferably alpha-amylase), Creatinine, dehydroepiandrosterone sulfate (DHEA-S), free cortisol, hemoglobin in reticulocytes, iron, Leukocytes, Lymphocytes, transferrin, transferrin saturation, urea, Vitamin B12, Vitamin D, 3-hydroxyisobutyrate, 3-hydroxykynurenine, adiponectin, basophils, bio-available testosterone, C-reactive protein (CRP), cystathionine, flavin mononucleotide, folic acid, free testosterone, growth hormone, IL-10, IL-6, kynurenic acid (KYNA), L-kynurenine, myeloperoxidase (MPO), N1-methylnicotinamide, nicotinamide, picolinic acid, pyridoxal, pyridoxal 5'-phosphate, quinolinic acid (QUIN), riboflavin, S100 calcium-binding protein B (S100B), secretory Immunoglobulin A (sIgA), total cortisol, triglycerides, trigonelline, brain-derived neurotrophic factor (BDNF), xanthurenic acid, alpha1 antitrypsin, $\alpha$-macroglobulin, anandamide, angiotensin-converting-enzyme (ACE) activity, apolipoprotein CIII, Calcineurin, calcitonin, chromogranin A, c-terminal agrin fragment (CAF), epidermal growth factor (EGF), E-selectin, IGF-1, IGF-BP3, IL-1ra, inhibin B, insulin, isoleucine, leucine, MCP-1/CCL-2, neopterin, nicotinic acid, osteonectin (SPARC), prostaglandin 2, pyridoxine, resistin, serum amyloid A (SAA), sex hormone binding globulin (SHBG), soluble tumor necrosis factor alpha receptor type II, Thiamine, thiamine monophosphate, TNF-$\alpha$, total testosterone, trans-3'-hydroxycotinine, tryptophan, valine, visfatin, and $\gamma$-amuniobutyric acid (GABA) (which corresponds to the biomarkers listed in Table 9 (Figure 1); preferably selected from the group of biomarkers consisting of amylase (preferably alpha-amylase), Creatinine, dehydroepiandrosterone sulfate (DHEA-S), free cortisol, hemoglobin in reticulocytes, iron, Leukocytes, Lymphocytes, transferrin, transferrin saturation, urea, Vitamin B12, Vitamin D, 3-hydroxyanthranilic acid, 3-hydroxyisobutyrate, 3-hydroxykynurenine, 4-pyridoxic acid, adiponectin, basophils, bio-available testosterone, C-reactive protein (CRP), cystathionine, flavin mononucleotide, folic acid, free testosterone, growth hormone, IL-10, IL-6, kynurenic acid (KYNA), L-kynurenine, myeloperoxidase (MPO), N1-methylnicotinamide, nicotinamide, picolinic acid, pyridoxal, pyridoxal 5'-phosphate, quinolinic acid (QUIN), riboflavin, S100 calcium-binding protein B (S100B), secretory Immunoglobulin A (sIgA), total cortisol, triglycerides, trigonelline, brain-derived neurotrophic factor (BDNF) and xanthurenic acid; more preferably selected from the group of biomarkers consisting of amylase (preferably alpha-amylase), Creatinine, dehydroepiandrosterone sulfate (DHEA-S), free cortisol, hemoglobin in reticulocytes, iron, Leukocytes, Lymphocytes, transferrin, transferrin saturation, urea, Vitamin B12 and Vitamin D (which corresponds to the biomarkers listed in Table 10 (Figure 1).

In particular embodiments, the fatigue syndrome is depression and the at least three biomarkers are independently selected from the group of biomarkers consisting of Riboflavin, Visfatin, amylase (preferably alpha-amylase), estimated glomular filtration rate (eGFR), C-reactive protein (CRP), Triglycerides, adiponectin, alanine aminotransferase (ALT), albumin, alkaline phosphatase, Alpha1 antitrypsin, Apolipoprotein CIII, aspartate aminotransferase (AST), BCAA, Brain-derived neurotrophic factor (BDNF), CBG, ceruloplasmin, cholesterol, co-enzym Q10, Cortisol, Cotinine, c-peptide, dehydroepiandrosterone sulfate (DHEA-S), dopamine, Eotaxin, Epidermal growth factor (EGF), Erythrocytes, estradiol, ferritin, folic acid, free fatty acids, free T3/T4, free testosterone, Free tryptophan, GABA, gamma-glutamyl transpeptidase, gamma-OH-boterzuur, ghrelin, glucose, glutamaat, glutamaat receptor, glutamine, glutathione (GSH), glutathione disulfide (GSSG), glutathione peroxidase (GXP), glycogen, Growth hormone, Haptoglobin, HDL, Hematocrit, hemoglobin, homocysteine, HSP70, HSP72, IGF-1, IGF-BP3, IL-10, IL-15, IL1-beta, IL-1ra, IL-2, IL-4, IL-6, IL-7, IL-8, INF-gamma, inhibin B, insulin, iron, potassium, kynurenic acid (KYNA), lactaat dehydrogenase (LDH), LDL, Leukocytes, Lipase, lipoperoxyl alkoxyl, L-kynurenine, Magnesium, Malondialdehyde, MCP-1/CCL-2, MMP-9, Myeloperoxidase (MPO), sodium, Neopterin, Nicotinamide, PGC-1a, Picolinic acid, Prolactin, Prostaglandin 2, PTH (parathyroid hormone), Quinolonic acid (QUIN), Resistin, ROS/RNS, S100 calcium-binding protein B (S100B), secretory Immunoglobulin A (sIgA), serotonin (5-HT) receptor, serum amyloid A (SAA), Serum free light chain, sex hormone binding globulin (SHBG), skeletal troponin I, Soluble tumor necrosis factor alpha receptor type II, Thiamine, Thiocyanate, thyroid-stimulating hormone (TSH), TNF-$\alpha$, total antioxidant capacity, Total cortisol, total protein, total testosterone, transferrin, Thrombocytes, Tyramine, urea, vitamin A, vitamin B12, Vitamin B6, Vitamin C, vitamin D, Vitamin E and zinc (which corresponds to the biomarkers listed in Table 11 (Figure 1).

In particular embodiments, the *in vitro* method for predicting, diagnosing, prognosing and/or monitoring a fatigue syndrome in a subject or for determining whether a subject is in need of therapeutic or prophylactic treatment of a fatigue syndrome in a subject is a method for detecting the early stage (onset) and/or the existence of a fatigue syndrome in a subject.

In particular embodiments, the *in vitro* method for predicting, diagnosing, prognosing and/or monitoring a fatigue syndrome in a subject is a method for detecting the early stage (onset) of burn-out and/or depression in a subject.

In particular embodiments, the fatigue syndrome is chronic mental stress, preferably wherein chronic mental stress is the mental status prior to development of mood disorders, such as depression or burn-out, and the method comprises detecting at least three, preferably at least four, more preferably at least five, even more preferably at least six, biomarkers independently selected from at least two, preferably at least three, more preferably at least four, even more preferably at least five, different groups of biomarkers selected from:

- a group of immune function biomarkers consisting of neopterin, sIgA, IL-6, IL-10, IL-1RA, EGF, leukocytes, neutrophils, lymphocytes, monocytes, basophils, CRP and MPO (also referred to herein as biomarker group 100);

- a group of iron metabolism biomarkers consisting of ferritin, transferrin, iron, transferrin saturation, hemoglobin in reticulocytes, erythrocytes, MCH, MCV, MCHC and reticulocytes (also referred to herein as biomarker group 200);

- a group of metabolism biomarkers consisting of creatinine, urea, vitamin D, folic acid, riboflavin, vitamin B12, 4-pyridoxic acid, flavin mononucleotide, thiamine, nicotinamide, pyridoxal 5'-phosphate, N1-methylnicotinamide, trigonelline, thiamine monophosphate, and visfatin (also referred to herein as biomarker group 300));

- a group of steroid hormone biomarkers consisting of DHEA-S, SHBG, bio-available testosterone, total testosterone, free testosterone, and DHEA (also referred to herein as biomarker group 400); and

- a group of sympathetic adrenal medullary axis biomarkers consisting of amylase (preferably alpha-amylase), epinephrine, and norepinephrine (also referred to herein as biomarker group 500); and

- optionally a group of neurotransmitter biomarkers consisting of anthranilic acid, cystathionine, picolinic acid, KYNA, L-kynurenine, QUIN, free tryptophan, xanthurenic acid, BDNF, S100B, and GABA (also referred to herein as biomarker group 600);

in said sample obtained from said subject, preferably wherein the at least three, preferably at least four, more preferably at least five, even more preferably at least six, biomarkers are independently selected from at least two, preferably at least three, more preferably at least four, even more preferably at least five, different groups of biomarkers selected from the biomarker group 100, the biomarker group 200, the biomarker group 300, the biomarker group 400 or the biomarker group 500.

Several biomarkers of the group of immune function biomarkers (e.g. neopterin, sIgA, IL-6, IL-10, IL-1RA, and C-reactive protein (CRP)), the group of iron metabolism biomarkers (e.g. transferrin, iron, transferrin saturation, and ferritin), the group of metabolism biomarkers (e.g. creatinine, urea, vitamin D, folic acid, riboflavin, vitamin B12, 4-pyridoxic acid, pyridoxal 5'-phosphate, N1-methylnicotinamide, and visfatin), the group of steroid hormone biomarkers (e.g. DHEA-S and free testosterone) and the group of sympathetic adrenal medullary axis biomarkers (e.g. amylase, preferably alpha-amylase) are of particular interest as (i) they can be determined by easy sampling conditions (e.g. the biomarkers do not require fresh blood or cold sample storage); and (ii) they can be measured with routine, low-cost, analytical techniques (such as enzymatic or immunoassays), and do not require, for example, expensive analytical techniques such as liquid chromatography-mass spectrometry (LC-MS).

Accordingly, in particular embodiments, the fatigue syndrome in chronic mental stress, preferably wherein chronic mental stress is the mental status prior to development of mood disorders, such as depression or burn-out, and the method comprises detecting at least three, preferably at least four, more preferably at least five, even more preferably at least six, biomarkers independently selected from at least two, preferably at least three, more preferably at least four, even more preferably at least five, different groups of biomarkers selected from:

- a group of immune function biomarkers consisting of neopterin, sIgA, IL-6, IL-10, IL-1RA, and CRP (also referred to herein as biomarker group 100a);

- a group of iron metabolism biomarkers consisting of transferrin, iron, transferrin saturation, and ferritin (also referred to herein as biomarker group 200a);

- a group of metabolism biomarkers consisting of creatinine, urea, vitamin D, folic acid, riboflavin, vitamin B12, 4-pyridoxic acid, pyridoxal 5'-phosphate, N1-methylnicotinamide, and visfatin (also referred to herein as biomarker group 300a);

- a group of steroid hormone biomarkers consisting of DHEA-S and free testosterone (also referred to herein as biomarker group 400a); and

- a group of sympathetic adrenal medullary axis biomarkers consisting of amylase, preferably alpha-amylase (also referred to herein as biomarker group 500a).

In particular embodiments, the fatigue syndrome in chronic mental stress, preferably wherein chronic mental stress is the mental status prior to development of mood disorders, such as depression or burn-out, and the method comprises detecting at least four biomarkers independently selected from at least four different groups of biomarkers selected from: biomarker group 100, biomarker group 200, biomarker group 300, biomarker group 400, biomarker group 500, and optionally biomarker group 600;
preferably selected from: biomarker group 100a, biomarker group 200a, biomarker group 300a, biomarker group 400a and biomarker group 500a, as described elsewhere herein.

For example, but the method as taught herein is not to be considered limited thereto, the fatigue syndrome in chronic mental stress, preferably wherein chronic mental stress is the mental status prior to development of mood disorders, such as depression or burn-out, and the method comprises detecting:

- Vitamin B12, Vitamin D, Iron, Transferrin, Lymphocytes, DHEA-S, Urea, Creatinine, and Amylase;
- Vitamin B 12, Vitamin D, Iron, Transferrin, Lymphocytes, and Amylase;
- Transferrin saturation, DHEA-S, Urea, and Amylase;
- DHEA-S, Transferrin saturation, Vitamin D, Urea, Creatinine and Amylase;
- IL-1RA, 4-Pyridoxic acid, and Riboflavin;
- Amylase, 4-Pyridoxic acid, iron, and transferrin; or
- Transferrin, Amylase, 4-Pyridoxic acid, and Free testosterone,

in said sample obtained from said subject.

In contrast to previous methods used to diagnose the syndromes and disorders referred to in the current invention, which were often based on the individuals' perception of symptoms, the method as taught herein is based on a combination of biomarkers, a biosignature, whose status can be accurately, objectively and reproducibly measured. As such, the methodology is objective.

Different types of biomarkers can be used in the method as taught herein, usually biomarkers are classified on their clinical applications and, in the case of the invention described herein, include but are not limited to:

- Molecular biomarkers such as genomic biomarkers, transcriptomic biomarkers, proteomic biomarkers and metabolic biomarkers.
- Cellular biomarkers allow cells to be isolated, sorted, quantified and characterized by their morphology and physiology. Cellular biomarkers can discriminate between a large sample of cells based on their antigens

- Feature or characteristics biomarkers include indicators with complex characteristics that can be influenced by multiple factors but whose status can be objectively, reproducibly and accurately determined. Characteristics biomarkers used in the method according to the current invention include, but are not limited to, for example the erythrocyte sedimentation rate.

A biomarker is "detected" in a sample when the presence, absence and/or quantity (or amount), preferably the quantity, of said biomarker is detected or determined in the sample, preferably substantially to the exclusion of other molecules and analytes.

Depending on factors that can be evaluated and decided on by a skilled person, such as *inter alia* the type of a biomarker (e.g., molecular biomarker, cellular biomarker, feature or characteristics biomarkers), the type of a sample, the expected abundance of the biomarker in the sample, the type, robustness, sensitivity and/or specificity of the detection method used to detect the biomarker, etc., the biomarker may be measured directly in the sample, or the sample may be subjected to one or more processing steps aimed at achieving an adequate measurement of the biomarker.

By means of example, the sample may be subjected to one or more isolation or separation steps, aimed at whereby the biomarker is isolated from the sample or whereby a fraction of the sample is enriched for the biomarker. For example, if the biomarker is a peptide, polypeptide, or protein, any known protein purification technique may be applied to the sample to isolate peptides, polypeptides, and proteins therefrom. If the biomarker is a nucleic acid, any known nucleic acid purification technique may be applied to the sample to isolate nucleic acids therefrom. Non-limiting examples of methods to purify peptides, polypeptides, proteins, or nucleic acids may include chromatography, preparative electrophoresis, centrifugation, precipitation, affinity purification, etc.

In particular embodiments, detecting the at least three biomarkers in a sample comprises determining the status of the at least three biomarkers, wherein said status relates to the presence, absence and/or the amount of said biomarkers.

Depending on their nature, biomarkers can be measured using various different methods known to the person skilled in the art. These methods include, but are not limited to, immunological assays, mass spectrometry-based methodologies and biochemical assays. Proteomic biomarkers and peptide hormones are in most cases measured using methods based on immunoassays or on mass spectrometry. Genetic biomarkers and transcriptomic biomarkers are in most cases measured using PCR-based methods or biochemical assays. Metabolic biomarkers are most often measured using biochemical assays and mass-spectrometry based methods. In addition, fluorescence-based assays - based on the detection of fluorescent dyes - are often used in the detection of all kinds of biomarkers and are especially suited for the detection of cellular biomarkers. It goes without saying that the list of methods given here is a non-exhaustive list of examples and that the method according to the invention may implement other assays, known to the skilled person, suited to measure the status of the biomarkers as currently disclosed.

The method as taught herein provides an early screening procedure that may be carried out on a sample obtained from a subject, before the subject presents symptoms or before he is aware of any symptoms. The method thus allows for detection of the earliest stages of the fatigue syndrome, such as NFO/OTS, burn-out and/or depression, allowing an adaptation of the training, work-load or lifestyle to avoid the development of NFO/OTS, the worsening of NFO/OTS or the development of CFS or burn-out which could eventually result in depression. For example, the methods as taught herein allow for detection of chronic mental stress prior to the development of mood disorders, such as burn-out or depression. Eventually, early diagnosis of these syndromes/disorders results in a better standard of living as they can then be adequately treated before they have a more detrimental effect on the individual's health.

Another advantage of the method according to the invention is that early diagnosis in professional athletes can be used to prevent disappointing performance by introducing more recovery time in between trainings, resulting in a positive effect on the athlete's career. The method of the invention, therefore, provides the opportunity for athletes, and their coaches, to monitor training effects and performance such that early intervention may be made to the athletes' training schedules before the development of OTS and the serious and long term symptoms associated with it.

Furthermore, recreational athletes who often combine their job with intensive training also benefit from the current invention. The combination of training, work and social activities is very challenging, and involves a notable amount of stress which impacts both physical performance at sport as well as performance at work. The current invention allows medical doctors, training coaches, employers and athletes themselves to objectively monitor the resilience of the athletes, enabling them to interfere with the different stressors in time, therefore avoiding the possible negative impact of a failure to accomplish a sport challenge in a satisfying manner on their future sports performances, their mental health and their performance at work.

In particular embodiments, such as in an *in vitro* method for predicting, diagnosing, prognosing and/or monitoring non-functional overreaching and/or overtraining syndrome, the subject is an athlete, preferably a triathlon athlete, a cyclist, a runner, a rower or a soccer player.

In particular embodiments, such as in an *in vitro* method for predicting, diagnosing, prognosing and/or monitoring chronic stress (preferably chronic mental stress), burn-out and/or depression, the subject is an employee.

In addition to early diagnostic screening, the method according to the current invention also offers the advantage that reliable and accurate diagnosis of the fatigue syndrome, such as chronic mental stress, NFO/OTS, burn-out syndrome and/or depression disorder, avoids the need of running a large number of other medical tests in order to exclude other causes. The method may be particularly useful in the screening of employees for e.g. the detection of chronic mental stress prior the development of a mood disorder, such as burn-out or depression, or the detection of the onset of burnout.

In a particular embodiment, the method as taught herein comprises detecting at least 3 (such as 3, 4, 5, 6, 7, 8, 9, 10, 11, 12, 13, 14, 15, 16, 17, 18, 19, 20, 21, 22, 23, 24 or 25), at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, or at least 25 biomarkers of a group of biomarkers as described herein, such as the group of biomarkers of Tables 1, 2, 3, 4, 5, 6, 7, 8, 9, 10, or 11. More by preference, the status of at least 4, even more by preference, at least 5 of said biomarkers is determined. Even more by preference, the status of at least 6, more by preference, at least 7 of said biomarkers is determined. Most by preference, the status of at least 8 to 10 of said biomarkers is determined.

In a particular embodiment, the fatigue syndrome in chronic mental stress, preferably wherein chronic mental stress is the mental status prior to development of mood disorders, such as depression or burn-out, and the method comprises detecting from 3 to 15, such 3, 4, 5, 6, 7, 8, 9, 10, 11, 12, 13, 14, 15, from 3 to 9, from 3 to 10, from 4 to 15, from 5 to 15, from 6 to 15, from 7 to 15, from 8 to 15, from 9 to 15, from 10 to 15, from 11 to 15, from 12 to 15, or from 13 to 15, biomarkers of at least two, preferably at least three, more preferably at least four, even more preferably at least five, different group of biomarkers selected from the biomarker group 100, the biomarker group 200, the biomarker group 300, the biomarker group 400, the biomarker group 500, and optionally the biomarker group 600, as described elsewhere herein.

In a particular embodiment, the fatigue syndrome in chronic mental stress, preferably wherein chronic mental stress is the mental status prior to development of mood disorders, such as depression or burn-out, and the method comprises detecting from 3 to 15, such 3, 4, 5, 6, 7, 8, 9, 10, 11, 12, 13, 14, 15, from 3 to 9, from 3 to 10, from 4 to 15, from 5 to 15, from 6 to 15, from 7 to 15, from 8 to 15, from 9 to 15, from 10 to 15, from 11 to 15, from 12 to 15, or from 13 to 15, biomarkers of at least two, preferably at least three, more preferably at least four, even more preferably at least five, different group of biomarkers selected from the biomarker group 100a, the biomarker group 200a, the biomarker group 300a, the biomarker group 400a, and the biomarker group 500a, as described elsewhere herein.

In particular embodiments, the fatigue syndrome is non-functional overreaching and/or overtraining syndrome, preferably non-functional overreaching, and at least three biomarkers are independently selected from at least two, preferably at least three, more preferably at least four, even more preferably at least 5, different groups of biomarkers selected from:

- a group of neurotransmitter biomarkers consisting of anthranilic acid, cystathionine, picolinic acid, kynurenic acid (KYNA), L-kynurenine, quinolinic acid (QUIN), free tryptophan, xanthurenic acid, brain-derived neurotrophic factor (BDNF), S100 calcium-binding protein B (S100B), γ-aminobutyric acid (GABA), 3-hydroxyanthranilic acid, 3-hydroxykynurenine, Magnesium, Zinc, Glutamine, glutamaat, calcineurin, glutamate receptor, indoleamine (2,3)-dioxygenase (IDO), kynurenine mono-oxygenase, kynureninase, and kynurenine transaminase (KAT) (also referred to as group 1 or group 7); preferably consisting of anthranilic acid, cystathionine, picolinic acid, kynurenic acid (KYNA), L-kynurenine, quinolinic acid (QUIN), free tryptophan, xanthurenic acid, brain-derived neurotrophic factor (BDNF), S100 calcium-binding protein B (S100B), and γ-amuniobutyric acid (GABA) (also referred to as group 1a or group 7a); more preferably consisting of anthranilic acid, cystathionine, picolinic acid, xanthurenic acid, and S100 calcium-binding protein B (S100B) (also referred to as group 1b);

- a group of metabolism biomarkers consisting of N1-methylnicotinamide, flavin mononucleotide, creatinine, urea, vitamin D, folic acid, riboflavin, vitamin B12, pyridoxine, pyridoxic acid, thiamine, nicotinic acid, nicotinamide, pyridoxal 5'-phosphate, niacin (vitamin B3), N1-methylnicotinamide, pyridoxal, trigonelline, thiamine monophosphate, visfatin, and cholecalciferol (also referred to as group 2 or group 10); preferably consisting of flavin mononucleotide, creatinine, urea, vitamin D, folic acid, riboflavin, vitamin B12, pyridoxine, 4-pyridoxic acid, thiamine, nicotinic acid, nicotinamide, pyridoxal 5'-phosphate and niacin (vitamin B3) (also referred to as group 2a or group 10a); more preferably consisting of creatinine, urea, vitamin D, riboflavin, vitamin B12, 4-pyridoxic acid, flavin mononucleotide, thiamine, nicotinamide, and pyridoxal 5'-phosphate (also referred to as group 2b);

- a group of steroid hormone biomarkers consisting of dehydroepiandrosterone sulfate (DHEA-S), sex hormone binding globulin (SHBG), bio-available testosterone, total testosterone, free testosterone, and dehydroepiandrosterone (DHEA) (also referred to as group 3 or group 11); preferably consisting of dehydroepiandrosterone sulfate (DHEA-S), sex hormone binding globulin (SHBG), bio-available testosterone, total testosterone, and free testosterone (also referred to as group 3a or group 11a); more preferably consisting of DHEA-S and free testosterone (also referred to as group 3b);

- a group of thyroid hormone biomarkers consisting of insulin-like growth factor (IGF-1), thyroid-stimulating hormone (TSH), insulin-like growth factor binding protein 3 (IGF-BP3), calcitonin, triiodothyronine (T3) and thyroxine (T4) (also referred to as group 4); preferably consisting of insulin-like growth factor (IGF-1) and thyroid-stimulating hormone (TSH) (also referred to as group 4a);

- a group of iron metabolism biomarkers consisting of ferritin, transferrin, iron, transferrin saturation, hemoglobin in reticulocytes, erythrocytes, mean cell haemoglobin (MCH), mean cell volume (MCV), mean cell haemoglobin concentration (MCHC) and reticulocytes (also referred to as group 5); preferably consisting of reticulocytes, iron, transferrin, and ferritin(also referred to as group 5a) and

- a group of hypothalamic-pituitary-adrenal (HPA) axis hormone biomarkers consisting of total cortisol, free cortisol, cortisol releasing hormone (CRH) and adrenocorticotropic hormone (ACTH) (also referred to as group 6), preferably free cortisol or total cortisol (also referred to as group 6a).

Also encompassed by present invention are all combinations of at least three biomarkers independently selected from at least two, preferably at least three, more preferably at least four, different groups of biomarkers selected from groups 1, 2, 3, 4, 5 and 6.

In particular embodiments, the fatigue syndrome is non-functional overreaching and/or overtraining syndrome, preferably non-functional overreaching, and at least three biomarkers are independently selected from at least two, preferably at least three, more preferably at least four, even more preferably at least 5, different groups of biomarkers selected from biomarker groups 1a, 2a, 3a, 4a, 5a, 6a as described above; preferably selected from biomarker groups 1b, 2b, 3b, 4a, 5a, 6a as described above.

For example, a group of 3 biomarkers consisting of the group of anthranilic acid (groups 1 and 1a), bioavailable testosterone (groups 3 and 3a), and TSH (groups 4 and 4a) can be used for predicting, diagnosing, prognosing and/or monitoring non-functional overreaching or overtraining syndrome in a subject.

In particular embodiments, the fatigue syndrome is non-functional overreaching and/or overtraining syndrome, preferably non-functional overreaching, and the method as taught herein comprises detecting at least reticulocytes, Testosterone (free or bioavailable), Cortisol, IGF-1, DHEA-S, Creatinin, Vitamin D and Erythrocytes.

In particular embodiments, the fatigue syndrome is non-functional overreaching or overtraining syndrome and at least four biomarkers are independently selected from at least two, preferably at least three, more preferably at least four, different groups of biomarkers selected from biomarker groups 1, 1a, 1b, 2, 2a, 2b, 3, 3a, 3b, 4, 4a, 5, 5a, 6 and 6a as described elsewhere herein.

In particular embodiments, the fatigue syndrome is non-functional overreaching or overtraining syndrome and at least five biomarkers are independently selected from at least two, preferably at least three, more preferably at least four, different groups of biomarkers selected from biomarker groups 1, 1a, 1b, 2, 2a, 2b, 3, 3a, 3b, 4, 4a, 5, 5a, 6 and 6a as described elsewhere herein.

In particular embodiments, the fatigue syndrome is non-functional overreaching or overtraining syndrome and at least six biomarkers are independently selected from at least two, preferably at least three, more preferably at least four, different groups of biomarkers selected from biomarker groups 1, 1a, 1b, 2, 2a, 2b, 3, 3a, 3b, 4, 4a, 5, 5a, 6 and 6a as described elsewhere herein.

In particular embodiments, the fatigue syndrome is non-functional overreaching or overtraining syndrome and at least seven biomarkers are independently selected from at least two, preferably at least three, more preferably at least four, different groups of biomarkers selected from biomarker groups 1, 1a, 1b, 2, 2a, 2b, 3, 3a, 3b, 4, 4a, 5, 5a, 6 and 6a as described elsewhere herein.

In particular embodiments, the fatigue syndrome is non-functional overreaching or overtraining syndrome and at least eight biomarkers are independently selected from at least two, preferably at least three, more preferably at least four, different groups of biomarkers selected from biomarker groups 1, 1a, 1b, 2, 2a, 2b, 3, 3a, 3b, 4, 4a, 5, 5a, 6 and 6a as described elsewhere herein.

In particular embodiments, the *in vitro* method for predicting, diagnosing, prognosing and/or monitoring a fatigue syndrome in a subject is a method for detecting the early stage (onset) of burn-out and/or depression in a subject and/or the *in vitro* method for predicting, diagnosing, prognosing and/or monitoring a fatigue syndrome in a subject wherein the fatigue syndrome is chronic stress, burn-out and/or depression, preferably chronic stress or chronic mental stress, more preferably chronic stress or chronic mental stress as a precursor or the indication of the early stage (onset) of burn-out and/or depression, and at least three biomarkers are independently selected from at least two, preferably at least three, more preferably at least four, even more preferably at least five, different groups of biomarkers selected from:

- a group of neurotransmitter biomarkers consisting of anthranilic acid, cystathionine, picolinic acid, kynurenic acid (KYNA), L-kynurenine, quinolinic acid (QUIN), free tryptophan, xanthurenic acid, brain-derived neurotrophic factor (BDNF), S100 calcium-binding protein B (S100B), $\gamma$-aminobutyric acid (GABA), 3-hydroxyanthranilic acid, 3-hydroxykynurenine, Magnesium, Zinc, glutamine, glutamaat, calcineurin, glutamate receptor, indoleamine (2,3)-dioxygenase (IDO), kynurenine mono-oxygenase, kynureninase, and kynurenine transaminase (KAT) (also referred to herein as group 7 or group 1); preferably consisting of cystathionine, anthranilic acid, picolinic acid, kynurenic acid (KYNA), L-kynurenine, quinolinic acid (QUIN), free tryptophan, xanthurenic acid, brain-derived neurotrophic factor (BDNF), S100 calcium-binding protein B (S100B), and y-amuniobutyric acid (GABA) (also referred to herein as group 7a or group 1a); more preferably consisting of anthranilic acid, cystathionine, picolinic acid, quinolinic acid (QUIN), xanthurenic acid (also referred to herein as group 7b);

- a group of immune function biomarkers consisting of neopterin, secretory immunoglobulin A (sIgA), interleukin 6 (IL-6), interleukin 10 (IL-10), interleukin-1 receptor A (IL-1RA), epidermal growth factor (EGF), leukocytes, neutrophils, lymphocytes, monocytes, basophils, C-reactive protein (CRP) and myeloperoxidase (MPO) (also referred to herein as group 8); preferably consisting of neopterin, secretory immunoglobulin A (sIgA), interleukin 6 (IL-6), interleukin 10 (IL-10), interleukin-1 receptor A (IL-1RA), C-reactive protein (CRP) (also referred to herein as group 8a);

- a group of iron metabolism biomarkers consisting of ferritin, transferrin, iron, transferrin saturation, hemoglobin in reticulocytes, erythrocytes, mean cell haemoglobin (MCH), mean cell volume (MCV), mean cell haemoglobin concentration (MCHC) and reticulocytes (also referred to herein as group 9); preferably consisting of transferrin, iron, transferrin saturation, mean cell volume (MCV) (also referred to herein as group 9a);

- a group of metabolism biomarkers consisting of creatinine, urea, vitamin D, folic acid, riboflavin, vitamin B12, pyridoxine, 4-pyridoxic acid, flavin mononucleotide, thiamine, nicotinic acid, nicotinamide, pyridoxal 5'-phosphate, niacin (vitamin B3) N1-methylnicotinamide, pyridoxal, trigonelline, thiamine monophosphate, visfatin, and cholecalciferol (also referred to as group 10 or group 2); preferably consisting of creatinine, urea, vitamin D, folic acid, riboflavin, vitamin B12, pyridoxine, 4-pyridoxic acid, flavin mononucleotide, thiamine, nicotinic acid, nicotinamide, and pyridoxal 5'-phosphate and niacin (vitamin B3) (also referred to as group 10a or group 2a); more preferably consisting of creatinine, urea, vitamin D, riboflavin, vitamin B12,4- pyridoxic acid, flavin mononucleotide, thiamine,nicotinamide, and pyridoxal 5'-phosphate (also referred to as group 10b);
  - - - a group of steroid hormone biomarkers consisting of dehydroepiandrosterone sulfate (DHEA-S), sex hormone binding globulin (SHBG), bio-available testosterone, total testosterone, free testosterone, and dehydroepiandrosterone (DHEA) (also referred to as group 11 or group 3); preferably consisting of dehydroepiandrosterone sulfate (DHEA-S), sex hormone binding globulin (SHBG), bio-available testosterone, total testosterone, and free testosterone (also referred to herein as group 11a or group 3a); more preferably consisting of DHEA-S and free testosterone (also referred to herein as group 11b); and

- a group of sympathetic adrenal medullary (SAM) biomarkers consisting of amylase (preferably alpha-amylase), epinephrine, and norepinephrine (also referred to herein as group 12), preferably amylase (also refered to herein as group 12a).

Also encompassed by present invention are all combinations of at least three biomarkers independently selected from at least two, preferably at least three, more preferably at least four, different groups of biomarkers selected from groups 7, 8, 9, 10, 11 and 12.

As described elsewhere herein, in preferred embodiments, if the fatigue syndrome in chronic mental stress, preferably wherein chronic mental stress is the mental status prior to development of mood disorders, such as depression or burn-out, the method comprises detecting at least three biomarkers independently selected from at least two, preferably at least three, more preferably at least four, even more preferably at least five, different groups of biomarkers selected from: biomarker group 100, biomarker group 200, biomarker group 300, biomarker group 400, biomarker group 500, and optionally biomarker group 600; preferably selected from: biomarker group 100a, biomarker group 200a, biomarker group 300a, biomarker group 400a and biomarker group 500a.

In particular embodiments, the fatigue syndrome is non-functional overreaching and/or overtraining syndrome, preferably non-functional overreaching, and at least three biomarkers are independently selected from at least two, preferably at least three, more preferably at least four, even more preferably at least 5, different groups of biomarkers selected from biomarker groups 7a, 8a, 9a, 10a, 11a, and 12a as described above; preferably selected from biomarker groups 7b, 8a, 9a,10b, 11b and 12a as described above.

For example, a group of 4 biomarkers consisting of the group of 4-Pyridoxic acid (groups 10 or 10a), amylase (group 12), iron (group 9), transferrin (group 9) can be used for predicting, diagnosing, prognosing and/or monitoring chronic stress, such as chronic stress as a precursor of burn-out and/or depression, in a subject.

In particular embodiments, the fatigue syndrome is chronic stress, burn-out and/or depression, preferably chronic stress or chronic mental stress, more preferably chronic stress or chronic mental stress as a precursor or the indication of the early stage (onset) of burn-out and/or depression, and the method as taught herein comprises detecting at least Vitamin B12, Vitamin D, Iron, Transferrin, Lymphocytes, DHEA-S, Urea, Creatinine, and Amylase.

In particular embodiments, the fatigue syndrome is chronic stress, burn-out and/or depression, preferably chronic stress or chronic mental stress, more preferably chronic stress or chronic mental stress as a precursor or the indication of the early stage (onset) of burn-out and/or depression, and at least four biomarkers are independently selected from at least two, preferably at least three, more preferably at least four, different groups of biomarkers selected from biomarker groups 7, 7a, 7b, 8, 8a, 9, 9a, 10, 10a, 10b, 11, 11a, 11b and 12 as described elsewhere herein.

In particular embodiments, the fatigue syndrome is chronic stress, burn-out and/or depression, preferably chronic stress or chronic mental stress, more preferably chronic stress or chronic mental stress as a precursor or the indication of the early stage (onset) of burn-out and/or depression, and at least five biomarkers are independently selected from at least two, preferably at least three, more preferably at least four, different groups of biomarkers selected from biomarker groups 7, 7a, 7b, 8, 8a, 9, 9a, 10, 10a, 10b, 11, 11a, 11b and 12 as described elsewhere herein.

In particular embodiments, the fatigue syndrome is chronic stress, burn-out and/or depression, preferably chronic stress or chronic mental stress, more preferably chronic stress or chronic mental stress as a precursor or the indication of the early stage (onset) of burn-out and/or depression, and at least six biomarkers are independently selected from at least two, preferably at least three, more preferably at least four, different groups of biomarkers selected from biomarker groups 7, 7a, 7b, 8, 8a, 9, 9a, 10, 10a, 10b, 11, 11a, 11b and 12 as described elsewhere herein.

In particular embodiments, the fatigue syndrome is chronic stress, burn-out and/or depression, preferably chronic stress or chronic mental stress, more preferably chronic stress or chronic mental stress as a precursor or the indication of the early stage (onset) of burn-out and/or depression, and at least seven biomarkers are independently selected from at least three, preferably at least two, preferably at least three, more preferably at least four, different groups of biomarkers selected from biomarker groups 7, 7a, 7b, 8, 8a, 9, 9a, 10, 10a, 10b, 11, 11a, 11b and 12 as described elsewhere herein.

In particular embodiments, the fatigue syndrome is chronic stress, burn-out and/or depression, preferably chronic stress or chronic mental stress, more preferably chronic stress or chronic mental stress as a precursor or the indication of the early stage (onset) of burn-out and/or depression, and at least eight biomarkers are independently selected from at least two, preferably at least three, more preferably at least four, different groups of biomarkers selected from biomarker groups 7, 7a, 7b, 8, 8a, 9, 9a, 10, 10a, 10b, 11, 11a, 11b and 12 as described elsewhere herein.

In particular embodiments, the fatigue syndrome is chronic stress, burn-out and/or depression, preferably chronic stress or chronic mental stress, more preferably chronic stress or chronic mental stress as a precursor or the indication of the early stage (onset) of burn-out and/or depression, and at least nine biomarkers are independently selected from at least three, preferably at least four, more preferably at least five, different groups of biomarkers selected from biomarker groups 7, 7a, 7b, 8, 8a, 9, 9a, 10, 10a, 10b, 11, 11a, 11b and 12 as described elsewhere herein.

Groups indicated by "group na" are subgroups from the groups "group $n$", wherein $n$ is the same integer. Accordingly, selecting biomarkers from at least two different groups of biomarkers does not encompass selecting biomarkers only from "group $n$" and "group na", wherein $n$ is the same integer, for example such as selecting biomarkers only from "group 1" and "group 1a".

The person skilled in the art will understand that if a subject from which the sample is obtained is subjected to pharmacotherapy which is known to severely affect the status (e.g. presence, absence or quantity) of one of the biomarkers as described herein, this biomarker is preferably not one of the at least three biomarkers to be detected in the method as taught herein.

Accordingly, in particular embodiments, when the subject is subjected to a hormonal treatment, such as hormonal contraception or cortisol, the at least three biomarkers are preferably not selected from the group of hypothalamic-pituitary-adrenal (HPA) axis hormone biomarkers consisting of cortisol, cortisol releasing hormone (CRH) and adrenocorticotropic hormone (ACTH) (also referred to as group 6).

Detection of at least three biomarkers belonging to at least two, preferably at least three different groups of biomarkers according to the grouping described above enriches the diagnostic performance of the biomarkers resulting in enhanced diagnostic accuracy.

The terms "predicting" or "prediction", "diagnosing" or "diagnosis" and "prognosticating" or "prognosis" are commonplace and well-understood in medical and clinical practice. It shall be understood that the phrase "a method for the diagnosis, prediction and/or prognosis" a given disease or condition, such as chronic mental stress, may also be interchanged with phrases such as "a method for diagnosing, predicting and/or prognosticating" of said disease or condition, such as chronic mental stress, or "a method for making (or determining or establishing) the diagnosis, prediction and/or prognosis" of said disease or condition, such as chronic mental stress, or the like.

By means of further explanation and without limitation, "predicting" or "prediction" generally refer to an advance declaration, indication or foretelling of a disease or condition, such as chronic mental stress, in a subject not (yet) having said disease or condition. For example, a prediction of a disease or condition in a subject may indicate a probability, chance or risk that the subject will develop said disease or condition, for example within a certain time period or by a certain age. Said probability, chance or risk may be indicated *inter alia* as an absolute value, range or statistics, or may be indicated relative to a suitable control subject or subject population (such as, e.g., relative to a general, normal or healthy subject or subject population). Hence, the probability, chance or risk that a subject will develop a disease or condition may be advantageously indicated as increased or decreased, or as fold-increased or fold-decreased relative to a suitable control subject or subject population. As used herein, the term "prediction" of the conditions or diseases as taught herein in a subject may also particularly mean that the subject has a 'positive' prediction of such, *i.e.,* that the subject is at risk of having such (e.g., the risk is significantly increased vis-à-vis a control subject or subject population). The term "prediction of no" diseases or conditions as taught herein as described herein in a subject may particularly mean that the subject has a 'negative' prediction of such, *i.e.,* that the subject's risk of having such is not significantly increased vis-à-vis a control subject or subject population.

The terms "diagnosing" or "diagnosis" generally refer to the process or act of recognising, deciding on or concluding on a disease or condition, such as chronic mental stress, in a subject on the basis of symptoms and signs and/or from results of various diagnostic procedures (such as, for example, from knowing the presence, absence and/or quantity of one or more biomarkers characteristic of the diagnosed disease or condition, such as chronic mental stress). As used herein, "diagnosis of' the diseases or conditions as taught herein in a subject may particularly mean that the subject has such, hence, is diagnosed as having such. "Diagnosis of no" diseases or conditions as taught herein in a subject may particularly mean that the subject does not have such, hence, is diagnosed as not having such. A subject may be diagnosed as not having such despite displaying one or more conventional symptoms or signs reminiscent of such.

The terms "prognosticating" or "prognosis" generally refer to an anticipation on the progression of a disease or condition, such as chronic mental stress, and the prospect (e.g., the probability, duration, and/or extent) of recovery. A good prognosis of the diseases or conditions taught herein may generally encompass anticipation of a satisfactory partial or complete recovery from the diseases or conditions, preferably within an acceptable time period. A good prognosis of such may more commonly encompass anticipation of not further worsening or aggravating of such, preferably within a given time period. A poor prognosis of the diseases or conditions as taught herein may generally encompass anticipation of a substandard recovery and/or unsatisfactorily slow recovery, or to substantially no recovery or even further worsening of such.

Hence, prediction or prognosis of a disease or condition can *inter alia* allow to predict or make a prognosis of the occurrence of the disease or condition, or to predict or make a prognosis of the progression, aggravation, alleviation or recurrence of the disease or condition or response to treatment or to other external or internal factors, situations or stressors, *etc.*

A good prognosis of the condition as taught herein may generally encompass anticipation of a satisfactory partial or complete recovery from the conditions back to before the condition was obtained, preferably within an acceptable time period. A good prognosis of such may more commonly encompass anticipation of not further worsening or aggravating the general health of the patient, preferably within a given time period.

A poor prognosis of the diseases or conditions as taught herein may generally encompass anticipation of a limited recovery and/or unsatisfactorily slow recovery, or to substantially no recovery or even further worsening of such in a subject.

Further, monitoring a disease or condition, such as chronic mental stress, can *inter alia* allow to predict the occurrence of the disease or condition, or to monitor the progression, aggravation, alleviation or recurrence of the disease or condition, or response to treatment or to other external or internal factors, situations or stressors, etc. Advantageously, monitoring may be applied in the course of a medical treatment of a subject, preferably medical treatment aimed at alleviating the so-monitored disease or condition. Such monitoring may be comprised, e.g., in decision making whether a patient may be discharged, needs a change in treatment or needs further hospitalisation. As intended herein, a reference to monitoring of a disease or condition also specifically includes monitoring of the probability, risk or chance of a subject to develop the disease or condition, i.e., monitoring change(s) in said probability, risk or chance over time.

In an embodiment, an analysis is performed on a body fluid sample. Said body fluid sample is preferably chosen from whole blood (optionally diluted), a blood fraction, isolated blood cells, serum, plasma, secretions of the respiratory, intestinal and genitourinary tracts, saliva, oral fluid, urine, feces, sweat, tears, sputum, ear flow, lymph fluid, swabs, smears and/or a tissue biopsy. Preferably, samples are obtained through collection of the fluid in a container (e.g. urine or saliva) or absorbing material or by inserting a needle into the body cavity and aspirating with a syringe a portion of the fluid (e.g. blood, lymph fluid). Isolated blood cells include white blood cells such as leukocytes, more in particular neutrophils, basophils, monocytes, or lymphocytes, as well as red blood cells or erythrocytes.

In particular embodiments, the sample is a body fluid sample selected from the group comprising whole blood, a blood fraction, isolated blood cells, serum, plasma, secretions of the respiratory, intestinal and genitourinary tracts, saliva, oral fluid, urine, feces, sweat, tears, sputum, ear flow, lymph fluid, swabs, smears or wherein the sample is a tissue biopsy sample. In a preferred embodiment, the sample is a blood or saliva sample, more preferably a blood sample, such as a whole blood sample or serum sample.

As mentioned before, the current methodology is compatible with various sorts of body fluid samples, including blood, serum, saliva and urine. The use of serum instead of whole blood allows the sample to be stored and analyzed later. Using urine or saliva instead of blood represents a truly non-invasive sample type and allows for e.g. home-testing and shipment of the sample to the test lab. This is obviously an additional advantage compared to other sample obtaining methods such as drawing blood.

Only a small sample is required for detection in the method, such as provided from a pin prick device or lancet rendered to the individual in order to obtain capillary blood, or from a mouth swab to obtain saliva. However, the sample can also be obtained from venous blood collected in the standard way. In the case of whole blood, it can be collected with an anticoagulant present in order to prevent the normal clotting process. It may also be used immediately, for example, a small amount of blood can be diluted with assay buffer.

Recreational athletes are usually combining sportive goals with work pressure and/or the care of their family. In order to prevent the development or worsening of fatigue syndromes such as burn-out, depression, chronic stress, chronic fatigue syndrome, non-functional overreaching and overtraining syndrome, these recreational athletes need to establish a good balance between training, work, social activities and rest. This population would highly benefit from the current invention, as numerous recreational athletes are unaware of the cumulative impact of these different stress factors and the invention enables them to objectively monitor their fatigue status.

Sport coaches and sport scientists working with elite or professional athletes are challenged to find a balance between the hard training required to optimize performance and the introduction of sufficient resting and recovery periods necessary for the prevention of physiological maladaptation and the occurrence of fatigue syndromes. These syndromes are likely to occur in high performance or elite level athletes because these athletes are driven towards achieving success in competitions at world class level and, as such, may ignore early warning signs of overtraining in the pursuit of their competitive goal.

The method according to any of the previous embodiments, is therefore especially well-suited when the individual is an athlete or a sportsperson trained in one or more sports including, but not limited to, archery, badminton, baseball, basketball, bowling, canoeing, car racing, cheerleading, climbing, combat sports such as aikido, jujutsu, judo, wrestling, boxing, capoeira, karate, kickboxing, taekwondo and fencing, cricket, cycling, dancing, decathlon, discus throwing, diving, football, golf, gymnastics, hammer throwing, handball, heptathlon, high jumping, hiking, hockey, horse racing, ice hockey, ice skating, kayaking, kitesurfing, long jumping, military, motorcycle racing, mountaineering, parachuting, paragliding, polo, racketlon, racquetball, rafting, rope jumping, rowing, rugby, running, sailing, skateboarding, skating, skiing, snowboarding, soccer, squash, surfing, swimming, table tennis, tennis, triathlon, volleyball, wakeboarding, water polo, weightlifting, windsurfing and wood chopping. Preferably, the individual is a runner, a rower, a cycler, a swimmer, a triathlon athlete or a soccer player. The current method allows sport coaches and sport scientists to objectively monitor the fatigue status of the athlete, thus introducing an objective criterion on which they can rely for developing/adapting the athlete's training program in order to achieve an optimal balance between training intensity and recovery.

In a further embodiment, information provided by the athlete or employee such as his or her training or work scheme, the measurement of biometric variables (e.g. hearth rhythm) and/or indicators derived from self-reported questionnaires are used to identify time-points or moments when the probability to develop a fatigue condition, such as chronic mental stress, is increased. This information is subsequently used to prompt the individual to use the *in vitro* method for predicting, diagnosing, prognosing and/or monitoring a fatigue condition according to the current invention. By introducing additional criteria to estimate when *in vitro* testing would be most meaningful, unnecessary continuous monitoring is avoided which could otherwise result in a needless build-up of costs.

It is also the case that certain non-human mammals that are exercised and trained to perform in racing competition events suffer from OTS. Examples of such non-human mammals include horses, dogs (including solo runners such as greyhounds and sleddogs such as huskies and Eurohounds), and camels. Also pet owners are well aware of mood changes in their companions, however, diagnosis of clinical depression is difficult since dogs and cats cannot talk and tell a physician about their feelings. Therefore, in another further embodiment, the method of the current invention can be used for predicting, diagnosing, prognosing and/or monitoring a in a human or in a non-human mammal. The method of the invention thus, for the first time, enables care-takers to objectively detect and diagnose depression disorder in animals, avoiding the need for complicated medical tests in order to exclude other medical reasons and allowing for an adequate therapy to be prescribed.

The method as taught herein, allows screening of employees on a regular basis, therefore providing a method to objectively monitor the chronic stress levels and/or the fatigue status of the employees. As described earlier, chronic stress and/or fatigue puts the employees at high risk for the development of burn-out and/or depression. The annual global cost of burn-out is huge. In addition, burn-out has a significant impact on the individual himself, especially affecting social aspects of his personal life. Thus, by detecting the early stage (onset) of burn-out symptoms using the method of the invention, work load can be adapted in time, resulting in huge benefits, both for the employee and the company.

Therefore, in a further embodiment, the method according to any of the previous embodiments, is especially well-suited when the individual is an employee.

In another embodiment, the method as taught herein, allows identifying those patients that will benefit from a certain type of therapy, such as psychotherapy or pharmacotherapy. A lot of therapies are not effective for the whole population of subjects suffering from a fatigue syndrome, such as burn-out and/or depression, resulting in huge costs without benefit to the subject. Therefore the invention is well-suited for governments, pharmaceutical industries or patients to objectively stratify patients according to their best therapy, reducing high economic costs.

Also described herein is a method for screening a group of individuals via the *in vitro* method as taught herein. Preferably, the group of individuals are employees. Screening of employees using the method according to the invention can avoid huge economical losses caused by continued pressure/performance of undiagnosed employees suffering from burn-out syndrome or depression disorder. Finally, preventing the development of more serious forms of the syndrome or disorder owing to early diagnosis can have a profound positive effect on the individual's emotional/cognitive health in addition to his physical health. A group of individuals, as used in the context of the current invention, refers to more than one single individual.

In another further embodiment, the group of individuals is a team of sportspersons such as, but not limited to, a hockey team, a soccer team, a football team, a basketball team, a rowing team, a swimming team or a relay team. The method allows to screen for the most fatigued team member and thus allows the person responsible of assembling the team, to adapt the training on an individual base or to avoid including this weak link in the team when the team ought to perform at its best, for example during a competition. Similarly, in another further embodiment, the individuals in the group of individuals can be mammals such as, but not limited to, dogs or horses.

In particular embodiments, the method as taught herein comprises concluding the prediction, diagnosis and/or prognosis from the detection of said at least three biomarkers in sad sample.

In particular embodiments, the method as taught herein is a method for monitoring a fatigue syndrome and comprises concluding a worsening, amelioration or stabilization of the fatigue syndrome.

In particular embodiments, detecting said at least three biomarkers as taught herein allows obtaining or results in a biosignature and the method further comprises a step of predicting, diagnosing, prognosing and/or monitoring the fatigue syndrome, based on said biosignature.

In particular embodiments, the step of predicting diagnosing, prognosing and/or monitoring chronic stress based on said biosignature comprises multivariate statistical modelling of said biosignature. Non-limiting examples of multivariate statistical modelling of biosignatures includes PLS-DA, logistic regression, discriminative analysis with higher order equations, vector machine with higher order equation made up of each biomarker variable that maximizes the boundary between groups, multiple regression analysis with a higher order equation, k-means method or decision trees.

In particular embodiments, the method as taught herein comprises the steps of

(i) determining the status of said at least three biomarkers, such as at least three biomarkers from Table 1, Table 2, Table 3, Table 4, Table 5, Table 6, Table 7, Table 8, Table 9, Table 10, or Table 11, in the sample from the subject;

(ii) combining the status of said at least three biomarkers as determined in (i) to a BIRIX;

(iii) finding a deviation or no deviation of the BIRIX compared to a reference value;

(iv) attributing said finding of deviation or no deviation to a particular diagnosis, prediction, or prognosis of the fatigue syndrome in the subject.

In particular embodiments, the fatigue syndrome in chronic mental stress, preferably wherein chronic mental stress is the mental status prior to development of mood disorders, such as depression or burn-out, and the method as taught herein comprises the steps of

(i) determining the status of said at least three, preferably at least four, more preferably at least five, even more preferably at least six, biomarkers independently selected from at least two, preferably at least three, more preferably at least four, even more preferably at least five, even more preferably at least six, different groups of biomarkers selected from: biomarker group 100, biomarker group 200, biomarker group 300, biomarker group 400, biomarker group 500, and optionally biomarker group 600; preferably selected from: biomarker group 100a, biomarker group 200a, biomarker group 300a, biomarker group 400a and biomarker group 500a, as described elsewhere herein, in the sample from the subject;

(ii) combining the status of said at least three biomarkers as determined in (i) to a BIRIX;

(iii) finding a deviation or no deviation of the BIRIX compared to a reference value;

(iv) attributing said finding of deviation or no deviation to a particular diagnosis, prediction, or prognosis of chronic mental stress in the subject.

The term "status" as used herein refers to the outcome of measurement of said biomarker and comprises the presence, absence and/or amount of said biomarker. The terms "quantity", "amount" and "level" are synonymous and generally well-understood in the art. The terms as used herein may particularly refer to an absolute quantification of a molecule, an analyte or the number of cells in a sample, or to a relative quantification of a molecule, an analyte or a number of cells in a sample, i.e., relative to another value such as relative to a reference value as taught herein, or to a range of values indicating a base-line expression of the biomarker. These values or ranges can be obtained from a single patient or from a group of patients.

The terms "quantity" and "expression level" of a biomarker, wherein the biomarker is a peptide, polypeptide, protein or nucleic acid, can be used interchangeably in this specification to refer to the absolute and/or relative quantification, concentration level or amount of any such product in a sample.

In particular embodiments, the status of said at least three biomarkers is normalized for gender, age, diurnal variations, food intake (e.g. diet), physical activity, or a combination thereof.

Similarly, in particular embodiments, a correction factor is applied to the status of said at least three biomarkers, preferably wherein said correction factor is a correction factor depending on gender, age, diurnal variations, food intake (e.g. diet), physical activity, or a combination thereof. For example, the correction factors can be determined based on a control individual or a group of control individuals or one or more clinical reference values.

Biomarkers such as bio-available testosterone can also be normalized based on albumin. Furthermore, the amount of protein, polypeptide or peptide can also be normalized using albumin.

In particular embodiments, the BIRIX is obtained by multiplying the status of each of said at least three biomarkers by a corresponding weight, wherein an expression signature defines the weight for each biomarker. The expression signature can be defined using methods known in the art, such as multivariate statistical modelling including logistic regression and machine learning. Preferably, the expression signature is defined using partial least squares-discriminant analysis (PLS-DA).

For example, the following statistical methodology can be applied:

1. Log-transforming the concentrations and relative concentrations of the at least three biomarkers before modelling.
2. Making PLS-DA multivariable models by selecting variables as follows:

- 1 to $N_p$ predictors.
- All possible combinations of variables.

3. Performing a three-fold cross validation stratifying over the outcomes.
4. Training the models on a large dataset, including control subjects.
5. Selecting models that have the best statistically significant prognostic performance for the outcome within the subgroup studied.

$N_p$ prefers to the number of biomarkers taken up in the PLS-DA model.

Distinct reference values may represent the diagnosis of a fatigue syndrome *vs.* the absence of a fatigue syndrome (such as, e.g., healthy or recovered from a fatigue syndrome). In another example, distinct reference values may represent the diagnosis a fatigue syndrome of varying severity.

Alternatively, distinct reference values may represent the prediction of a risk (e.g., an abnormally elevated risk) of developing a fatigue syndrome vs. the prediction of no or normal risk of developing a fatigue syndrome. In another example, distinct reference values may represent predictions of differing degrees of risk of developing a fatigue syndrome.

In yet another example, distinct reference values may represent the need of a subject for a therapeutic or prophylactic treatment of a fatigue syndrome, such as chronic mental stress, vs. no need of a subject for a therapeutic or prophylactic treatment of a fatigue syndrome, such as chronic mental stress. In a further example, distinct reference values may represent various urgencies of the need for a therapeutic or prophylactic treatment of a fatigue syndrome or the need for a specific type of therapeutic or prophylactic treatment of a fatigue syndrome.

Moreover, distinct reference values may represent a good prognosis for a fatigue syndrome *vs.* a poor prognosis for a fatigue syndrome. In a further example, distinct reference values may represent varyingly favourable or unfavourable prognoses for a fatigue syndrome.

As explained, the present methods, may involve finding a deviation or no deviation between the BIRIX and a given reference value.

A "deviation" of a first value from a second value may generally encompass any direction (e.g., increase: first value > second value; or decrease: first value < second value) and any extent of alteration.

For example, a deviation may encompass a decrease in a first value by, without limitation, at least about 10% (about 0.9-fold or less), or by at least about 20% (about 0.8-fold or less), or by at least about 30% (about 0.7-fold or less), or by at least about 40% (about 0.6-fold or less), or by at least about 50% (about 0.5-fold or less), or by at least about 60% (about 0.4-fold or less), or by at least about 70% (about 0.3-fold or less), or by at least about 80% (about 0.2-fold or less), or by at least about 90% (about 0.1-fold or less), relative to a second value with which a comparison is being made.

For example, a deviation may encompass an increase of a first value by, without limitation, at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 150% (about 2.5-fold or more), or by at least about 200% (about 3-fold or more), or by at least about 500% (about 6-fold or more), or by at least about 700% (about 8-fold or more), or like, relative to a second value with which a comparison is being made.

Preferably, a deviation may refer to a statistically significant observed alteration. For example, a deviation may refer to an observed alteration which falls outside of error margins of reference values in a given population (as expressed, for example, by standard deviation or standard error, or by a predetermined multiple thereof, e.g., $\pm 1 \times SD$ or $\pm 2 \times SD$ or $\pm 3 \times SD$, or $\pm 1 \times SE$ or $\pm 2 \times SE$ or $\pm 3 \times SE$). Deviation may also refer to a value falling outside of a reference range defined by values in a given population (for example, outside of a range which comprises $\geq 40\%$, $\geq 50\%$, $\geq 60\%$, $\geq 70\%$, $\geq 75\%$ or $\geq 80\%$ or $\geq 85\%$ or $\geq 90\%$ or $\geq 95\%$ or even $\geq 100\%$ of values in said population).

In particular embodiments, said deviation may be concluded if said deviation has a statistical significance of $p<0.05$.

In a further embodiment, a deviation may be concluded if an observed alteration is beyond a given threshold or cut-off. Such threshold or cut-off may be selected as generally known in the art to provide for a chosen sensitivity and/or specificity of the prediction methods, e.g., sensitivity and/or specificity of at least 50%, or at least 60%, or at least 70%, or at least 80%, or at least 85%, or at least 90%, or at least 95%.

For example, receiver-operating characteristic (ROC) curve analysis can be used to select an optimal cut-off value of the BIRIX, for clinical use of the present *in vitro* methods, based on acceptable sensitivity and specificity, or related performance measures which are well-known *per se,* such as positive predictive value (PPV), negative predictive value (NPV), positive likelihood ratio (LR+), negative likelihood ratio (LR-), Youden index, or similar.

The present methods may further involve attributing any finding of a deviation or no deviation between the BIRIX and a given reference value to a particular diagnosis, prediction, or prognosis of the fatigue syndrome in the subject.

In the methods provided herein the observation of a deviation between the BIRIX and a given reference value can lead to the conclusion that the diagnosis, prediction and/or prognosis of said fatigue syndrome, such as chronic mental stress, in said subject is different from that represented by said reference value. Similarly, when no deviation is found between the BIRIX and a given reference value, the absence of such deviation can lead to the conclusion that the diagnosis, prediction and/or prognosis of said fatigue syndrome in said subject is substantially the same as that represented by said reference value.

In particular embodiments, the reference value as used in the methods according to the invention is determined from a subject or a group of subjects not affected by said fatigue syndrome.For example, for non-functional overreaching and overtraining syndrome the reference value may be determined from a subject or a group of subjects not undergoing intensive training. For example, for chronic stress, preferably chronic mental stress, the reference value may be determined from a subject or a group of subjects which are not undergoing pressure and stressful situations, such as persons with a good work-life balance. For example, for burn-out the reference value may be determined from a subject or a group of subjects not diagnosed with any mental or psychological disorder using different types of validated questionnaires. For example, for depression the reference value may be determined from a subject or a group of subjects not diagnosed with any mental or psychological disorder using different types of validated questionnaires.

In particular embodiments, the reference value as used in the methods according to the invention is a BIRIX score for a subject or a group of subjects not affected by said fatigue syndrome, such as not affected by chronic mental stress.

In particular embodiments, in step (iii) of finding a deviation or no deviation of the BIRIX compared to a reference value, a decreased BIRIX compared to the reference value allows for the prediction, diagnosis, prognosis or monitoring, preferably the prediction, diagnosis or prognosis of the fatigue syndrome, such as chronic mental stress, in the subject.

The BIRIX may be decreased compared to (i.e., relative to) a reference value representing a healthy subject or a subject in who no onset or existence of a fatigue syndrome is detected. The so-decreased BIRIX may allow for the prediction, diagnosis, prognosis, or monitoring of the fatigue syndrome, such as chronic mental stress, in the subject.

In particular embodiments, the BIRIX may be decreased compared to (i.e., relative to) a reference value representing the prediction of normal (not elevated) probability of progression of a fatigue syndrome, such as chronic mental stress, within a given time period. The so-decreased BIRIX may allow for the prediction that the subject has an elevated risk of progression of a fatigue syndrome within the given time period.

In other preferred embodiments, the BIRIX may be decreased compared to (i.e., relative to) a reference value representing a given degree of probability of progression of a fatigue syndrome (e.g., "low probability" or "moderate probability") within a given time period. The so-decreased BIRIX may allow for the prediction that the subject has a comparatively greater probability of progression of a fatigue syndrome (e.g., "moderate probability" vs. "low probability", or "high probability" vs. "moderate probability" or "low probability") within the given time period.

In particular embodiments, the *in vitro* method as taught herein is a method for predicting a fatigue syndrome, such as chronic mental stress, in a subject, such as a method for detecting the onset of a fatigue syndrome and a BIRIX of less than 40 indicates a "high risk" to "very high risk" for the subject to develop the fatigue syndrome. In further particular embodiments, the *in vitro* method as taught herein is a method for predicting a fatigue syndrome in a subject, such as a method for detecting the onset of a fatigue syndrome and a BIRIX of more than 60 indicates a "low risk" to "very low risk" for the subject to develop the fatigue syndrome.

A further aspect provides an *in vitro* method for predicting the response of a subject diagnosed with a fatigue syndrome selected from the group consisting of burn-out, depression, chronic stress, chronic fatigue syndrome, non-functional overreaching and overtraining syndrome in a subject, to therapy, comprising detecting at least three biomarkers independently selected from Table 1 in a sample obtained from said subject.

In particular embodiments, the fatigue syndrome in chronic mental stress, preferably wherein chronic mental stress is the mental status prior to development of mood disorders, such as depression or burn-out, and the method comprises detecting at least three, preferably at least four, more preferably at least five, even more preferably at least six, biomarkers independently selected from at least two, preferably at least three, more preferably at least four, even more preferably at least five, even more preferably at least six, different groups of biomarkers selected from: biomarker group 100, biomarker group 200, biomarker group 300, biomarker group 400, biomarker group 500, and optionally biomarker group 600; preferably selected from: biomarker group 100a, biomarker group 200a, biomarker group 300a, biomarker group 400a and biomarker group 500a, as described elsewhere herein.

In particular embodiments, the method for predicting the response of a subject diagnosed with a fatigue syndrome, such as chronic mental stress, comprises the steps of:

- determining the status of said at least three biomarkers in the sample from the subject;

- combining the status of said at least three biomarkers as determined in the previous step to a BIRIX;

- finding a deviation or no deviation of the BIRIX compared to a reference value;

- classifying said subject as a good responder to said therapy or a poor responder to said therapy based on the finding of said deviation or no deviation.

If the method is a method for predicting the response of a subject diagnosed with a fatigue syndrome, such as chronic mental stress, to therapy, the reference value is preferably representative for a poor responder to the medicament or for a good responder to the therapy.

In particular embodiments, if the reference value represents a good responder, the subject can be classified as a good responder if no deviation is found.

In particular embodiments, if the reference value represents a poor responder, the subject can be classified as a poor responder if no deviation is found.

A further aspect provides an *in vitro* method for monitoring the therapy for a fatigue syndrome selected from the group consisting of burn-out, depression, chronic stress, chronic fatigue syndrome, non-functional overreaching and overtraining syndrome, preferably burn-out and/or depression, more preferably burn-out, in a subject, comprising detecting at least three biomarkers independently selected from Table 1 in a sample obtained from said subject.

In particular embodiments, the fatigue syndrome in chronic mental stress, preferably wherein chronic mental stress is the mental status prior to development of mood disorders, such as depression or burn-out, and the method comprises detecting at least three, preferably at least four, more preferably at least five, even more preferably at least six, biomarkers independently selected from at least two, preferably at least three, more preferably at least four, even more preferably at least five, different groups of biomarkers selected from: biomarker group 100, biomarker group 200, biomarker group 300, biomarker group 400, biomarker group 500, and optionally biomarker group 600; preferably selected from: biomarker group 100a, biomarker group 200a, biomarker group 300a, biomarker group 400a and biomarker group 500a, as described elsewhere herein.

In particular embodiments, the method comprises the steps of

- determining the status of said at least three biomarkers in the sample from the subject;

- combining the status of said at least three biomarkers as determined in the previous step to a BIRIX ("BIRIX1");

- finding a deviation or no deviation of the BIRIX 1 compared to a reference value;

- repeating the step of determining the status of said at least three biomarkers in the sample from the subject; combining the status of said at least three biomarkers as determined in the previous step to a BIRIX and finding a deviation or no deviation of the BIRIX compared to a reference value after a period of time during which said subject receives a therapy for said fatigue syndrome to obtain a post-treatment BIRIX score ("BIRIX2"); and

- classifying said therapy as being effective if the deviation of the BIRIX2 compared to the reference value is smaller than the deviation of the BIRIX1 compared to the reference value, preferably wherein the reference value represents a control subject, such as a subject not suffering from the fatigue syndrome.

In particular embodiments, the period of time ranges from weeks to months after the onset of said therapy. In more particular embodiments, the period of time may be at least one week, at least two weeks, at least three weeks, at least four weeks, at least five weeks, or at least six weeks. In even more particular embodiments, the period of time may be at least one month, at least two months, at least three months, at least four months, at least five months, or at least six months.

The person skilled in the art will understand that determining the status of the biomarkers and combining the status of the biomarkers to a BIRIX can be performed as described elsewhere for the *in vitro* method for predicting, diagnosing, prognosing and/or monitoring a fatigue syndrome, such as chronic mental stress, as taught herein.

The person skilled in the art will also understand that finding of a deviation or no deviation and attributing said finding of deviation or no deviation to a particular response can be performed as described elsewhere for the method for predicting, diagnosing, prognosing and/or monitoring a fatigue syndrome, such as chronic mental stress, as taught herein.

In particular embodiments, the therapy is psychotherapy or pharmacotherapy.

In more particular embodiments, the fatigue syndrome is chronic mental stress and the therapy is psychotherapy, balancing life-work, work related adaptations, recovery strategies, intake of supplements, exercise plan, sleep or relaxation therapy(such as meditation, yoga).

In more particular embodiments, the fatigue syndrome is depression or burn-out, preferably depression, and the therapy is cognitive behavioral therapy, short-term psychodynamic psychotherapy or pharmacotherapy.

A further aspect provides a diagnostic kit for predicting, diagnosing, prognosing and/or monitoring a fatigue syndrome selected from the group consisting of burn-out, depression, chronic stress, chronic fatigue syndrome, non-functional overreaching and overtraining syndrome, preferably chronic mental stress, in a subject.

In other words, a further aspect provides a diagnostic kit for use in the *in vitro* prediction, diagnosis, prognosis and/or monitoring of a fatigue syndrome selected from the group consisting of burn-out, depression, chronic stress, chronic fatigue syndrome, non-functional overreaching and overtraining syndrome in a subject.

A further aspect provides a diagnostic kit for use in the *in vitro* determination whether a subject is in need of therapeutic or prophylactic treatment of a fatigue syndrome selected from the group consisting of burn-out, depression, chronic stress, chronic fatigue syndrome, non-functional overreaching and overtraining syndrome as taught herein.

A further aspect provides a diagnostic kit for use in the in vitro prediction of the response of a subject diagnosed with a fatigue syndrome selected from the group consisting of burn-out, depression, chronic stress, chronic fatigue syndrome, non-functional overreaching and overtraining syndrome, to therapy as taught herein.

A further aspect provides a diagnostic kit for use in monitoring the therapy for a fatigue syndrome selected from the group consisting of burn-out, depression, chronic stress, chronic fatigue syndrome, non-functional overreaching and overtraining syndrome in a subject as taught herein.

In particular embodiments, the diagnostic kit comprises:

- means for measuring the status of at least three biomarkers independently selected from Table 1, Table 2, Table 3, Table 4, Table 5, Table 6, Table 7, Table 8, Table 9, Table 10, or Table 11 in a sample obtained from said subject, such as an assay; and

- a reference value or means for establishing said reference value, wherein said reference value represents a known diagnosis, prediction and/or prognosis of the fatigue syndrome.

In particular embodiments, the fatigue syndrome is non-functional overreaching and/or overtraining syndrome and the kit comprises means for measuring the status of at least three biomarkers independently selected from at least two, preferably at least three, more preferably at least four, different groups of biomarkers selected from 1, 1a, 1b, 2, 2a, 2b, 3, 3a, 3b, 4, 4a, 5, 5a, 6 and 6a, as described elsewhere herein. In particular embodiments, the fatigue syndrome is chronic stress, burn-out and/or depression, preferably chronic stress or chronic mental stress, more preferably chronic stress or chronic mental stress as a precursor or the indication of the early stage (onset) of burn-out and/or depression, and the kit comprises means for measuring the status of at least three biomarkers independently selected from at least two, preferably at least three, more preferably at least four, different groups of biomarkers selected from groups 7, 7a, 7b, 8, 8a, 9, 9a, 10, 10a, 10b, 11, 11a, 11b and 12, as described elsewhere herein.

In further particular embodiments, the reference value is a reference value for use in finding a deviation or no deviation of the BIRIX compared to said reference value as described elsewhere herein.

In further particular embodiments, the reference value is a reference value for the BIRIX for the combination of the at least three biomarkers, such as wherein said reference value corresponds to a BIRIX of said at least three biomarkers independently selected from Table 1, Table 2, Table 3, Table 4, Table 5, Table 6, Table 7, Table 8, Table 9, Table 10, or Table 11 in a subject not affected by the fatigue syndrome, or wherein said BIRIX corresponds to the score of said at least three biomarkers independently selected from Table 1, Table 2, Table 3, Table 4, Table 5, Table 6, Table 7, Table 8, Table 9, Table 10, or Table 11 in a subject affected by the fatigue syndrome.

In further particular embodiments, the fatigue syndrome in chronic mental stress, preferably wherein chronic mental stress is the mental status prior to development of mood disorders, such as depression or burn-out, and the reference value is a reference value for the BIRIX for the combination of the at least three biomarkers, such as wherein said reference value corresponds to a BIRIX of said at least three, preferably at least four, more preferably at least five, even more preferably at least six, biomarkers independently selected from at least two, at least three, preferably at least four, even more preferably at least five, different groups of biomarkers selected from: biomarker group 100, biomarker group 200, biomarker group 300, biomarker group 400, biomarker group 500, and optionally biomarker group 600; preferably selected from: biomarker group 100a, biomarker group 200a, biomarker group 300a, biomarker group 400a and biomarker group 500a, as described elsewhere herein, in a subject affected by chronic mental stress.

In particular embodiments, the kit further comprises a microfluidic reactor (also known as lab-on-a-chip). The microfluidic reactor enables the multiplex detection of enzymatic reactions or immunoassays.

In further particular embodiments, the kit further comprises a sample collection and/or storage means.

Integration of the method as taught herein into a suitable kit, allows individuals to monitor their fatigue status in a simple and efficient manner. The kit can be used by the individual himself or by a different individual (e.g. coach, trainer, employer, friends) to provide the individual with information about his current health condition, which enables them to make informed decisions relating to for example training load or workload. Detection of the onset and/or existence of burn-out, depression, chronic stress, chronic fatigue syndrome, non-functional overreaching and overtraining syndrome in an individual using the diagnostic kit provided by the invention, constitutes an easy to use and rapid mode of detection. In particular embodiments, for example when the kit is used in a home-environment, there is no need for additional instrumentation, since the kit contains all components needed for detection. In other embodiments, if the kit is used in a laboratory setting, the kit does not require to comprise all components required for detection and therefore, additional instrumentation may be required.

In one embodiment of the diagnostic kits described above, the kit generates an outcome value which is submitted in an online tool such as, but not limited to, a website or a mobile application, to generate or calculate a prognostic value for the onset and/or the existence of burn-out, depression, chronic stress, chronic fatigue syndrome, non-functional overreaching and/or overtraining syndrome in a subject. The online tool allows the data to be stored and to be consulted at different time points. In another embodiment, the diagnostic kit directly provides a prognostic value without the need of an external online tool and/or calculation.

In further particular embodiments, the kit further comprises a computer readable storage medium having recorded thereon one or more programs for carrying out the method of taught herein.

A "sample collection means" refers to a device to collect a body fluid sample from an individual, and includes invasive sample collection means (e.g. a needle to withdraw blood), home-collection kits (finger prick or small disposable device to simplify blood sampling) as well as non-invasive sample collection means (e.g. a tube to collect saliva). Non-invasive sampling includes for example, but is not limited to, respiratory secretions, saliva, buccal swabs, oral fluid, urine, feces, sweat, tears, sputum, ear flow and/or smears. Invasive sample types include but are not restricted to whole blood (optionally diluted), a blood fraction, isolated blood cells, serum, plasma, secretions of the intestinal and/or genitourinary tracts, lymph fluid and/or tissue biopsy.

A "sample storage means" refers to a device especially designed to store samples for diagnostic assays in a way as to maintain the informative status of the biomarkers to be measured in the sample as long as required, which in this case is until the status of one or more specific biomarkers in that sample have been determined.

In a further preferred embodiment, the sample collection and/or storage means of the diagnostic kit comprises a sample inlet site for introduction of a body fluid sample of the individual. In another preferred embodiment, the kit comprises a sample collection means that is easy to operate and does not require special training in order to be able to perform sample collection using the kit. The main advantage of this type of sample collection means is that it enables self or assisted sample collection thereby avoiding the need to consult a physician and considerably increasing the user friendliness of the diagnostic kit. The diagnostic kit provided by the invention allows at-home diagnostic testing which may provide an individual with valuable information and enable him to seek medical intervention earlier, preventing further medical complications.

In another further preferred embodiment of the invention, the sample is chosen from whole blood, a blood fraction, isolated blood cells, serum, plasma, secretions of the respiratory, intestinal and genitourinary tracts, saliva, oral fluid, urine, feces, sweat, tears, sputum, ear flow, lymph fluid, swabs, smears and/or a tissue biopsy.

In an embodiment, detection of the onset and/or existence of burn-out, depression, chronic stress, chronic fatigue syndrome, non-functional overreaching and/or overtraining syndrome in an individual using the diagnostic kit is concluded from the status of at least three biomarkers selected from the markers as listed in Table 1, Table 2, Table 3, Table 4, Table 5, Table 6, Table 7, Table 8, Table 9, Table 10, or Table 11.

In an embodiment, detection of chronic mental stress in an individual using the diagnostic kit is concluded from the status of at least three, preferably at least four, more preferably at least five, even more preferably at least six, biomarkers independently selected from at least two, preferably at least three, more preferably at least four, even more preferably at least five, different groups of biomarkers selected from: biomarker group 100, biomarker group 200, biomarker group 300, biomarker group 400, biomarker group 500, and optionally biomarker group 600; preferably selected from: biomarker group 100a, biomarker group 200a, biomarker group 300a, biomarker group 400a and biomarker group 500a, as described elsewhere herein.

The inventors have found that combining the status of at least three biomarkers selected from Table 1, Table 2, Table 3, Table 4, Table 5, Table 6, Table 7, Table 8, Table 9, Table 10, or Table 11 leads to an increase in accuracy and sensitivity of predicting, diagnosing, prognosing and/or monitoring burn-out, depression, chronic stress, chronic fatigue syndrome, non-functional overreaching and/or overtraining syndrome in an individual.

More particularly, the inventors have found that combining the status of at least three biomarkers independently selected from at least two, preferably at least three, more preferably at least four, even more preferably at least 5, different groups of biomarkers selected from biomarker groups 1, 2, 3, 4, 5 and 6, preferably biomarker groups 1a, 2a, 3a, 4a, 5a, 6a, more preferably biomarker groups 1b, 2b, 3b, 4a, 5a, 6a as described elsewhere herein , leads to an increase in accuracy and sensitivity of predicting, diagnosing, prognosing and/or monitoring non-functional overreaching and/or overtraining syndrome in a subject.

Furthermore, the inventors have found that combining the status of at least three biomarkers independently selected from at least two, preferably at least three, more preferably at least four, even more preferably at least 5, different groups of biomarkers selected from biomarker groups 7, 8, 9, 10, 11 and 12, preferably biomarker groups 7a, 8a, 9a, 10a, 11a, and 12a, more preferably biomarker groups 7b, 8a, 9a,10b, 11b and 12a as described elsewhere herein , leads to an increase in accuracy and sensitivity of predicting, diagnosing, prognosing and/or monitoring chronic stress, burn-out and/or depression, preferably chronic stress or chronic mental stress, more preferably chronic stress or chronic mental stress as a precursor or the indication of the early stage (onset) of burn-out and/or depression in a subject. The inventors have additionally found that the status of biomarkers belonging to the groups listed above are the most informative for drawing conclusions on the onset and/or existence of burn-out, depression, chronic stress, chronic fatigue syndrome, non-functional overreaching and/or overtraining syndrome. This allows to infer an unambiguous conclusion related to the onset and/or the existence of said fatigue conditions in an individual based on the determination of the status of individual biomarkers which may not be informative on their own.

Furthermore, the inventors have found that combining the status of at least three , preferably at least four, more preferably at least five, even more preferably at least six, biomarkers independently selected from at least two, preferably at least three, more preferably at least four, even more preferably at least five, different groups of biomarkers selected from: biomarker group 100, biomarker group 200, biomarker group 300, biomarker group 400, biomarker group 500, and optionally biomarker group 600; preferably selected from: biomarker group 100a, biomarker group 200a, biomarker group 300a, biomarker group 400a and biomarker group 500a, as described elsewhere herein, leads to an increase in accuracy and sensitivity of predicting, diagnosing, prognosing and/or monitoring chronic mental stress. The inventors have additionally found that the status of biomarkers belonging to the groups listed above are the most informative for drawing conclusions on the chronic mental stress. This allows to infer an unambiguous conclusion related to the chronic mental stress in an individual based on the determination of the status of individual biomarkers which may not be informative on their own.

In a further embodiment, the diagnostic kit according to the invention is provided as a collection of such kits which is suited for the screening of a group of individuals, preferably of a group of employees. The term collection as used herein refers to a quantity of at least two. This kit thus enables for example the employer with a fast, easy and objective means to monitor the fatigue status of its employees.

Also disclosed herein is an assay to determine the status of at least three biomarkers selected from Table 1, Table 2, Table 3, Table 4, Table 5, Table 6, Table 7, Table 8, Table 9, Table 10, or Table 11 in a sample obtained from the body of an individual, wherein said status relates to the presence, absence and/or the amount of said biomarkers and wherein the prediction, diagnosis, prognosis and/or monitoring of burn-out, depression, chronic stress, chronic fatigue syndrome, non-functional overreaching and/or overtraining syndrome can be concluded from said biomarker status in said sample.

In particular embodiments, the fatigue syndrome in chronic mental stress, preferably wherein chronic mental stress is the mental status prior to development of mood disorders, such as depression or burn-out, and the assay is to determine the status of at least three, preferably at least four, more preferably at least five, even more preferably at least six, biomarkers independently selected from at least three, preferably at least four, different groups of biomarkers selected from: biomarker group 100, biomarker group 200, biomarker group 300, biomarker group 400, biomarker group 500, and optionally biomarker group 600; preferably selected from: biomarker group 100a, biomarker group 200a, biomarker group 300a, biomarker group 400a and biomarker group 500a, as described elsewhere herein, in a sample obtained from the body of an individual, wherein said status relates to the presence, absence and/or the amount of said biomarkers and wherein the prediction, diagnosis, prognosis and/or monitoring of chronic mental stress can be concluded from said biomarker status in said sample.

In particular embodiments, assay comprises means for performing the steps of

(i) determining the status of said at least three biomarkers in the sample from the subject;

(ii) combining the status of said at least three biomarkers as determined in (i) to a BIRIX;

(iii) finding a deviation or no deviation of the BIRIX compared to a reference value; and

(iv) attributing said finding of deviation or no deviation to a particular response of said subject to said medicament.

In a preferred embodiment the assay is portable. In another preferred embodiment the assay can be performed on a body fluid sample that can comprise whole blood, a blood fraction, isolated blood cells, serum, plasma, secretions of the respiratory/intestinal/genitourinary tracts, saliva, oral fluid, urine, feces, sweat, tears, sputum, ear flow, lymph fluid, swabs, smears and/or a tissue biopsy.

A further aspect provides a computer program product for use in conjunction with a computer having a processor and a memory connected to the processor, said computer program product comprising a computer readable storage medium having a computer program mechanism encoded thereon, wherein said computer program mechanism is loaded into the memory of said computer and causes said computer to carry out the method for predicting, diagnosing, prognosing and/or monitoring a fatigue syndrome, such as chronic mental stress, as taught herein, the method for determining whether a subject is in need of therapeutic or prophylactic treatment of a fatigue syndrome, such as chronic mental stress, as taught herein; the method for predicting the response of a subject diagnosed with a fatigue syndrome, such as chronic mental stress, as taught herein; and/or the method for monitoring the therapy for a fatigue syndrome, such as chronic mental stress, in a subject as taught herein.

The present invention further relates to a computer system comprising a processor, and a memory coupled to said processor and encoding one or more programs, wherein said one or more programs instruct the processor to carry out the methods as taught herein. The inventors have found that combining the status of at least three biomarkers leads to an unexpected increase in sensitivity allowing to infer a conclusion related to the onset and/or the existence of said conditions in an individual based on the determination of the status of individual biomarkers which may not be informative on their own. Therefore, in a further preferred embodiment, the assay is used to determine the status of at least three biomarkers in the sample.

The person skilled in the art that all particular embodiments as described herein, can be used for the *in vitro* method for predicting, diagnosing, prognosing and/or monitoring a fatigue syndrome, such as chronic mental stress, can also be applied to the *in vitro* method for determining whether a subject is in need of therapeutic or prophylactic treatment of a fatigue syndrome, such as chronic mental stress,, the *in vitro* method for the therapy for a fatigue syndrome, such as chronic mental stress, the kit, the assay and the computer program product as described herein.

The present application also provides aspects and embodiments as set forth in the following Statements:

Statement 1. An *in vitro* method for predicting, diagnosing, prognosing and/or monitoring a fatigue syndrome selected from the group consisting of burn-out, depression, chronic stress, chronic fatigue syndrome, non-functional overreaching and overtraining syndrome in a subject, comprising detecting at least three biomarkers independently selected from Table 1 in a sample obtained from said subject.

Statement 2. The method according to statement 1, wherein the fatigue syndrome is chronic stress, burn-out and/or depression, and at least three biomarkers are independently selected from at least two, preferably at least three, more preferably at least four, different groups of biomarkers selected from:

- a group of neurotransmitter biomarkers consisting of anthranilic acid, cystathionine, picolinic acid, kynurenic acid (KYNA), L-kynurenine, quinolinic acid (QUIN), free tryptophan, xanthurenic acid, brain-derived neurotrophic factor (BDNF), S100 calcium-binding protein B (S100B), γ-aminobutyric acid (GABA), 3-hydroxyanthranilic acid, 3-hydroxykynurenine, Magnesium, Zinc, glutamine, glutamaat, calcineurin, glutamate receptor, indoleamine (2,3)-dioxygenase (IDO), kynurenine mono-oxygenase, kynureninase, and kynurenine transaminase (KAT);

- a group of immune function biomarkers consisting of neopterin, secretory immunoglobulin A (sIgA), interleukin 6 (IL-6), interleukin 10 (IL-10), interleukin-1 receptor A (IL-1RA), epidermal growth factor (EGF), leukocytes, neutrophils, lymphocytes, monocytes, basophils, C-reactive protein (CRP) and myeloperoxidase (MPO);

- a group of iron metabolism biomarkers consisting of ferritin, transferrin, iron, transferrin saturation, hemoglobin in reticulocytes, erythrocytes, mean cell haemoglobin (MCH), mean cell volume (MCV), mean cell haemoglobin concentration (MCHC) and reticulocytes;

- a group of metabolism biomarkers consisting of creatinine, urea, vitamin D, folic acid, riboflavin, vitamin B12, pyridoxine, 4-pyridoxic acid, flavin mononucleotide, thiamine, nicotinic acid, nicotinamide, pyridoxal 5'-phosphate, niacin (vitamin B3) N1-methylnicotinamide, pyridoxal, trigonelline, thiamine monophosphate, visfatin, and cholecalciferol;

- a group of steroid hormone biomarkers consisting of dehydroepiandrosterone sulfate (DHEA-S), sex hormone binding globulin (SHBG), bio-available testosterone, total testosterone, free testosterone, and dehydroepiandrosterone (DHEA); and

- a group of sympathetic adrenal medullary axis biomarkers consisting of amylase (preferably alpha-amylase), epinephrine, and norepinephrine.

Statement 3. The method according to statement 1, wherein the fatigue syndrome is non-functional overreaching and/or overtraining syndrome and at least three biomarkers are independently selected from at least two, preferably at least three, more preferably at least four, different groups of biomarkers selected from:

- a group of neurotransmitter biomarkers consisting of anthranilic acid, cystathionine, picolinic acid, kynurenic acid (KYNA), L-kynurenine, quinolinic acid (QUIN), free tryptophan, xanthurenic acid, brain-derived neurotrophic factor (BDNF), S100 calcium-binding protein B (S100B), γ-aminobutyric acid (GABA), 3-hydroxyanthranilic acid, 3-hydroxykynurenine, Magnesium, Zinc, Glutamine, glutamaat, calcineurin, glutamate receptor, indoleamine (2,3)-dioxygenase (IDO), kynurenine mono-oxygenase, kynureninase, and kynurenine transaminase (KAT);

- a group of metabolism biomarkers consisting of N1-methylnicotinamide, flavin mononucleotide, creatinine, urea, vitamin D, folic acid, riboflavin, vitamin B12, pyridoxine, 4-pyridoxic acid, thiamine, nicotinic acid, nicotinamide, pyridoxal 5'-phosphate, niacin (vitamin B3), pyridoxal, trigonelline, thiamine monophosphate, visfatin, and cholecalciferol;

- a group of steroid hormone biomarkers consisting of dehydroepiandrosterone sulfate (DHEA-S), sex hormone binding globulin (SHBG), bio-available testosterone, total testosterone, free testosterone, and dehydroepiandrosterone (DHEA); preferably consisting of dehydroepiandrosterone sulfate (DHEA-S), sex hormone binding globulin (SHBG), bio-available testosterone, total testosterone, and free testosterone;

- a group of thyroid hormone biomarkers consisting of insulin-like growth factor (IGF-1), thyroid-stimulating hormone (TSH), insulin-like growth factor binding protein 3 (IGF-BP3), calcitonin, triiodothyronine (T3) and thyroxine (T4); preferably consisting of insulin-like growth factor (IGF-1) and thyroid-stimulating hormone (TSH);

- a group of iron metabolism biomarkers consisting of ferritin, transferrin, iron, transferrin saturation, hemoglobin in reticulocytes, erythrocytes, mean cell haemoglobin (MCH), mean cell volume (MCV), mean cell haemoglobin concentration (MCHC) and reticulocytes; and

- a group of hypothalamic-pituitary-adrenal (HPA) axis hormone biomarkers consisting of total cortisol, free cortisol, cortisol releasing hormone (CRH) and adrenocorticotropic hormone (ACTH).

Statement 4. The method according to any one of statements 1 to 3, comprising the steps of

(i) determining the status of said at least three biomarkers in the sample from the subject;

(ii) combining the status of said at least three biomarkers as determined in step (i) to a biomarker imbalance risk index (BIRIX);

(iii) finding a deviation or no deviation of the BIRIX compared to a reference value; and

(iv) attributing said finding of deviation or no deviation to a particular diagnosis, prediction, or

prognosis of the fatigue syndrome in the subject.

Statement 5. The method according to statement 4, wherein the BIRIX is obtained by multiplying the status of each of said at least three biomarkers by a corresponding weight, and wherein an expression signature defines the weight for each biomarker, preferably wherein the expression signature is defined using partial least squares-discriminant analysis (PLS-DA).

Statement 6. The method according to any one of statements 1 to 5, comprising detecting at least four, preferably at least five, more preferably at least six, biomarkers in a sample obtained from said subject.

Statement 7. A kit for predicting, diagnosing, prognosing and/or monitoring a fatigue syndrome selected from the group consisting of burn-out, depression, chronic stress, chronic fatigue syndrome, non-functional overreaching and overtraining syndrome in a subject, the kit comprising:

- means for measuring the status of at least three biomarkers independently selected from Table 1 in a sample obtained from said subject; and

- a reference value or means for establishing said reference value, wherein said reference value represents a known diagnosis, prediction and/or prognosis of the fatigue syndrome.

Statement 8. The method or the kit according to any of the preceding statementss, wherein said subject is an athlete, preferably a triathlon athlete, a cyclist, a runner, a rower or a soccer player.

Statement 9. The method or the kit according to any of the preceding statements wherein said subject is an employee.

Statement 10. The kit according to any of the preceding statements, wherein the kit further comprises a computer readable storage medium having recorded thereon one or more programs for carrying out the method of any one of statements 1 to 6, 8 or 9.

Statement 11. A computer program product for use in conjunction with a computer having a processor and a memory connected to the processor, said computer program product comprising a computer readable storage medium having a computer program mechanism encoded thereon, wherein said computer program mechanism is loaded into the memory of said computer and causes said computer to carry out the method of statements 1 to 6, 8 or 9.

Statement 12. An *in vitro* method for determining whether a subject is in need of therapeutic or prophylactic treatment of a fatigue syndrome selected from the group consisting of burn-out, depression, chronic stress, chronic fatigue syndrome, non-functional overreaching and overtraining syndrome, comprising detecting at least three, preferably at least four, biomarkers independently selected from Table 1 in a sample obtained from said subject.

Statement 13. An *in vitro* method for predicting the response of a subject diagnosed with a fatigue syndrome selected from the group consisting of burn-out, depression, chronic stress, chronic fatigue syndrome, non-functional overreaching and overtraining syndrome, to therapy, comprising detecting at least three, preferably at least four, biomarkers independently selected from Table 1 in a sample obtained from said subject.

The invention is further described by the following non-limiting examples which further illustrate the invention, and are not intended to, nor should they be interpreted to, limit the scope of the invention.

**EXAMPLES**

Example 1: Selection of biomarkers to detect onset and/or existence of fatigue syndromes

Biomarkers related to NFO/OTS/burn-out syndrome, CFS or depression disorder have been described in the past, however, they have often been tested only on a moderate number of patients or have often generated conflicting results and are therefore not considered reliable biomarkers for the detection of these conditions. Furthermore, up to now, researchers and medical doctors are mainly interpreting the biomarkers one by one. The inventors combine single biomarkers in a multidimensional approach and selected a number of factors to be tested as potential biomarkers for the onset and/or existence of the fatigue syndromes and /or disorders using standard sampling and assay methods in a clinically relevant setup. The markers were tested during a scientific experiment in a subpopulation of mostly recreational athletes with potential to develop NFO/OTS and/or burnout.

The experiment consisted of 300 endurance sports athletes who were monitored during the course of one training season. A training season was subdivided in different phases starting with a maintenance phase, then a basic training phase, a development phase, a peak phase, a tapering phase and eventually a challenge phase. Apart from their sport activities the participants of the study also have a professional occupation and often care of their family.

The fatigue and stress status of the athletes was estimated during the course of the training season using three different indicators. First, athletes reported on their psychological and physical mood on a daily basis by filling in a diary. In this diary, the intensity of the training session (if any) of that day was recorded by the patient as the rating of perceived exertion (RPE), measured according to the Borg RPE scale, which is known to the skilled person, together with the duration of the training. Furthermore the psychological status of the athlete was recorded on a daily basis by answering 7 questions in the diary. Second, the athletes completed the "Profile of Mood States" (POMS) questionnaire on a weekly basis. POMS is a simple psychological rating scale used to assess transient, distinct mood states. Finally, the stress and recovery of the athletes was recorded at sampling points (see next paragraph) using the "Recovery-Stress Questionnaire for Athletes" (RESTQ-Sport) which allows the simultaneous assessment of stress and recovery of the athlete.

Additionally, four blood and saliva samples were analyzed at sampling points spread over the course of the training season. Upon indication of the onset or occurrence of non-functional overreaching based on the information provided in the diary and POMS, two additional blood and saliva samples were taken: one before the introduction and one after termination of an additional recovery period. All samples were taken after a 36 hour rest period or a 36 hour period with light training. More than 100 biomarkers were analyzed in the blood and saliva samples.

When all the information was combined, it resulted in a unique dataset, which was never before available. All data was subjected to extensive quality control to assure the correctness of the data. To identify the participants with increased risk for NFO/overtraining/burn-out/depression, the answers on the different questionnaires were interpreted as described in literature or with help from experts in the field.

The samples were subdivided in 3 subgroups: a prediction, verification and validation group, to select those biosignatures that showed confident trends in all groups.

Firstly, the discriminative performance of each individual variable was quantified using the area under the receiver operating curve (AUC) and positive and negative predictive values (respectively PPV and NPV). An AUC of 0.5 or lower indicates an absence of predictive power for the outcome.

Secondly, Partial Least Squares - Discriminant Analysis (PLS-DA) models were generated for all combinations of biomarkers and possible clinical predictors as identified earlier.

The following statistical methodology was applied:

- The concentrations and relative concentrations of biomarkers were log-transformed before modelling.

    - PLS-DA multivariable models were made by selecting variables as follows:

        - 1 to $N_p$ predictors.
        - All possible combinations of variables.

- A three-fold cross validation was performed stratifying over the outcomes.
- Models were additionally trained on the entire dataset.
- Models were selected corresponding to models that have the best statistically significant prognostic performance for the outcome within the subgroup studied.

The statistical analysis described above showed that none of the univariate biomarkers showed sufficient power to discriminate between people with low or high risk for fatigue syndromes (AUC <0.656; Table 12).

**Table 12: AUC statistical analysis of univariate versus multivariate (LAS, MAS) approach AUC**

| Risk outcome | NFO/OTS | Reduced mental fitness |
|---|---|---|
| Leukocytes | 0.548 | 0.532 |
| secretory Immunoglobulin A | 0.525 | 0.520 |
| Iron | 0.606 | 0.624 |
| Transferrin saturation | 0.558 | 0.636 |
| Transferrin saturation | 0.574 | 0.557 |
| Ferritin | 0.527 | 0.560 |
| creatine kinase | 0.520 | 0.509 |
| Prolactin | 0.527 | 0.656 |
| Cortisol | 0.555 | 0.513 |
| DHEA-S | 0.585 | 0.617 |
| Testosteron | 0.555 | 0.601 |
| DHEA-S/cortisol | 0.582 | 0.510 |
| Testosteron/Cortisol | 0.563 | 0.533 |
| | | |
| LAS | 0.855 | 0,817 |
| MAS | 0.634 | 0.856 |

Using the statistical method as described, the best performing combinations of biomarkers (biosignatures) were selected to calculate the BIRIX.

Strikingly, the inventors found a unique and surprising correlation between the statuses of several of the biomarkers (biosignatures) tested and the onset and/or existence of fatigue syndromes such as, but not limited to NFO, OTS, burn-out, CFS or depression disorder. Depending on the outcome of the fatigue syndrome (e.g. overtraining, burnout, depression) a different BIRIX score could be calculated based on the measures of a specific set of biomarkers (biosignatures), which is specific for gender and age. As an example of the usefulness of the BIRIX, we provide 3 more examples: a load adaptation score (LAS), which explains how an individual reacts upon physical training stress and provides a risk index for the onset of NFO/overtraining (example 2); the mental adaptation score (MAS), which defines how an individual reacts upon mental stressors, such as work environment (example 3); and training adaptation score (TAS), which defines how team athletes are coping with the external training load taking into account their personal life, their individual characteristics and the groups and organizational pressure (example 4).

The biomarkers included in this dataset are disclosed in Table 1.

Based on this dataset, the inventors have thus identified several biomarker panels or combinations of biomarkers (biosignatures) whose status can be used to detect the onset and/or the existence of fatigue syndromes including NFO, OTS, burn-out syndrome, CFS and depression disorder.

The panels of biomarkers are combined in specific BIRIX formulae. An example of the formulae is provided below. Nevertheless, other variants of the formulae combining different biomarkers or biosignatures are possible.

$$sqrt((f(\alpha * f(\log[Biomarker1]\,10, time) + \beta * f(\log[Biomarker2]\,10, age) + \gamma$$
$$* f(\log[Biomarker3]\,10, age), gender) - s)^2)$$
$$+ ((f(\delta * f(\log[Biomarker4/Biomarker5]\,10, age * time) + \varepsilon$$
$$* f(\log[Biomarker6]\,10, age))^2)) - t)^2)$$

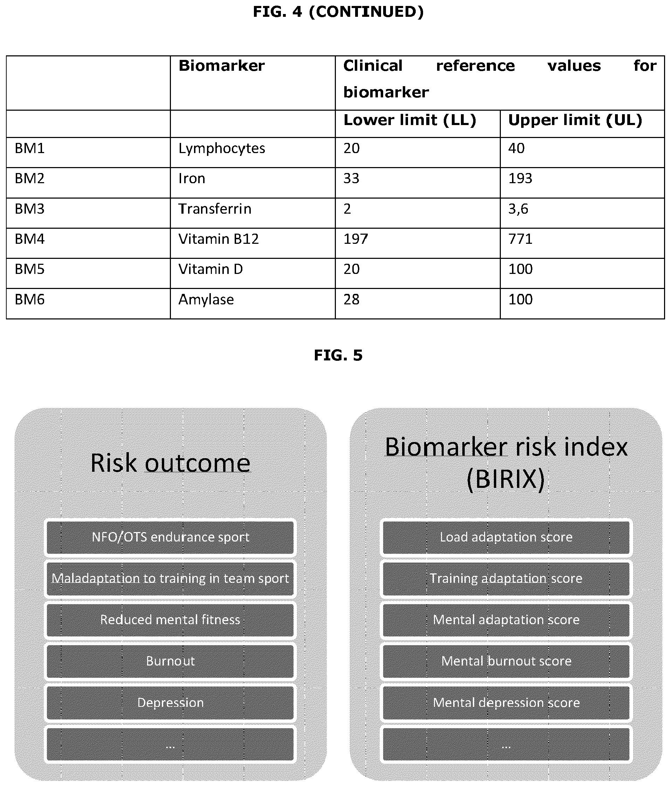

The BIRIX were evaluated using ROC-curves. Therefore an AUC of at least 0.70 was accepted, with a sensitivity and specificity above 70%. In Figure 5, the ROC curve of the LAS score is compared to the ROC curves of two biomarkers that are currently used in practice to identify the risk and/or existence of NFO/overtraining. As is shown, the performance of the LAS is significantly (p<0.001) better compared to the univariate interpretation (Table 13).

**Table 13: comparison of ROC curves of LAS and two commonly used univariate biomarkers**

| Parameter | AUC | Sens@spec80 | spec@sens80 |
|---|---|---|---|
| LAS | 0.855 | 83.33 | 82.17 |
| DHEA-S/Cortisol | 0.582 | 27.78 | 40.46 |
| Testosteron/Cortisol | 0.563 | 19.44 | 39.69 |

Example 2: Selection of biomarkers to predict, diagnose, prognose and/or monitor non-functional overreaching (NFO) and/or overtraining syndrome

The fatigue status of 243 recreational athletes was monitored using a method as taught herein, more precisely using the LAS (load adaptation score; BIRIX for NFO and/or overtraining syndrome).

Of these 37 subjects showed clear signs of non-functional overreaching and/or overtraining syndrome (patient group). Control subjects did not report any problems (nor physically or mentally) during their training schedule.

*Univariate approach*

Discriminative performance of individual variables was quantified using the area under the receiver operating curve (AUC). An AUC of 0.5 or lower indicates an absence of predictive power for the outcome.

| Biomarker | AUC |
|---|---|
| Anthranilic acid | 0,653 |
| Cortisol | 0,527 |
| Creatinin | 0,504 |
| Erythrocytes | 0,488 |
| Free testosteron | 0,622 |
| IGF-1 | 0,525 |
| N1-methylnicotinamide | 0,541 |
| Thiamine | 0,623 |
| Vitamin D | 0,512 |
| Bioavailable testosteron | 0,634 |
| Dehydroepiandrosterone sulfate (DHEA-S) | 0,628 |
| Reticulocytes | 0,548 |
| Thyroid-stimulating hormone (TSH) | 0,519 |
| Total testosteron | 0,546 |

The univariate biomarkers were normalized in a similar manner as the biomarkers in the multivariate approach.

None of the univariate biomarkers showed sufficient power to discriminate between subjects with low or high risk for functional overreaching and/or overtraining syndrome.

*Multivariate approach as taught herein*

| Biomarker set | Combination | Biomarkers |
|---|---|---|
| Biomarker set 1 | 8 biomarkers from 5 different groups of biomarkers | Reticulocytes (group 5), Testosterone (free or bioavailable) (groups 3 and 3a), Cortisol (group 6), IGF-1 (group 4 and group 4a), DHEA-S (group 3 and 3a), Creatinin (groups 2 and 2a), Vitamin D (groups 2 and 2a) and Erythrocytes (group 5) |
| Biomarker set 2 | 5 biomarkers from 4 different groups of biomarkers | Reticulocytes (group 5), Testosterone (free or bioavailable) (groups 3 and 3a), DHEA-S (group 3 and 3a), Cortisol (group 6), and IGF-1 (group 4 and group 4a) |

(continued)

| Biomarker set | Combination | Biomarkers |
|---|---|---|
| Biomarker set 3 | 4 biomarkers from 4 different groups of biomarkers | Reticulocytes (group 5), Creatinin (groups 2 and 2a), IGF-1 (group 4 and group 4a), Vitamin D (groups 2 and 2a) |
| Biomarker set 4 | 4 biomarkers from 3 different groups of biomarkers | DHEA-S (group 3 and 3a), Testosterone (free or bioavailable) (groups 3 and 3a), Anthranilic acid (groups 1 and 1a), N1-methylnicotinamide (groups 2 and 2a) |
| Biomarker set 5 | 4 biomarkers from 4 different groups of biomarkers | Bioavailable testosteron, Anthranilic acid (groups 1 and 1a), TSH (groups 4 and 4a), N1-methylnicotinamide (groups 2 and 2a) |
| Biomarker set 6 | 4 biomarkers from 4 different groups of biomarkers | Bioavailable testosterone (groups 3 and 3a), Anthranilic acid (groups 1 and 1a), TSH (groups 4 and 4a), Thiamine (groups 2 and 2a) |
| Biomarker set 7 | 3 biomarkers from 3 different groups of biomarkers | Bioavailable testosterone (groups 3 and 3a), Anthranilic acid (groups 1 and 1a), TSH (groups 4 and 4a) |

Sets of biomarkers were selected using the statistical method as described in Example 1. Good performing combinations of biomarkers (biosignatures or biomarker sets) were selected to calculate the load adaptation score (LAS), as described in Example 1.

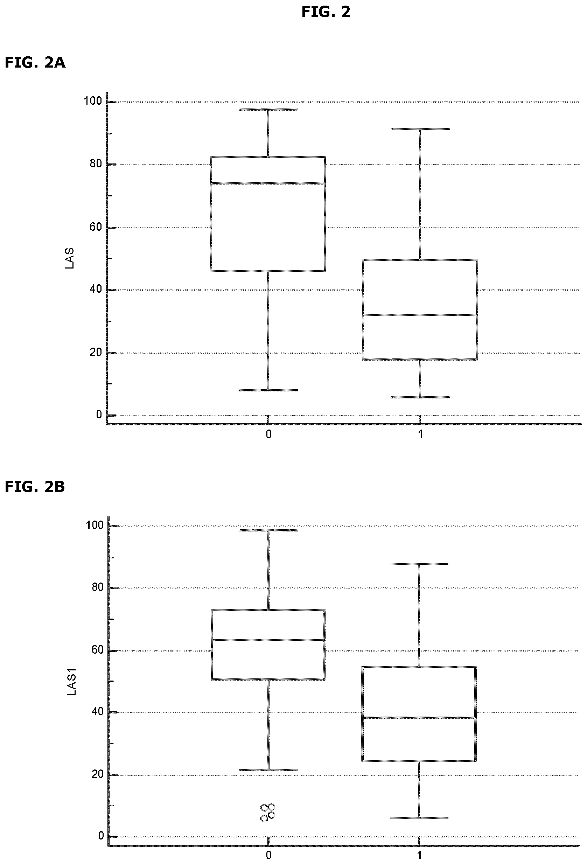

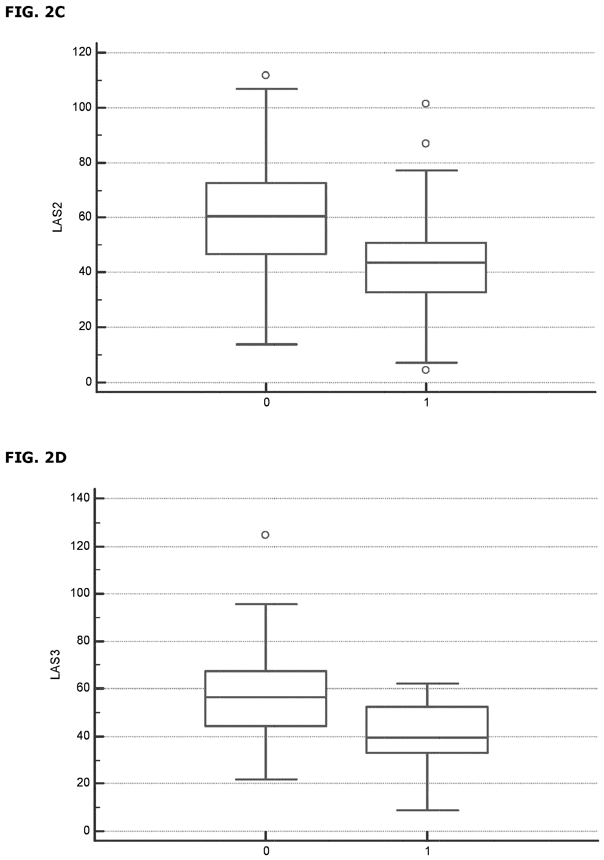

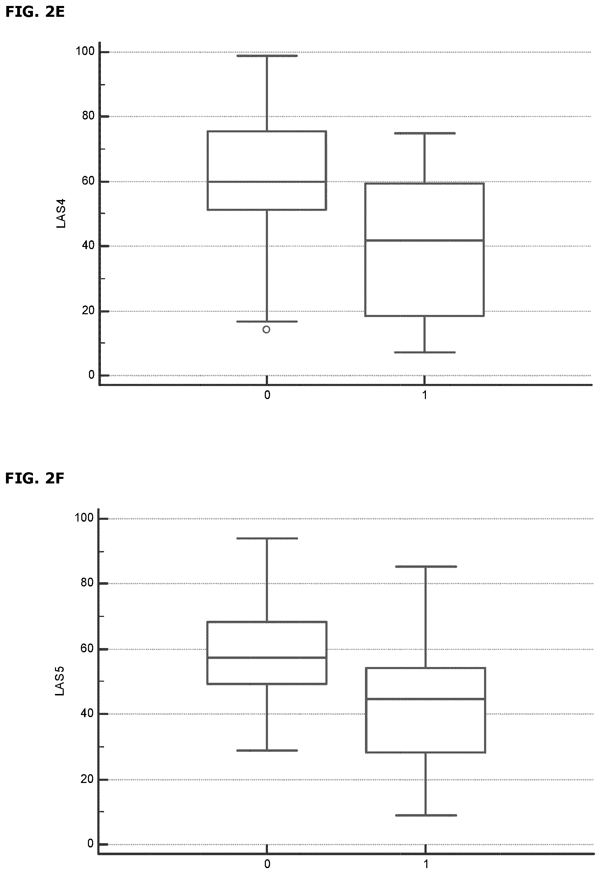

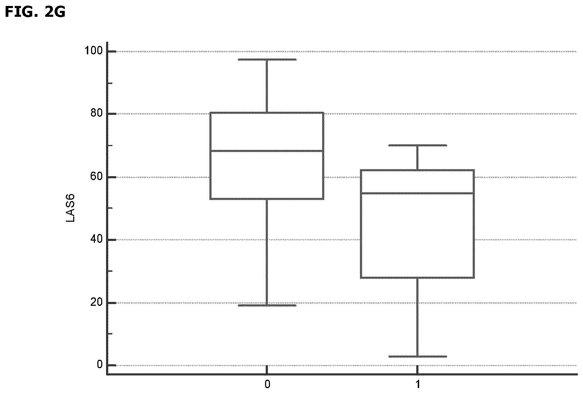

From the boxplots in Figure 2 it is clear that samples of individuals where no onset nor existence of functional overreaching and/or overtraining syndrome was detected ("0" in Figure 2) could be discriminated using biomarker sets 1, 2, 3, 4, 5, 6 and 7 with a high negative and/or high positive predictive value from the samples obtained from individuals suffering from functional overreaching and/or overtraining syndrome ("1" in Figure 2).

The LAS was evaluated using ROC-curves. Therefore, an AUC of at least 0.70 was accepted, with a sensitivity and specificity above 70%.

| LAS (biomarker set used to calculate LAS) | Figure 2 | AUC |
|---|---|---|
| LAS (Biomarker set 1) | Figure 2a | 0.791 |
| LAS1 (Biomarker set 2) | Figure 2b | 0.765 |
| LAS2 (Biomarker set 3) | Figure 2c | 0.727 |
| LAS3 (Biomarker set 4) | Figure 2d | 0.808 |
| LAS4 (Biomarker set 5) | Figure 2e | 0.769 |
| LAS5 (Biomarker set 6) | Figure 2f | 0.718 |
| LAS6 (Biomarker set 7) | Figure 2g | 0.748 |

The LAS based on a set of at least three biomarkers independently selected from at least three, preferably at least four, different groups of biomarkers selected from neurotransmitter biomarkers (groups 1 and 1a), metabolism biomarkers (groups 2 and 2a), steroid hormone biomarkers (groups 3 and 3a), thyroid hormone biomarkers (groups 4 and 4a), iron metabolism biomarkers (group 5), and hypothalamic-pituitary-adrenal (HPA) axis hormone biomarkers (group 6) as described herein performs better (e.g. increased the AUC) to discriminate between people with low or high risk for functional overreaching and/or overtraining syndrome compared to the univariate interpretation. Individuals with a high risk for functional overreaching and/or overtraining syndrome typically have a LAS of less or equal to 40. On the other hand, individuals with a low risk for functional overreaching and/or overtraining syndrome typically have a LAS higher or equal to 60.

Example 3: Selection of biomarkers to predict, diagnose, prognose and/or monitor chronic stress

A group of 212 employees with a demanding job and/or personal life and thus a risk for chronic stress, preferably chronic mental stress, as a prelude for burn-out or depression, was monitored using a method as taught herein, more precisely using the MAS (mental adaptation score). 36 subjects showed clear disturbances in their answers on multiple psychological questionnaires (patient group). In the control population no disturbances in the psychological questionnaires were reported.

*Univariate approach*

Discriminative performance of individual variables was quantified using the area under the receiver operating curve (AUC). An AUC of 0.5 or lower indicates an absence of predictive power for the outcome.

| Biomarker | AUC |
| --- | --- |
| anthranilic acid | 0.510 |
| amylase | 0.557 |
| creatinine | 0.573 |
| dehydroepiandrosterone sulfate (DHEA-S) | 0.515 |
| free cortisol | 0.544 |
| hemoglobin in reticulocytes | 0.647 |
| iron | 0.676 |
| leukocytes | 0.536 |
| lymphocytes | 0.642 |
| MCH | 0.612 |
| monocytes | 0.502 |
| transferrin | 0.587 |
| transferrin saturation | 0.629 |
| urea | 0.506 |
| vitamin B12 | 0.512 |
| pyridoxal 5'-phosphate | 0.695 |
| vitamin D | 0.665 |
| 3-hydroxyanthranilic acid | 0.572 |
| 3-hydroxyisobutyrate | 0.691 |
| 3-hydroxykynurenine | 0.523 |
| 4-pyridoxic acid | 0.692 |
| interleukin 1 receptor antagonist (IL-Ira) | 0.596 |
| C-reactive protein (CRP) | 0.558 |
| cystathionine | 0.523 |
| IL-6 | 0.594 |
| folic acid | 0.530 |
| Free testosterone | 0.513 |
| growth hormone | 0.562 |
| IL-10 | 0.643 |
| flavin mononucleotide | 0.597 |

(continued)

| Biomarker | AUC |
|---|---|
| kynurenic acid (KYNA) | 0.554 |
| L-kynurenine | 0.528 |
| magnesium | 0.530 |
| MCHC | 0.651 |
| myeloperoxidase (MPO) | 0.597 |
| N 1-methylnicotinamide | 0.573 |
| nicotinamide | 0.660 |
| picolinic acid | 0.648 |
| pyridoxal | 0.632 |
| secretory Immunoglobulin A (sIgA) | 0.503 |
| quinolinic acid (QUIN) | 0.514 |
| reticulocytes | 0.618 |
| riboflavin | 0.689 |

The univariate biomarkers were normalized in a similar manner as the biomarkers in the multivariate approach.

None of the univariate biomarkers showed sufficient power to discriminate between subjects with low or high risk for maladaptation towards chronic stress.

*Multivariate approach as taught herein*

| Biomarker set | Combination | Biomarkers |
|---|---|---|
| Biomarker set 1 | 9 biomarkers from 5 different groups of biomarkers | Vitamin B12 (groups 10 or 10a, group 300, or 300a), Vitamin D (groups 10 or 10a, group 300 or 300a), Iron (group 9, group 200 or 200a), Transferrin (group 9, group 200 or 200a), Lymphocytes (group 8, group 100), DHEA-S (groups 11 or 11a, group 400 or 400a), Urea (groups 10 or 10a, group 300 or 300a), Creatinine (groups 10 or 10a, group 300 or 300a), Amylase (group 12, group 500 or 500a) |
| Biomarker set 2 | 6 biomarkers from 4 different groups of biomarkers | Vitamin B12 (groups 10 or 10a, group 300 or 300a), Vitamin D (groups 10 or 10a, group 300 or 300a), Iron (group 9, group 200 or 200a), Transferrin (group 9, group 200 or 200a), Lymphocytes (group 8, group 100), Amylase (group 12, group 500 or 500a) |
| Biomarker set 3 | 4 biomarkers from 4 different groups of biomarkers | Transferrin saturation (group 9, group 200 or 200a), DHEA-S (groups 11 or 11a, group 400 or 400a), Urea (groups 10 or 10a, group 300 or 300a), Amylase (group 12, group 500 or 500a) |
| Biomarker set 4 | 6 biomarkers from 4 different groups of biomarkers | DHEA-S (groups 11 or 11a, group 400 or 400a), Transferrin saturation (group 9, group 200 or 200a), Vitamin D (groups 10 or 10a, group 300 or 300a), Urea (group 10, group 300 or 300a), Creatinine (groups 10 or 10a, group 300 or 300a), Amylase (group 12, group 500 or 500a) |
| Biomarker set 5 | 3 biomarkers from 2 different groups of biomarkers | IL-1RA (group 8, group 100 or 100a), 4-Pyridoxic acid (groups 10 or 10a, group 300 or 300a), Riboflavin (groups 10 or 10a, group 300 or 300a) |

(continued)

| Biomarker set | Combination | Biomarkers |
|---|---|---|
| Biomarker set 6 | 4 biomarkers from 3 different groups of biomarkers | Amylase (group 12, group 500 or 500a), 4-Pyridoxic acid (group 10 or 10a, group 300 or 300a), iron (group 9, group 200 or 200a), transferrin (group 9, group 200 or 200a) |
| Biomarker set 7 | 4 biomarkers from 4 different groups of biomarkers | Transferrin (group 9, group 200 or 200a), Amylase (group 12, group 500, or 500a), 4-Pyridoxic acid (groups 10 or 10a, group 300 or 300a), Free testosterone (groups 11 or 11a, group 400 or 400a) |

Sets of biomarkers were selected using the statistical method as described in Example 1. Good performing combinations of biomarkers (biosignatures or biomarker sets) were selected to calculate the mental adaptation score (MAS), as described in Example 1.

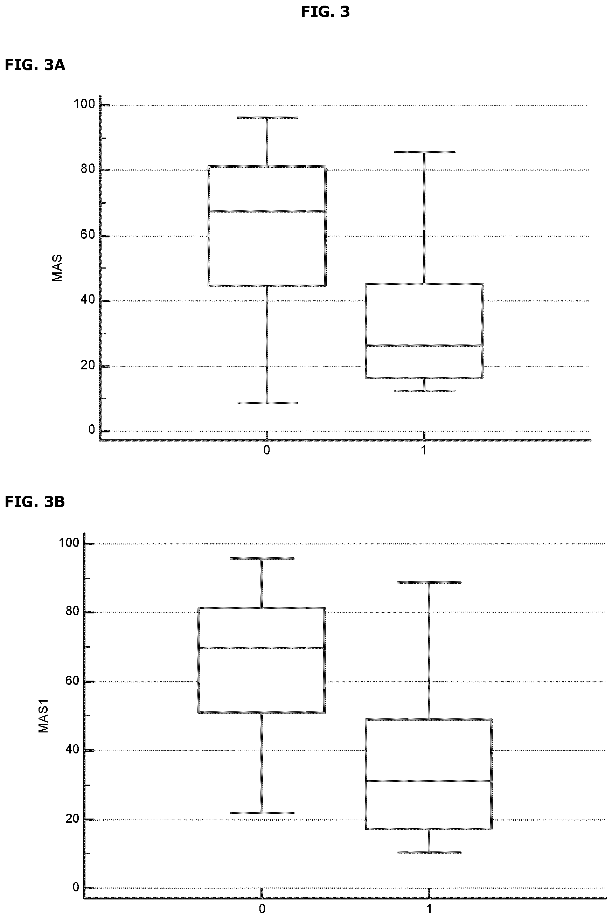

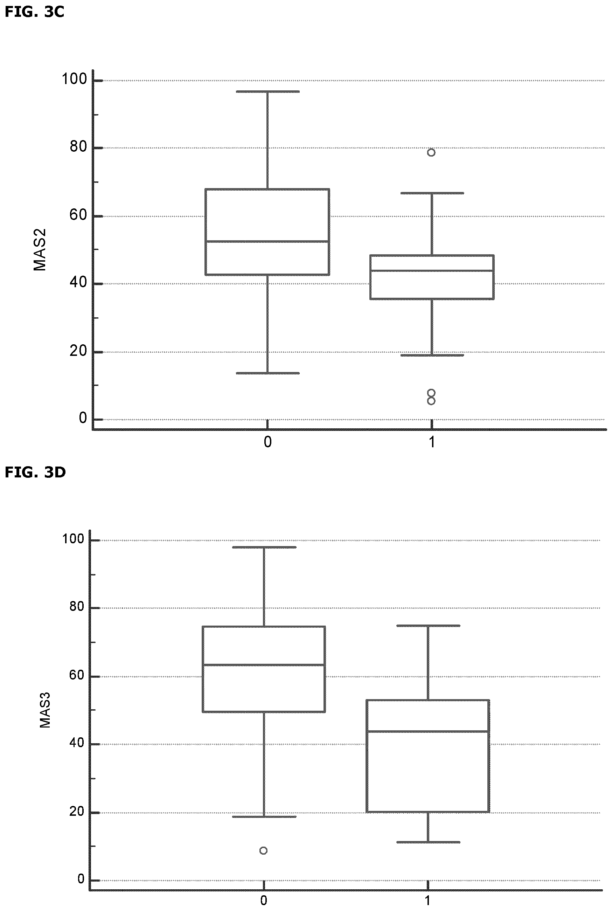

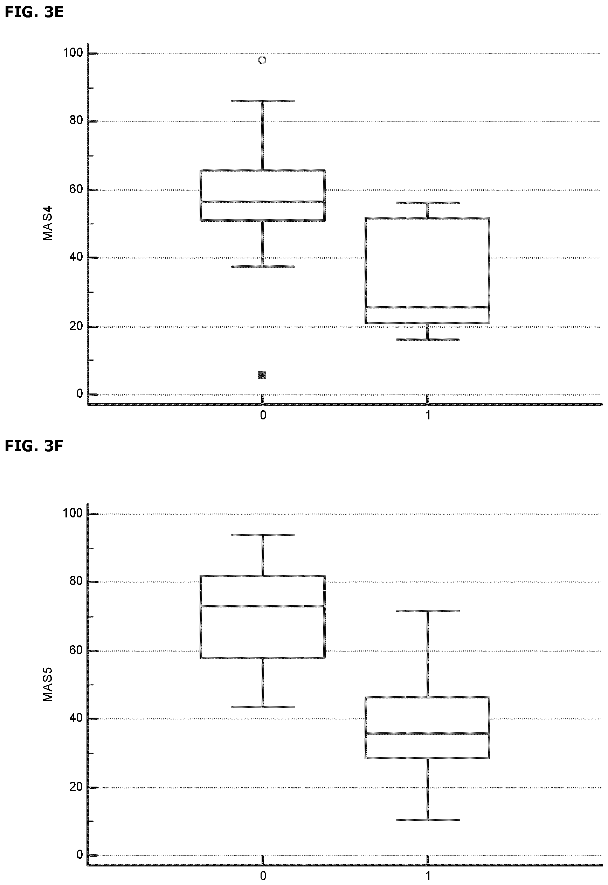

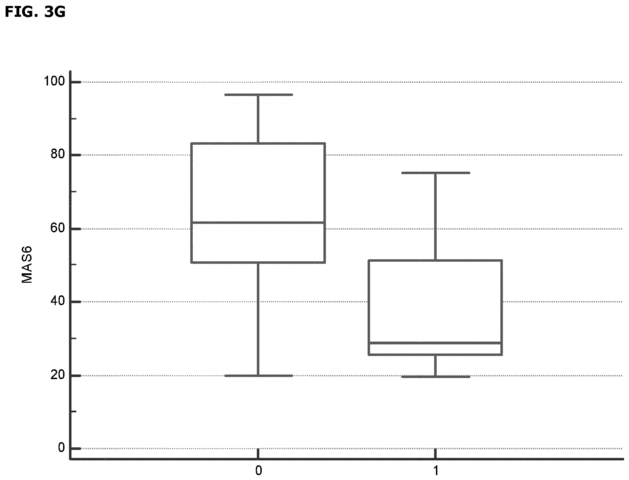

From the boxplots in Figure 3 it is clear that samples of individuals where no onset nor existence of chronic stress was detected ("0" in Figure 3) could be discriminated using biomarker sets 1, 2, 3, 4, 5, 6 and 7 with a high negative and/or high positive predictive value from the samples obtained from individuals suffering from chronic stress ("1" in Figure 3).

The MAS was evaluated using ROC-curves. Therefore, an AUC of at least 0.70 was accepted, with a sensitivity and specificity above 70%. Preferably, the AUC is at least 0.80, with a sensitivity and specificity above 80%

| MAS (biomarker set used to calculate MAS) | Figure 3 | AUC |
|---|---|---|
| MAS (Biomarker set 1) | Figure 3a | 0.854 |
| MAS 1 (Biomarker set 2) | Figure 3b | 0.826 |
| MAS2 (Biomarker set 3) | Figure 3c | 0.709 |
| MAS3 (Biomarker set 4) | Figure 3d | 0.799 |
| MAS4 (Biomarker set 5) | Figure 3e | 0.836 |
| MAS5 (Biomarker set 6) | Figure 3f | 0.909 |
| MAS6 (Biomarker set 7) | Figure 3g | 0.818 |

The MAS based on a set of at least three biomarkers independently selected from at least two, preferably at least three, more preferably at least four, different groups of biomarkers selected from neurotransmitter biomarkers, immune function biomarkers, iron metabolism biomarkers, metabolism biomarkers, steroid hormone biomarkers, and sympathetic adrenal medullary axis biomarkers as described herein performs better (e.g. increased the AUC) to discriminate between people with low or high risk for chronic stress compared to the univariate interpretation.

Chronic stress can be a precursor of burn-out and/or depression. Accordingly, the MAS based on a set of at least three biomarkers independently selected from at least two, preferably at least three, more preferably at least four, different groups of biomarkers selected from neurotransmitter biomarkers, immune function biomarkers, iron metabolism biomarkers, metabolism biomarkers, steroid hormone biomarkers, and salivary amylase hormone biomarkers as described herein also allows to discriminate between people with low or high risk for burn-out and/or depression.

Individuals with a high risk for chronic stress, preferably chronic mental stress, (and consequently also for burn-out and/or depression) typically have a MAS of less or equal to 40. On the other hand, individuals with a low risk for chronic stress, preferably chronic mental stress, (and consequently also for burn-out and/or depression) typically have a MAS higher or equal to 60.

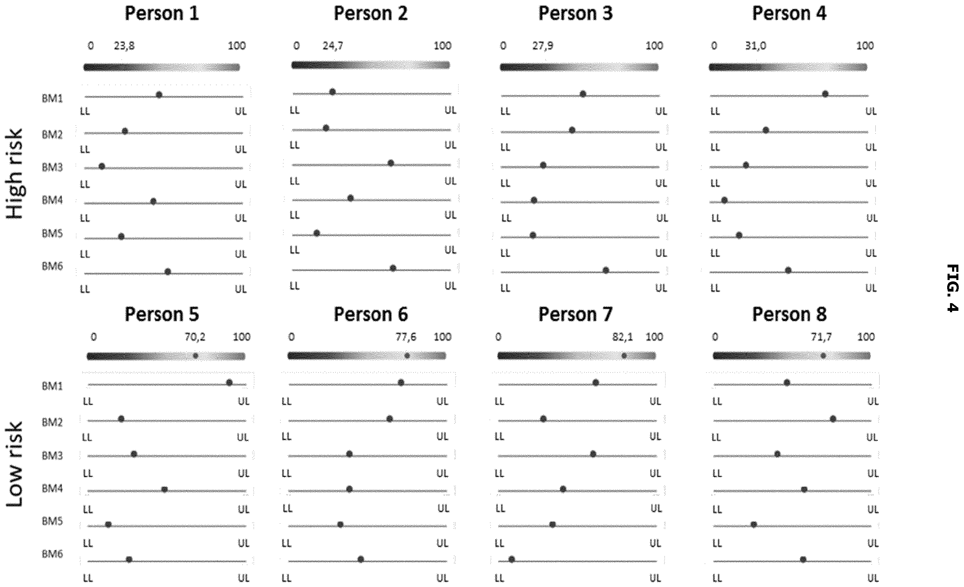

For example, the MAS for biomarker set 2 of 8 individual subjects is shown in Figure 4.

Although each biomarker detected lies within the lower and upper range of the clinical reference values for said biomarker, the method as taught herein allows to discriminate between people with low or high risk for chronic stress as a result of the multidimensional approach.

Individuals (Figure 4, persons 1-4) with a high risk for chronic stress have a MAS of at most 40, preferably of at most 35. On the other hand, individuals with a low risk for chronic stress (and consequently also for burn-out and/or depression) (Figure 4, persons 5-8) typically have a MAS of at least 60, preferably at least 70.

Example 4: Detecting the onset of overtraining syndrome in an individual by measuring the load adaptation score

The fatigue status of a recreational athlete was monitored using a method according to an embodiment of the invention, more precisely the LAS (load adaptation score).

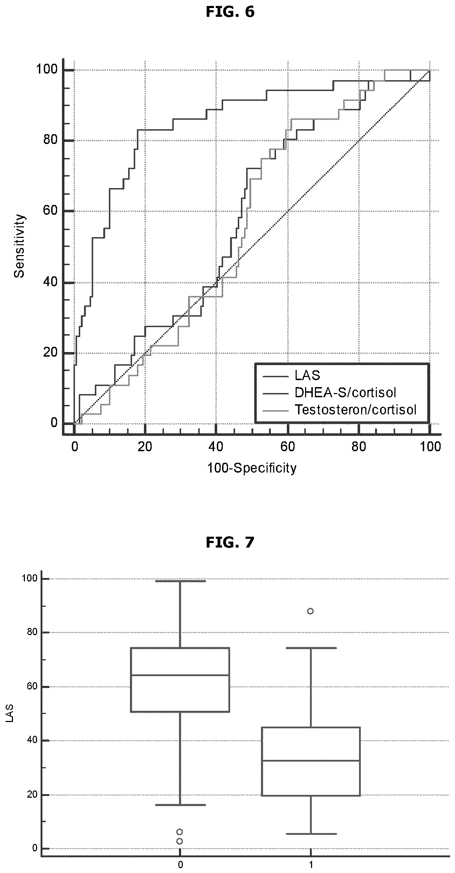

In Figure 6 the relevance of the selected biomarkers and biomarker combinations (biosignature) is illustrated where, according to an embodiment of the in vitro method of the invention, the status of three or more of these biomarkers was determined in 165 samples. From the boxplot in Figure 7 it is clear that samples of individuals where no onset nor existence of a fatigue condition was detected ("0" in Figure 7) could be discriminated with a high negative predictive value form the samples obtained from individuals suffering from non-functional overreaching ("1" in Figure 7).

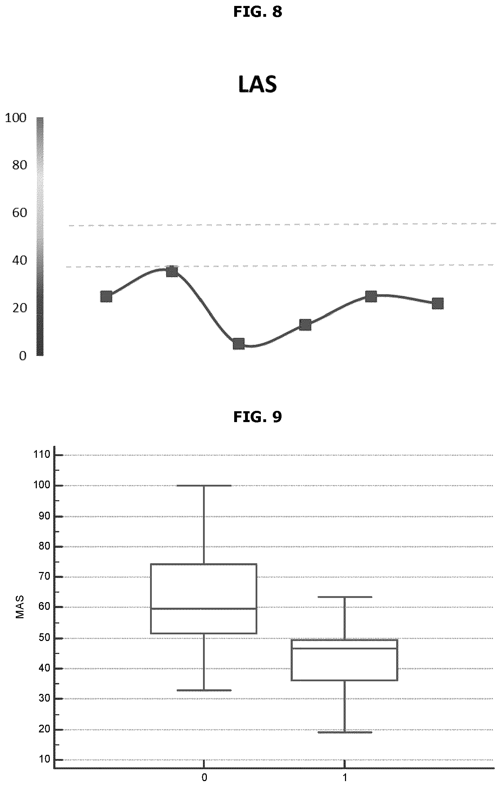

In Figure 8, the profile of the LAS of an athlete during the training towards an Iron Man is shown. At fixed time points during his training period, saliva and serum samples were taken from the athlete and analyzed. At a regular base, a brief questionnaire was provided and the training sessions were monitored using wearables, to assure close follow-up and specific advice by the medical team during the training.

At the third fixed sampling point (8 weeks before the Iron Man), the prognostic value of the LAS pointed towards an increased risk for non-functional overreaching. Due to the result of the test the athlete, the medical doctor, coach and athlete agreed to lower the amount of training volume for 1.5 week. After accomplishing a deadline at work, the stress level of the athlete was reduced, and the training intensity was subsequently increased again. They agreed that indeed the current work load combined with the training was too intensive. The results of the next follow-up sample 1 month later showed a higher LAS score together with a better recovery of the athlete. Thanks to the biomarker analysis the development of overtraining was prevented and the athlete managed to perform at his personal best during the Iron Man.

It is supposed that the present invention is not restricted to any form of realization described previously and that some modifications can be added to the presented example without reappraisal of the appended claims. For example, the present invention has been described referring to NFO/OTS, but it is clear that the invention can be applied to other fatigue syndromes.

Example 5: Screening of employees for the onset of burnout syndrome by measuring the mental adaptation score

A group of 35 employees at a firm was monitored during 12 months using a method according to an embodiment of the invention, more precisely the MAS, in order to detect the onset and/or existence of fatigue syndromes, particularly of burn-out.

In Figure 9 the boxplots of the MAS, the status of three or more of these biomarkers (biosignatures) as an embodiment of the in vitro method of the invention based on measurements in 92 samples, is shown. It is clear that the people with an increased stress/fatigue level as a possible precursor of burn-out or depression ("1" in Figure 9) have a higher MAS score compared to the people where no onset or existence of a fatigue condition was detected ("0" in Figure 9).

More in detail, the fatigue status of the employees was measured every three months by analyzing saliva and/or serum samples of each individual. Furthermore a brief two-weekly questionnaire was provided to assure close follow-up and to enable introducing extra sampling points when needed. The human resources department of the firm, which was responsible for the screening received overviews of the objective MAS-scores in the different departments to identify those departments where extra coaching is needed.

Furthermore, all employees received their personal MAS together with some practical advice. In case a high risk was detected, the employee received private sessions with a psychologist/mental coach. Extra blood and saliva samples were taken for the follow up of this employee.

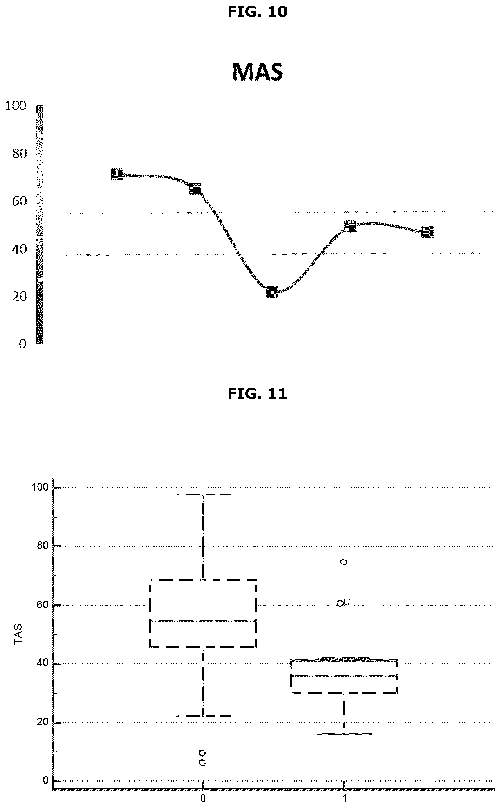

In Figure 10, the MAS profile of an employee was shown. At the third sampling point (during the third quarter), the prognostic value of the test pointed towards an increased risk for fatigue syndromes such as burn-out in one of the employees. This employee was notified and he contacted his medical doctor for a more elaborated diagnosis (e.g. mental burnout score). The medical doctor decided that the results of these test pointed towards an increase wrought-out of the employee. The employee's medical doctor and the human resources department decided to reorganize the tasks of the employee, such as more structured deadlines and clear communication with his supervisor. This resulted in a decrease in the stress pattern of the employee and after one month, the normal workload was restored. The results of the next sampling (during the fourth quarter) also pointed towards the recovery of the employee. Thanks to the screening method, the development of burn-out in this employee was prevented and the employee was spared of a persistent fatigue condition that would otherwise have had a negative effect both on work-related aspects and on his personal life. Furthermore the costs associated with the absenteeism of the employee was avoided.

It is supposed that the present invention is not restricted to any form of realization described previously and that some modifications can be added to the presented example without reappraisal of the appended claims. For example, the present invention has been described referring to burn-out, but it is clear that the invention can be applied for instance to chronic fatigue syndrome or to depression disorder.

Example 6: Follow up of the training adaptation in a professional soccer team

The training efficiency in team sport athletes was monitored using a method according to an embodiment of the invention, more precisely the TAS (training adaptation score). In team sports training sessions are often in groups, hardly differentiated depending on the training capacity and adaptation possibilities of the athlete. Nevertheless, none of the athletes in a team are the same. They all have a different mental and physical load during games and their home situations are often different as some of them are living apart from their family in a foreign country, while others have a partner and children to look after.

In Figure 11 the relevancy of the selected biomarkers and biomarker combinations (biosignature) is illustrated where, according to an embodiment of the in vitro method of the invention, the status of three or more of these biomarkers (biosignatures) was determined in 100 samples. From the boxplot in Figure 11 it is clear that samples of individuals that benefit from the group training sessions ("0" in Figure 11) could be discriminated form the samples obtained from individuals suffering from fatigue and maladaptation towards the training sessions ("1" in Figure 11).

A pro-league soccer team was monitored during one season. Every 6 weeks the TAS of all team players was determined by analyzing their blood and saliva samples. An overview of the BIRIX was provided to the physical coach who was able to personalize the intensive training sessions to reach the maximal potential of every player.

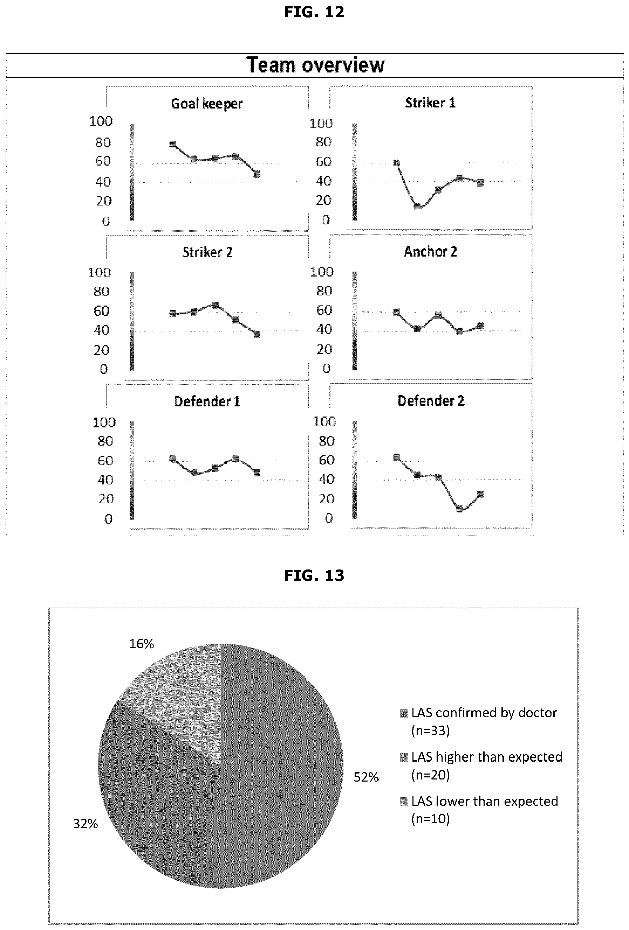

At the second sampling point, at the beginning of the competition period one of the strikers (striker 1 in Figure 12) was flagged in the TAS. After profound interaction with the striker, it appeared that he did not respect the off-season rest-period. As he had a severe injury at the end of previous season he was convinced that he needed to have extra training sessions to reach his previous potential. Nevertheless, the lack of a rest period combined with an intensive training camp, resulted in the overload of the athlete. As a result the striker was not able to perform at his maximal capacity during sufficient time during the game. A more personalized training schedule was set up allowing the player to recover, which was also visualized in the following TAS scores.

At the 4th sampling point defender 2 showed an decrease in the TAS score. A mental questionnaire which was filled at the sampling time showed an increase in fatigue and loss of concentration. The sport psychologist further discussed the answers on the questionnaire with the athlete. It became clear that the care of a sick child had a huge demand of the player. The nights were not as they used to be, resulting in a build-up of sleep shortage. The player did not notify the physical coach and trainer as he was afraid to be excluded from the starting team. The medical staff discussed this case and decided to give the defender extra credits to miss the early morning sessions. His personal feedback moments with the coach/medical doctor/psychologist were rescheduled, allowing the player to be more relaxed in the morning. By this small interference, the athlete was more relaxed and his fatigue feeling was restored, resulting in a better concentration and performance at match days.

By using the TAS analyses in the follow up of the athletes, the pro league team was able to objectively identify those athletes that are in need of an individual approach. In this way, the athletes were able to perform at their personal best during the matches, resulting in very good results and gain against their most feared opponents.

It is supposed that the present invention is not restricted to any form of realization described previously and that some modifications can be added to the presented example without reappraisal of the appended claims. For example, the present invention has been described for a soccer team, but it is clear that the invention can be applied for instance for other team sports like volleyball, handball, hockey...

Example 7: Evaluation of the value of the BIRIX for non-functional overreaching in the medical sport doctor's practice

As evaluation of the value of the Load adaptation score (LAS; BIRIX for NFO) in practice, medical sport doctors were asked to give a score for non-functional overreaching for the endurance athletes based on their insights and experience. The LAS was compared with the score given by the doctor. In half of the cases (52%) the LAS corresponded well with the evaluation of the medical doctor. In quite a lot of cases (32%) the LAS was higher than expected and in 16% of the cases was lower than expected (Figure 13).

This is not surprising as the doctors already indicated that they have no good tools to detect NFO and all had their own way of diagnosing NFO. After reporting the scores, a second interaction with the medical sport doctors was planned. In most cases (85%) the LAS score enabled them to interact with the athletes and to agree upon a new training strategy. In the other cases (15%) there was either no follow-up of the athlete or there was a skeptical attitude towards the new test (BIRIX). In 41% of the cases, the doctor would not have been able to identify the people at risk using their standard follow up methods, like lactate thresholds, anamnesis, questionnaires, etc.

Example 8: Implementation of score in work environment

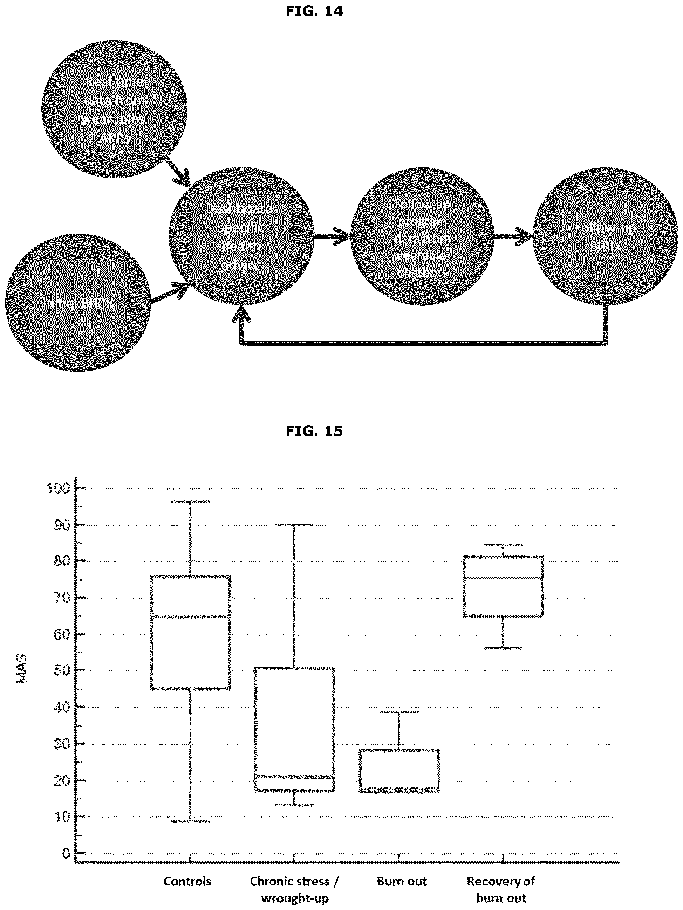

The trend in medicine is to go towards prevention of diseases. For this, biomarkers play an important role as they can be a disease predictor before the symptoms appear. Based on the measures of biomarkers specific risk factors can be calculated (BIRIX) (Figure 14).

A first measure of the BIRIX should be done upon the start of a health program. As most of the people have wearables or cell phones which registers many data, these data can also be incorporated in a health survey system that is patient or consumer centered. This additional information can help to adjust the personalized advices based on the BIRIX outcomes (eg. MAS). Employees at risk will receive personal follow up and practical actions using chat bots or private sessions with a psychologist or coach. During the follow up period, extra MAS analysis can be performed to measure the impact of the actions taken by the employee. In case no improvement was detected, the employee was send to his medical doctor.

In the future certain data coming from wearables will be more reliable and will be able to monitor changes in behavior. They will be used as monitoring system after the first BIRIX analysis. When certain alarms in those data appear, the employee can be notified to perform an extra MAS analysis. This enables a cost effective follow-up of the employee.

As the employer is not aware of which employees are performing analysis, it is not in violation of the privacy legislation. Nevertheless the human resources department and prevention department receive overviews of the profiles subdivided in the different departments.

It is supposed that the present invention is not restricted to any form of realization described previously and that some modifications can be added to the presented example without reappraisal of the appended claims. For example, the present invention has been described referring to stress monitoring, but it is clear that the invention can be applied to other fatigue syndromes.

Example 9: Use of the biomarkers as described herein for predicting the response of a patient diagnosed with depression to psychotherapy or to a medicament

*Response of a patient diagnosed with depression to psychotherapy*

60 blood samples are collected from patients diagnosed with depression. Furthermore, multiple questionnaires are filled in and patients are subdivided in two groups: one group received cognitive behavioral therapy for depression (pre-structured approach), while the others received short-term psychodynamic psychotherapy for depression (explorative approach). The severity of depression is measured by the Hamilton Rating Scale for Depression at completion of therapy.

Using PLS-DA, at least three important biomarkers to distinguish depressed patients from controls are selected from Table 1. Recovery from the depression is monitored using these biomarkers.

Furthermore PLS-DA analysis is used to identify those biomarkers to stratify the patients according to the therapy (e.g. cognitive behavioral therapy or short-term psychodynamic psychotherapy) that would suit them most.

Results could inform therapists on how to match psychotherapeutic treatments to specific personality characteristics of their patients.

*Response of a patient diagnosed with depression to a medicament*

PLS-DA analysis is used to identify those patients that benefit from specific anti-depressants. In this way, patients can be stratified based on a set of at least three biomarkers selected from Table 1 at the start of the therapy to lower the amount of anti-depressants taken by non-responders. Furthermore, the benefit from the anti-depressants can be quantified, as the recovery of the depression can be followed using the set of at least three biomarkers selected from Table 1.

Example 10: Use of the biomarkers as described herein for monitoring burn-out in a subject in response to therapy

Fatigue syndromes have often different phases. For burn-out for example, chronic stress and maladaptation towards high pressure and a demanding job, results in wrought-up employees. When the situation is not changed, further development towards a burn-out will occur. The employee will be unable to work and needs rest and specific actions suitable for the patient. These different phases can be monitored using the MAS.

60 burn-out patients are monitored for their recovery from burn-out. Multivariate statistical analysis results in sets of at least three biomarkers selected from Table 1 which either show the need of the patient for further therapy or the readiness to re-integrate in their work environment. As shown in Figure 15 the MAS score is decreasing from controls towards burn-out patients. After applying recovery strategies and burn-out therapy, the MAS score is again restored to the reference values (controls). This shows the possibility to monitor if the chosen therapy is fitting with the patients problem. If there is no recovery in the MAS score, other therapy or actions must be taken to enable the patient to recover from the burn-out.

**Claims**

1. An *in vitro* method for predicting, diagnosing, prognosing and/or monitoring chronic mental stress, comprising detecting at least three biomarkers independently selected from at least three different groups of biomarkers selected from:

 - a group of immune function biomarkers consisting of neopterin, secretory immunoglobulin A (sIgA), interleukin 6 (IL-6), interleukin 10 (IL-10), interleukin-1 receptor A (IL-1RA), epidermal growth factor (EGF), leukocytes, neutrophils, lymphocytes, monocytes, basophils, C-reactive protein (CRP) and myeloperoxidase (MPO);
 - a group of iron metabolism biomarkers consisting of ferritin, transferrin, iron, transferrin saturation, hemoglobin in reticulocytes, erythrocytes, mean cell haemoglobin (MCH), mean cell volume (MCV), mean cell haemoglobin concentration (MCHC) and reticulocytes;
 - a group of metabolism biomarkers consisting of creatinine, urea, vitamin D, folic acid, riboflavin, vitamin B12, 4-pyridoxic acid, flavin mononucleotide, thiamine, nicotinamide, pyridoxal 5'-phosphate, N1-methylnicotinamide, trigonelline, thiamine monophosphate, and visfatin;
 - a group of steroid hormone biomarkers consisting of dehydroepiandrosterone sulfate (DHEA-S), sex hormone binding globulin (SHBG), bio-available testosterone, total testosterone, free testosterone, and dehydroepiandrosterone (DHEA); and
 - a group of sympathetic adrenal medullary axis biomarkers consisting of amylase, epinephrine, and norepinephrine, preferably wherein amylase is alpha-amylase; and
 - optionally a group of neurotransmitter biomarkers consisting of anthranilic acid, cystathionine, picolinic acid, kynurenic acid (KYNA), L-kynurenine, quinolinic acid (QUIN), free tryptophan, xanthurenic acid, brain-derived neurotrophic factor (BDNF), S100 calcium-binding protein B (S100B), and γ-aminobutyric acid (GABA);

 in a sample obtained from said subject;
 wherein chronic mental stress is the mental status prior to development of mood disorders, such as depression or burn-out.

2. The method according to claim 1, wherein the at least three biomarkers are independently selected from at least three different groups of biomarkers selected from:

 - a group of immune function biomarkers consisting of neopterin, sIgA, IL-6, IL-10, IL-1RA, and CRP;
 - a group of iron metabolism biomarkers consisting of transferrin, iron, transferrin saturation, and ferritin;
 - a group of metabolism biomarkers consisting of creatinine, urea, vitamin D, folic acid, riboflavin, vitamin B12, 4-pyridoxic acid, pyridoxal 5'-phosphate, N1-methylnicotinamide, and visfatin;
 - a group of steroid hormone biomarkers consisting of DHEA-S and free testosterone; and
 - a group of sympathetic adrenal medullary axis biomarkers consisting of amylase, preferably alpha-amylase.

3. The method according to claim 1 or 2, comprising detecting at least four, preferably at least five, more preferably at least six, biomarkers in a sample obtained from said subject.

4. The method according to claim 3, wherein at least four biomarkers are independently selected from at least four different groups of biomarkers selected from the groups of biomarkers as defined in claim 1 or 2.

5. The method according to any one of claims 1 to 4, wherein detecting said at least three biomarkers allows obtaining a biosignature and wherein said method further comprises a step of predicting diagnosing, prognosing and/or monitoring chronic mental stress based on said biosignature comprising multivariate statistical modelling of said biosignature.

6. The method according to any one of claims 1 to 5, comprising the steps of

 (i) determining the status of said at least three biomarkers in the sample from the subject;
 (ii) combining the status of said at least three biomarkers as determined in step (i) to a biomarker imbalance risk index (BIRIX);
 (iii) finding a deviation or no deviation of the BIRIX compared to a reference value; and
 (iv) attributing said finding of deviation or no deviation to a particular diagnosis, prediction, or prognosis of the fatigue syndrome in the subject.

7. The method according to claim 6, wherein the BIRIX is obtained by multiplying the status of each of said at least three biomarkers by a corresponding weight, and wherein an expression signature defines the weight for each biomarker, preferably wherein the expression signature is defined using partial least squares-discriminant analysis (PLS-DA).

8. A kit for predicting, diagnosing, prognosing and/or monitoring chronic mental stress, the kit comprising:

- means for measuring the status of at least three biomarkers independently selected from at least three different groups of biomarkers, wherein the groups of biomarkers are as defined in claim 1 or 2, preferably as defined in claim 2, in a sample obtained from said subject; and
- a reference value or means for establishing said reference value, wherein said reference value represents a known diagnosis, prediction and/or prognosis of chronic mental stress;

wherein chronic mental stress is the mental status prior to development of mood disorders, such as depression or burn-out.

9. The method according to any one of claims 1 to 7 or the kit according to claim 8, wherein said subject is an employee.

10. The kit according to claim 8 or 9, wherein the kit further comprises a computer readable storage medium having recorded thereon one or more programs for carrying out the method of any one of claims 1 to 7 or 9.

11. A computer program product for use in conjunction with a computer having a processor and a memory connected to the processor, said computer program product comprising a computer readable storage medium having a computer program mechanism encoded thereon, wherein said computer program mechanism is loaded into the memory of said computer and causes said computer to carry out the method of claims 1 to 7 or 9.

12. An *in vitro* method for determining whether a subject is in need of therapeutic or prophylactic treatment of chronic mental stress comprising detecting at least three biomarkers independently selected from at least three different groups of biomarkers, wherein the groups of biomarkers are as defined in claim 1 or 2, preferably as defined in claim 2, in a sample obtained from said subject; wherein chronic mental stress is the mental status prior to development of mood disorders, such as depression or burn-out.

13. An *in vitro* method for predicting the response of a subject diagnosed with chronic mental stress to therapy, comprising detecting at least three biomarkers independently selected from at least three different groups of biomarkers, wherein the groups of biomarkers are as defined in claim 1 or 2, preferably as defined in claim 2, in a sample obtained from said subject; wherein chronic mental stress is the mental status prior to development of mood disorders, such as depression or burn-out

**FIG. 1**

## Table 1: list of relevant biomarkers

| | | |
|---|---|---|
| anthranilic acid | trigonelline | vitamin B12 |
| thyroid-stimulating hormone (TSH) | C-reactive protein (CRP) | serum free light chains |
| inhibin B | free fatty acids | thiamine monophosphate |
| citrate synthase | beta-endorphins | plasma viscosity |
| serotonin (5-hydroxytryptamine or 5-HT ) | erythrocytes | flavin mononucleotide |
| reactive oxygen species (ROS) | reactive nitrogen species (RNS) | aspartate aminotransferase (AST) |
| xanthurenic acid | total antioxidant capacity | pyridoxal |
| eotaxin family members | visfatin | thrombocytes |
| interleukin 8 (IL-8) | co-enzym Q10 | hemoglobin in reticulocytes |
| chromogranin A | amylase | folic acid |
| 3-nitrotyrosine | c-terminal agrin fragment (CAF) | gamma-glutamyl transpeptidase |
| epidermal growth factor (EGF) | a-macroglobulin | erythrocyte sedimentation rate |
| interleukin 10 (IL-10) | secretory Immunoglobulin A (sIgA) | transferrin |
| creatinine | free testosterone | vitamin C |
| ferritin | glutathione disulfide (GSSG) | trans-3'-hydroxycotinine |
| chemokine (C-C motif) ligand 2 (CCL2) | calcineurin | creatine kinase |
| total testosterone | basophils | glutamine |
| heat shock protein 70 (HSP70) | natural killer cells (NK cells) | pyridoxal 5'-phosphate |
| intracellular adhesion molecule (ICAM) family | thiobarbituric acid-reactive substances (TBARS) | N1-methylnicotinamide |
| skeletal troponin I | apolipoprotein CIII | transferrin saturation |
| vascular cell adhesion protein (VCAM) family | brain-derived neurotrophic factor | nicotinamide |
| 4-pyridoxic acid | macrophages | insulin resistance |
| vitamin A | glutathione peroxidase (GXP) | albumin |
| osteonectin (SPARC) | free tryptophan | lactate dehydrogenase (LDH) |
| vitamin E | neutrophils | eosinophils |
| prostaglandin 2 | granulocytes | lactate |
| urea | soluble CD146 | nicotinic acid |
| glutathione (GSH) | cotinine | glycogen |
| vascular endothelial growth factor A (VEGF-A) | cystathionine | leucin |
| glutathione reductase activity in erythrocytes | picolinic acid | insulin |
| free cortisol | dehydroepiandrosterone (DHEA) | hematocrit |
| E-selectin | γ-amuniobutyric acid (GABA) | glutamate |
| nitric oxide | insulin-like growth factor (IGF-1) | lipase |
| TNF-α | adrenocorticotropic hormone (ACTH) | myoglobulin |
| leptin | serum amyloid A (SAA) | pyridoxine |
| angiotensin-converting-enzyme (ACE) activity | total cortisol | alanine aminotransferase (ALT) |
| vitamin D | adiponectin | thiamine |

## FIG. 1 (continued)

| | | |
|---|---|---|
| oxipurin | 3-methyl histidine | valine |
| riboflavin | hemoglobin | serum total protein |
| malondialdehyde | zinc | 3-hydroxyisobutyrate |
| resistin | uric acid | tryptophan |
| vascular endothelial growth factor-C (VEGF-C) | kynurenic acid (KYNA) | alkaline phosphatase |
| interleukin 1 receptor antagonist (IL-1ra) | 4-hydroxynonenal (4-HNE) | triglycerides |
| calcitonin | neopterin | iron |
| myeloperoxidase (MPO) | kynurenine transaminase (KAT) | isoleucin |
| osteoprotegerin | alpha1 antitrypsin | mean corpuscular haemoglobin (MCH) |
| quinolinic acid (QUIN) | matrixmetalloproteinase 9 (MMP-9) | magnesium |
| sex hormone binding globulin (SHBG) | serotonin (5-hydroxytryptamine or 5-HT) receptor | mean corpuscular hemoglobin concentration (MCHC) |
| peroxisome proliferator-activated receptor gamma coactivator 1-alpha (PGC-1α) | soluble tumor necrosis factor alpha receptor type II | triglycerides |
| NF-κB (nuclear factor kappa-light-chain-enhancer of activated B cells) | catalase | branched-chain amino acids (BCAA) |
| interleukin 7 (IL-7) | cortisol-binding globulin (CBG) | thyroxine (T4) |
| interleukin 1 beta (IL1-beta) | S100 calcium-binding protein B (S100B) | ceruloplasmin |
| lipoperoxyl | F2-isoprostane | cholesterol |
| elastase | L-kynurenine | C-peptide |
| prolactin | free DNA | dopamine |
| 3-hydroxyanthranilic acid | anandamide | eotaxin |
| protein carbonyls | interleukin 6 (IL-6) | estradiol |
| heat shock protein 72 (HSP72) | glutamate receptor | free fatty acids |
| 3-hydroxykynurenine | leukocytes | triiodothyronine (T3) |
| catecholamines | chromium | gamma-OH-butyric acid |
| reticulocytes | interleukin 4 (IL-4) | glucose |
| alkoxyl | haptoglobin | high-density lipoprotein (HDL) |
| a-acid glycoprotein | dehydroepiandrosterone sulfate (DHEA-S) | hemoglobin |
| interleukin 2 (IL-2) | cholecalciferol | homocysteine |
| interferon gamma (INF-gamma) | growth hormone | potassium |
| bio-available testosterone | lymphocytes | low-density lipoprotein (LDL) |
| interleukin 15 (IL-15) | monocytes | lipase |
| insulin-like growth factor-binding protein 3 (IGF-BP3) | ghrelin | lipoperoxyl alkoxyl |
| superoxide dismutase (SOD) | sodium | tyramine |
| parathyroid hormone (PTH) | thiocyanate | niacin |
| cortisol releasing hormone (CRH) | mean cell volume (MCV) | kynurenine mono-oxygenase |
| kynureninase | indoleamine (2,3)-dioxygenase (IDO) | epinephrine |
| norepinephrine | vitamin B6 | |

**FIG. 1 (continued)**

Table 2: list of relevant biomarkers

| | |
|---|---|
| 3-hydroxyanthranilic acid | Magnesium |
| 3-hydroxyisobutyrate | mean corpuscular hemoglobin (MCH) |
| 3-hydroxykynurenine | mean corpuscular hemoglobin concentration (MCHC) |
| 4-pyridoxic acid | monocytes |
| adiponectin | myeloperoxidase (MPO) |
| | N1-methylnicotinamide |
| amylase | nicotinamide |
| anthranilic acid | nicotinic acid |
| basophils | picolinic acid |
| bio-available testosteron | pyridoxal |
| brain-derived neurotrophic factor | pyridoxal 5'-phosphate |
| C-reactive protein | quinolinic acid (QUIN) |
| creatinine | Reticulocytes |
| cystathionine | riboflavin |
| dehydroepiandrosterone sulfate (DHEA-S) | S100 calcium-binding protein B (S100B) |
| erythrocytes | secretory Immunoglobulin A (sIgA) |
| ferritin | sex hormone binding globulin |
| flavin mononucleotide | thyroid-stimulating hormone (TSH) |
| folic acid | Total cortisol |
| free cortisol | Total testosteron |
| Free testosteron | transferrin |
| Growth hormone | transferrin saturation |
| hemoglobin in reticulocytes | triglycerides |
| Insuling-like growth factor (IGF-1) | trigonelline |
| Interleukin 10 (IL-10) | tryptophan |
| Interleukin 6 (IL-6) | urea |
| iron | vascular endothelial growth factor A (VEGF-A) |
| kynurenic acid (KYNA) | vitamin B12 |
| Leukocytes | Vitamin D |
| L-kynurenine | xanthurenic acid |
| lymphocytes | |
| Magnesium | |

## FIG. 1 (continued)

### Table 3: list of relevant biomarkers

| | | |
|---|---|---|
| thiamine | Thiamine monophosphate | interleukin-1 receptor antagonist (IL-1ra) |
| Adiponectin | glutathione disulfide (GSSG) | PTH (parathyroid hormone) |
| alanine aminotransferase (ALT) | glutathione peroxidase (GXP) | pyridoxine |
| Prolactin | glycogen | Quinolonic acid (QUIN) |
| alkaline phosphatase | Growth hormone | resistin |
| alpha1 antitrypsin | haptoglobin | riboflavin |
| amylase | Heat shock protein (HSP72) | reactive oxygen species (ROS)/ reactive nitrogen species (RNS) |
| a-macroglobulin | Heat shock protein 70 (HSP70) | S100 calcium-binding protein B (S100B) |
| anandamide | hematocrit | secretory Immunoglobulin A (sIgA) |
| angiotensin-converting-enzyme (ACE) activity | hemoglobine | serotonin (5-HT) receptor |
| apolipoprotein CIII | High-density lipoprotein (HDL) | serum amyloid A (SAA) |
| aspartate aminotransferase (AST) | homocysteine | Serum free light chain levels |
| basophils | INF-gamma | sex hormone binding globulin |
| bio-available testosteron | inhibin B | skeletal troponin I |
| Brain-derived neurotrophic factor | insulin | soluble tumor necrosis factor alpha receptor type II |
| Branched-chain amino acid (BCAA) | Insuling-like growth factor (IGF-1) | prostaglandin 2 |
| calcineurin | Insulin-like growth factor-binding protein 3 (IGF-BP3) | glutathione (GSH) |
| calcitonin | Interleukin 1 beta (IL1-beta) | thiocyanate |
| ceruloplasmin | Interleukin 10 (IL-10) | thyroid-stimulating hormone (TSH) |
| cholesterol | Interleukin 15 (IL-15) | total antioxidant capacity |
| chromogranin A | Interleukin 2 (IL-2) | Total cortisol |
| co-enzym Q10 | Interleukin 4 (IL-4) | total protein |
| corticoid-binding globulin (CBG) | Interleukin 6 (IL-6) | total testosteron |
| Cortisol | Interleukin 7 (IL-7) | trans-3'-hydroxycotinine |
| cotinine | Interleukin 8 (IL-8) | transferrin |
| c-peptide | glutamine | triglycerides |
| C-reactive protein | iron | thrombocytes |
| estimated glomular filtration rate (eGFR) | isoleucine | tryptophan |
| c-terminal agrin fragment (CAF) | potassium | Tumor necrosis factor alpha (TNF-α) |
| dehydroepiandrosterone sulfate (DHEA-S) | kynurenic acid (KYNA) | tyramine |
| dopamine | lactaat dehydrogenase (LDH) | urea |
| Eotaxin | leucin | valine |
| epidermal growth factor (EGF) | Leukocytes | visfatin |
| erythrocyte sedimentation rate | lipase | vitamin A |

## FIG. 1 (continued)

| erythrocytes | lipoperoxyl alkoxyl | vitamin B12 |
|---|---|---|
| E-selectin | L-kynurenine | Vitamin B6 |
| estradiol | low density lipoprotein (LDL) | Vitamin C |
| ferritin | Magnesium | vitamin D |
| folic acid | malondialdehyde | Vitamin E |
| free fatty acids | Matrix metallopeptidase (MMP-9) | zink |
| triiodothyronine (T3) | chemokine (C-C motif) ligand 2 (CCL2) | γ-amuniobutyric acid (GABA) |
| free testosteron | Myeloperoxidase (MPO) | thyroxine (T4) |
| Free tryptophan | sodium | |
| gamma-aminobutyric acid (GABA) | neopterin | |
| gamma-glutamyl transpeptidase | neutrophils | |
| gamma-OH-boterzuur | Nicotinamide | |
| ghrelin | nicotinic acid | |
| glucose | osteonectin (SPARC) | |
| glutamaat | Peroxisome proliferator-activated receptor-gamma coactivator (PGC-1a) | |
| glutamaat receptor | Picolinic acid | |

## Table 4: list of relevant biomarkers

| cystathionine | urea | amylase |
|---|---|---|
| transferrin saturation | free cortisol | Total testosterone |
| bio-available testosteron | Free testosterone | vitamin D |
| creatinine | IGF-1 | hemoglobin in reticulocytes |
| vitamin B12 | lymphocytes | iron |
| dehydroepiandrosterone sulfate (DHEA-S) | Reticulocytes | leukocytes |
| erythrocytes | sex hormone binding globulin | MCH |
| ferritin | thyroid-stimulating hormone (TSH) | monocytes |
| folic acid | Total cortisol | transferrin |

## Table 5: list of relevant biomarkers for non-functional overreaching and/or overtraining syndrome

| anthranilic acid | cystathionine | N1-methylnicontinamide, |
|---|---|---|
| bio-available testosterone | reticulocytes | nicotinic acid |
| amylase | sex hormone binding globulin | quinolinic acid (QUIN) |
| bio-available testosterone | thyroid-stimulating hormone (TSH) | tryptophan |
| creatinine | total cortisol | xanthurenic acid |
| cystathionine | total testosterone | 3-hydroxyanthranilic acid |
| dehydroepiandrosterone sulfate (DHEA-S) | urea | 3-hydroxykynurenine |
| erythrocytes | vitamin D | brain-derived neurotrophic factor |
| ferritin | picolinic acid | S100 calcium-binding protein B (S100B) |
| folic acid | nicotinamide | **thiamine** |
| free cortisol | lymphocytes | |
| free testosterone | kynurenic acid (KYNA) | |
| IGF-1 | L-kynurenine | |

## FIG. 1 (continued)

### Table 6: list of most relevant biomarkers for non-functional overreaching and/or overtraining syndrome

| cystathionine | N1-methylnicontinamide | **thiamine** |
|---|---|---|
| amylase | ferritin | thyroid-stimulating hormone (TSH) |
| bio-available testosteron | folic acid | Total cortisol |
| creatinine | free cortisol | Total testosteron |
| Reticulocytes | Free testosteron | urea |
| dehydroepiandrosterone sulfate (DHEA-S) | IGF-1 | Vitamin D |
| erythrocytes | lymphocytes | sex hormone binding globulin |

### Table 7: list of relevant biomarkers for chronic stress, burn-out and/or depression

| anthranilic acid | pyridoxal 5'-phosphate | flavin mononucleotide |
|---|---|---|
| amylase | Vitamin D | kynurenic acid (KYNA) |
| creatinine | 3-hydroxyanthranilic acid | L-kynurenine |
| dehydroepiandrosterone sulfate (DHEA-S) | 3-hydroxyisobutyrate | Magnesium |
| free cortisol | 3-hydroxykynurenine | MCHC |
| hemoglobin in reticulocytes | 4-pyridoxic acid | myeloperoxidase (MPO) |
| iron | interleukin 1 receptor antagonist (IL-1ra) | N1-methylnicotinamide |
| Leukocytes | C-reactive protein (CRP) | nicotinamide |
| lymphocytes | cystathionine | picolinic acid |
| MCH | IL-6 | pyridoxal |
| monocytes | folic acid | secretory Immunoglobulin A (sIgA) |
| transferrin | Free testosterone | quinolinic acid (QUIN) |
| transferrin saturation | Growth hormone | Reticulocytes |
| urea | IL-10 | riboflavin |
| vitamin B12 | | |

### Table 8: list of most relevant biomarkers for chronic stress, burn-out and/or depression

| anthranilic acid | pyridoxal 5'-phosphate | flavin mononucleotide |
|---|---|---|
| Amylase (preferably alpha-amylase) | iron | transferrin |
| interleukin 1 receptor antagonist (IL-1ra), | 3-hydroxyanthranilic acid | 3-hydroxyisobutyrate |
| creatinine | Leukocytes | transferrin saturation |
| 3-hydroxykynurenine | 4-pyridoxic acid | C-reactive protein |
| dehydroepiandrosterone sulfate (DHEA-S) | lymphocytes | urea |
| free cortisol | MCH | vitamin B12 |
| cystathionine | folic acid | Free testosterone |
| Growth hormone | IL-10 | IL-6 |
| kynurenic acid (KYNA) | L-kynurenine | Magnesium |
| MCHC | myeloperoxidase (MPO) | N1-methylnicotinamide |
| nicotinamide | picolinic acid | pyridoxal |
| quinolinic acid (QUIN) | Reticulocytes | riboflavin |
| secretory Immunoglobulin A (sIgA) | Total cortisol | Triglycerides |

## FIG. 1 (continued)

| trigonelline | vascular endothelial growth factor A (VEGF-A) | xanthurenic acid |
|---|---|---|
| hemoglobin in reticulocytes | monocytes | Vitamin D |

### Table 9: list of relevant biomarkers for burn out

| amylase | 3-hydroxyanthranilic acid | IL-10 |
|---|---|---|
| Creatinine | 3-hydroxyisobutyrate | IL-6 |
| dehydroepiandrosterone sulfate (DHEA-S) | 3-hydroxykynurenine | kynurenic acid (KYNA) |
| free cortisol | 4-pyridoxic acid | L-kynurenine |
| hemoglobin in reticulocytes | adiponectin | myeloperoxidase (MPO) |
| iron | basophils | N1-methylnicotinamide |
| Leukocytes | bio-available testosterone | nicotinamide |
| Lymphocytes | C-reactive protein (CRP) | picolinic acid |
| transferrin | cystathionine | pyridoxal |
| transferrin saturation | flavin mononucleotide | pyridoxal 5'-phosphate |
| urea | folic acid | quinolinic acid (QUIN) |
| Vitamin B12 | free testosterone | riboflavin |
| Vitamin D | growth hormone | S100 calcium-binding protein B (S100B) |
| secretory Immunoglobulin A (sIgA) | total cortisol | triglycerides |
| trigonelline | brain-derived neurotrophic factor | xanthurenic acid |

### Table 10: list of most relevant biomarkers for burn out

| amylase | iron | urea |
|---|---|---|
| Creatinine | Leukocytes | Vitamin B12 |
| dehydroepiandrosterone sulfate (DHEA-S) | Lymphocytes | Vitamin D |
| free cortisol | transferrin | |
| hemoglobin in reticulocytes | transferrin saturation | |

### Table 11: list of relevant biomarkers for depression

| amylase | glutathione (GSH) | Nicotinamide |
|---|---|---|
| Creatinine | glutathione disulfide (GSSG) | peroxisome proliferator-activated receptor gamma coactivator 1-alpha (PGC-1α) |
| C-reactive protein (CRP) | glutathione peroxidase (GXP) | Picolinic acid |
| triglycerides | glycogen | Prolactin |
| adiponectin | Growth hormone | Prostaglandin 2 |
| alanine aminotransferase (ALT) | haptoglobin | PTH (parathyroid hormone) |
| | HDL | quinolinic acid (QUIN) |
| alkaline phosphatase | hematocrit | Resistin |
| Alpha1 antitrypsin | hemoglobine | riboflavin |
| | homocysteine | reactive oxygen species (ROS), reactive nitrogen species(RNS) |
| Apolipoprotein CIII | HSP70 | S100 calcium-binding protein B (S100B) |
| aspartate aminotransferase (AST) | HSP72 | secretory Immunoglobulin A (sIgA) |
| BCAA | IGF-1 | serotonin (5-hydroxytryptamine |

## FIG. 1 (continued)

| | | or 5-HT) receptor |
|---|---|---|
| Brain-derived neurotrophic factor | IGF-BP3 | serum amyloid A (SAA) |
| CBG | IL-10 | Serum free light chains |
| ceruloplasmin | IL-15 | sex hormone binding globulin |
| cholesterol | IL1-beta | skeletal troponin I |
| co-enzym Q10 | IL-1ra | Soluble tumor necrosis factor alpha receptor type II |
| free cortisol | IL-2 | Thiamine |
| total cortisol | IL-4 | thiocyanate |
| cotinine | IL-6 | thyroid-stimulating hormone (TSH) |
| c-peptide | IL-7 | TNF-α |
| dehydroepiandrosterone sulfate (DHEA-S) | IL-8 | total antioxidant capacity |
| dopamine | INF-gamma | Total cortisol |
| Eotaxin | inhibin B | serum total protein |
| Epidermal growth factor (EGF) | insulin | total testosterone |
| erythrocytes | iron | transferrin |
| estradiol | potassium | Thrombocytes |
| ferritin | kynurenic acid (KYNA) | tyramine |
| folic acid | lactate dehydrogenase (LDH) | urea |
| free fatty acids | LDL | Visfatin |
| thyroxine (T4) | Leukocytes | vitamin A |
| free testosterone | lipase | vitamin B12 |
| Free tryptophan | lipoperoxyl alkoxyl | Vitamin B6 |
| γ-amuniobutyric acid (GABA) | L-kynurenine | Vitamin C |
| gamma-glutamyl transpeptidase | Magnesium | vitamin D |
| gamma-OH-butiric acid | malondialdehyde | Vitamin E |
| ghrelin | chemokine (C-C motif) ligand 2 (CCL2) | zinc |
| glucose | MMP-9 | triiodothyronine (T3) |
| glutamate | Myeloperoxidase (MPO) | estimated glomular filtration rate (eGFR) |
| glutamate receptor | Sodium | |
| glutamine | neopterin | |

**FIG. 2**

**FIG. 2A**

**FIG. 2B**

**FIG. 2C**

**FIG. 2D**

**FIG. 2E**

**FIG. 2F**

**FIG. 2G**

**FIG. 3**

**FIG. 3A**

**FIG. 3B**

**FIG. 3C**

**FIG. 3D**

**FIG. 3E**

**FIG. 3F**

**FIG. 3G**

**FIG. 4**

**FIG. 4 (CONTINUED)**

|  | Biomarker | Clinical reference values for biomarker | |
|---|---|---|---|
|  |  | Lower limit (LL) | Upper limit (UL) |
| BM1 | Lymphocytes | 20 | 40 |
| BM2 | Iron | 33 | 193 |
| BM3 | Transferrin | 2 | 3,6 |
| BM4 | Vitamin B12 | 197 | 771 |
| BM5 | Vitamin D | 20 | 100 |
| BM6 | Amylase | 28 | 100 |

**FIG. 5**

## Risk outcome

- NFO/OTS endurance sport
- Maladaptation to training in team sport
- Reduced mental fitness
- Burnout
- Depression
- ...

## Biomarker risk index (BIRIX)

- Load adaptation score
- Training adaptation score
- Mental adaptation score
- Mental burnout score
- Mental depression score
- ...

**FIG. 6**

**FIG. 7**

**FIG. 8**

LAS

**FIG. 9**

**FIG. 10**

## MAS

**FIG. 11**

**FIG. 12**

**FIG. 13**

**FIG. 14**

**FIG. 15**